# EUROPEAN PATENT APPLICATION

(11) **EP 4 155 318 A1**
(43) Date of publication of application: **29.03.2023**
(21) Application number: 21832260.0
(22) Date of filing: 29.06.2021
(51) Int. Cl.: C07K 16/28, C07K 16/46, A61K 39/395, A61P 35/00

(54) **BISPECIFIC ANTIBODY AND USE THEREOF**

(30) Priority: 30.06.2020 CN 202010618149
(71) Applicant: Harbour Biomed US, Inc., Cambridge, MA 02142 (US)
(72) Inventor: SHI, Lei, Shanghai 201203 (CN); ZHONG, Chen, Shanghai 201203 (CN); WU, Xiaodong, Shanghai 201203 (CN); HE, Yun, Shanghai 201203 (CN); CHEN, Fei, Shanghai 201203 (CN); LV, Xiaocheng, Shanghai 201203 (CN); XIE, Jinli, Shanghai 201203 (CN); RONG, Yiping, Shanghai 201203 (CN); HUANG, Bing, Shanghai 201203 (CN); DU, Fangfang, Shanghai 201203 (CN); ZHAO, Jianxun, Shanghai 201203 (CN)
(74) Representative: Maiwald GmbH
(86) International application number: PCT/CN2021/103058
(87) International publication number: WO 2022/002036

(57) **Abstract**

Provided are a bispecific antibody and use thereof. The bispecific antibody comprises a B7-H4-targeting antigen-binding domain and a 4-1BB-targeting antigen-binding domain. The bispecific antibody has one or two or three sites for binding to 4-1BB, along with a novel fully human B7-H4 antibody. The bispecific antibody specifically binds to tumor cells by targeting B7-H4, reducing toxicity induced by 4-1BB activation. In addition, the bispecific antibody of the present invention comprises a human Fc fragment, and thus retains the binding of Fc to FcRn and has a longer half-life.

## Description

The present application claims priority to Chinese Patent Application No. 202010618149.1 filed on Jun. 30, 2020, the content of which is incorporated herein by reference in its entirety.

### TECHNICAL FIELD

The present invention relates to the field of biological pharmacy, particularly to a bispecific antibody, and especially to a B7-H4 and 4-1BB-targeting bispecific antibody and use thereof.

### BACKGROUND

B7-H4 (VTCN1, B7h.5, B7S1, B7x) is a transmembrane protein belonging to the B7/CD28 superfamily. The B7-H4 protein is expressed in activated T cells and B cells, monocytes and dendritic cells, and may negatively regulate immune responses of T cells.

Although B7-H4 mRNA is widely expressed, the B7-H4 protein is not expressed in most normal tissues. However, B7-H4 was overexpressed on the surfaces of tumor cells in breast cancer, ovarian cancer and endometrial cancer. Breast cancer is the second greatest malignancy worldwide with an increasing incidence. About one quarter of the female cancer patients are breast cancer patients. In 2019, there were 320 thousand new cases of breast cancer and a total of about 3.8 million in the United States. There are about 300 thousand new cases of breast cancer in China each year, and it is estimated that there will be up to 2.5 million patients in 2021. Ovarian cancer and endometrial cancer are common malignancies found in the female reproductive system. In China, about 50 thousand women are diagnosed with ovarian cancer and about 20 thousand deaths result from ovarian cancer each year; death rates from ovarian cancer are the highest among those from gynecologic malignancies. There are nearly 200 thousand new cases of endometrial cancer each year, which is the third cause of death among gynecologic malignancies.

B7-H4 is also overexpressed in non-small cell lung and renal cancer. Lung cancer is one of the most common cancers, and nearly 80% of lung cancer diagnoses are non-small cell lung cancer. The incidence and mortality of lung cancer are the highest either around the world or in China. There were about 2 million new cases of lung cancer and 1.76 million deaths in 2018 around the world. There are about 780 thousand new cases and about 630 thousand deaths in China each year. The incidence of renal cancer is low. There are about 70 thousand incidences of renal cancer in China each year.

As an emerging target for these tumors, B7-H4 has received attention in recent years. B7-H4 antibodies can act on tumor cells through multiple mechanisms. However, the development of B7-H4 antibodies has focused mainly on monoclonal antibodies and ADCs, and no bispecific antibody therapy is available at present.

4-1BB (TNFRSF9, CD137) is a transmembrane protein belonging to the TNF receptor superfamily. 4-1BB is a co-stimulatory molecule expressed on a variety of cells. It is a multifunctional modulator of immune activity. Its expression is induced in activated T cells, NK cells and monocytes, dendritic cells, macrophages, tumor-associated vascular endothelial cells, and the like. Its ligand is 4-1BBL, mainly expressed on professional APCs (such as mononuclear macrophages, DC cells and B cells), activated T cells and some tumor cells. Anti-4-1BB agonistic antibodies can inhibit tumors. However, the development of 4-1BB antibodies has focused mainly on monoclonal antibodies and antibodies that target both PD-L1 and 4-1BB. At present, there is no B7-H4 and 4-1BB-targeting bispecific antibody under clinical development.

Urelumab (BMS-663513) from Bristol-Myers Squibb (BMS) is a fully human IgG4 antibody of the anti-4-1BB antibody. It was the first anti-4-1BB antibody to be subjected to clinical trials. The initial clinical results of urelumab were published in 2008. Although encouraging efficacy was observed in some patients, the data showed urelumab to cause target and dose-associated hepatotoxicity. Seriously, two patients in the clinical trial died from hepatotoxicity, resulting in the clinical trial of urelumab as a single drug being discontinued. PD-L1 and 4-1BB-targeting bispecific antibodies are still currently under phase I clinical trial. Moreover, on tumor cells, the expression of PD-L1 overlaps very little with that of B7-H4. Therefore, there is an urgent need to develop safer and more effective bispecific antibodies that target both human B7-H4 and 4-1BB and can bind to cynomolgus monkey B7-H4 and 4-1BB.

### SUMMARY

The present invention addresses the technical problem that the prior art lacks a safe and effective bispecific antibody that targets both human B7-H4 and 4-1BB and can bind to cynomolgus monkey B7-H4 and 4-1BB, and provides a bispecific antibody, particularly a B7-H4 and 4-1BB-targeting bispecific antibody and use thereof.

In order to solve the technical problem described above, the present invention provides the following technical solutions.

A first aspect of the present invention provides a bispecific antibody comprising a B7-H4-targeting antigen-binding domain and a 4-1BB-targeting antigen-binding domain.

Preferably, the B7-H4-targeting antigen-binding domain comprises: HCDR1 with a sequence as set forth in SEQ ID NO: 16, HCDR2 with a sequence as set forth in SEQ ID NO: 46 and HCDR3 with a sequence as set forth in SEQ ID NO: 84; HCDR1 with a sequence as set forth in SEQ ID NO: 23, HCDR2 with a sequence as set forth in SEQ ID NO: 59 and HCDR3 with a sequence as set forth in SEQ ID NO: 98; or, HCDR1 with a sequence as set forth in SEQ ID NO: 16, HCDR2 with a sequence as set forth in SEQ ID NO: 60 and HCDR3 with a sequence as set forth in SEQ ID NO: 84; preferably comprises a VH with a sequence as set forth in SEQ ID NO: 142, SEQ ID NO: 159 or SEQ ID NO: 160; and more preferably comprises a heavy chain with a sequence as set forth in SEQ ID NO: 174, SEQ ID NO: 191 or SEQ ID NO: 192;

More preferably, the B7-H4-targeting antigen-binding domain further comprises LCDR1 with an amino acid sequence as set forth in SEQ ID NO: 112 or 113, LCDR2 with an amino acid sequence as set forth in SEQ ID NO: 118 and LCDR3 with an amino acid sequence as set forth in any one of SEQ ID NOs: 131-133;

Even more preferably, the B7-H4-targeting antigen-binding domain further comprises: LCDR1 with a sequence as set forth in SEQ ID NO: 112, LCDR2 with a sequence as set forth in SEQ ID NO: 118 and LCDR3 with a sequence as set forth in SEQ ID NO: 131; LCDR1 with a sequence as set forth in SEQ ID NO: 113, LCDR2 with a sequence as set forth in SEQ ID NO: 118 and LCDR3 with a sequence as set forth in SEQ ID NO: 132; or, LCDR1 with a sequence as set forth in SEQ ID NO: 112, LCDR2 with a sequence as set forth in SEQ ID NO: 118 and LCDR3 with a sequence as set forth in SEQ ID NO: 133; preferably further comprises a VL with a sequence as set forth in any one of SEQ ID NOs: 166-169; and more preferably further comprises a light chain with a sequence as set forth in any one of SEQ ID NOs: 198-201.

In the bispecific antibody described above:
The B7-H4-targeting antigen-binding domain preferably comprises HCDR1 with a sequence as set forth in SEQ ID NO: 16, HCDR2 with a sequence as set forth in SEQ ID NO: 46, HCDR3 with a sequence as set forth in SEQ ID NO: 84, LCDR1 with a sequence as set forth in SEQ ID NO: 112, LCDR2 with a sequence as set forth in SEQ ID NO: 118, and LCDR3 with a sequence as set forth in SEQ ID NO: 131; HCDR1 with a sequence as set forth in SEQ ID NO: 23, HCDR2 with a sequence as set forth in SEQ ID NO: 59, HCDR3 with a sequence as set forth in SEQ ID NO: 98, LCDR1 with a sequence as set forth in SEQ ID NO: 113, LCDR2 with a sequence as set forth in SEQ ID NO: 118, and LCDR3 with a sequence as set forth in SEQ ID NO: 132; or, HCDR1 with a sequence as set forth in SEQ ID NO: 16, HCDR2 with a sequence as set forth in SEQ ID NO: 60, HCDR3 with a sequence as set forth in SEQ ID NO: 84, LCDR1 with a sequence as set forth in SEQ ID NO: 112, LCDR2 with a sequence as set forth in SEQ ID NO: 118, and LCDR3 with a sequence as set forth in SEQ ID NO: 133.

More preferably, the B7-H4-targeting antigen-binding domain comprises a VH with a sequence as set forth in SEQ ID NO: 142 and a VL with a sequence as set forth in SEQ ID NO: 166; a VH with a sequence as set forth in SEQ ID NO: 159 and a VL with a sequence as set forth in SEQ ID NO: 167; a VH with a sequence as set forth in SEQ ID NO: 160 and a VL with a sequence as set forth in SEQ ID NO: 168; or, a VH with a sequence as set forth in SEQ ID NO: 159 and a VL with a sequence as set forth in SEQ ID NO: 169.

Even more preferably, the B7-H4-targeting antigen-binding domain comprises a heavy chain with a sequence as set forth in SEQ ID NO: 174 and a light chain with a sequence as set forth in SEQ ID NO: 198; a heavy chain with a sequence as set forth in SEQ ID NO: 191 and a light chain with a sequence as set forth in SEQ ID NO: 199; a heavy chain with a sequence as set forth in SEQ ID NO: 192 and a light chain with a sequence as set forth in SEQ ID NO: 200; or, a heavy chain with a sequence as set forth in SEQ ID NO: 191 and a light chain with a sequence as set forth in SEQ ID NO: 201.

With respect to the B7-H4-targeting antigen-binding domain, it is preferably in the form of single VH, tandem VH, ScFv, Fab or IgG. The tandem VH preferably is a tandem of two or more, e.g., 2, 3 or 4, VHs. When in the form of IgG, it comprises a constant region preferably derived from a human IgG1 comprising mutations L234A, L235A and P329G or mutations L234A and L235A.

With respect to the 4-1BB-targeting antigen-binding domains described above, it comprises HCDR1, HCDR2 and HCDR3, the HCDR1, the HCDR2 and the HCDR3 are preferably as follows:
the HCDR1 has a sequence as set forth in SEQ ID NO: 19 or a variant 1 thereof, or SEQ ID NO: 14; the HCDR2 has a sequence as set forth in SEQ ID NO: 49 or a variant 2 thereof, SEQ
ID NO: 51 or SEQ ID NO: 43; and the HCDR3 has a sequence as set forth in SEQ ID NO: 86 or a variant 3 thereof, SEQ ID NO: 96, SEQ ID NO: 89 or SEQ ID NO: 81; wherein
the variant 1 comprises mutations of one or more of T3I, S6N/R and Y7F; preferably, the variant 1 has a sequence as set forth in any one of SEQ ID NOs: 17-18 and SEQ ID NOs: 20-22 in the sequence listing;
the variant 2 comprises mutations including one or more of S1N/D, G2S/A, S3D/G, G5D/F/S/V and S6T/N/D; the variant 2 has a sequence preferably as set forth in any one of SEQ ID NOs: 47-48, SEQ ID NO: 50, SEQ ID NOs: 52-58 and SEQ ID NO: 61 in the sequence listing;
the variant 3 comprises mutations of one or more of G2R/D/A/K, S3A/T, S4G/N/A/T/H E5T/V/M/G, T6A, D7G/S, H9Y/S, Y10H, Y11F, N12G/D and V13I/M/T; the variant 3 has an amino acid sequence preferably as set forth in any one of SEQ ID NO: 85, SEQ ID NOs: 87-88 and SEQ ID NOs: 90-95 in the sequence listing.

In a specific embodiment, the 4-1BB-targeting antigen-binding domain comprises: HCDR1, HCDR2 and HCDR3 with sequences as set forth in SEQ ID NOs: 17, 47 and 85, respectively;
or, HCDR1, HCDR2 and HCDR3 with sequences as set forth in SEQ ID NOs: 18, 48 and 86, respectively;
or, HCDR1, HCDR2 and HCDR3 with sequences as set forth in SEQ ID NOs: 18, 49 and 87, respectively;
or, HCDR1, HCDR2 and HCDR3 with sequences as set forth in SEQ ID NOs: 19, 50 and 88, respectively;
or, HCDR1, HCDR2 and HCDR3 with sequences as set forth in SEQ ID NOs: 20, 51 and 89, respectively;
or, HCDR1, HCDR2 and HCDR3 with sequences as set forth in SEQ ID NOs: 18, 52 and 90, respectively;
or, HCDR1, HCDR2 and HCDR3 with sequences as set forth in SEQ ID NOs: 18, 49 and 90, respectively;
or, HCDR1, HCDR2 and HCDR3 with sequences as set forth in SEQ ID NOs: 21, 53 and 91, respectively;
or, HCDR1, HCDR2 and HCDR3 with sequences as set forth in SEQ ID NOs: 21, 54 and 92, respectively;
or, HCDR1, HCDR2 and HCDR3 with sequences as set forth in SEQ ID NOs: 19, 55 and 93, respectively;
or, HCDR1, HCDR2 and HCDR3 with sequences as set forth in SEQ ID NOs: 18, 49 and 86, respectively;
or, HCDR1, HCDR2 and HCDR3 with sequences as set forth in SEQ ID NOs: 19, 49 and 94, respectively;
or, HCDR1, HCDR2 and HCDR3 with sequences as set forth in SEQ ID NOs: 22, 56 and 86, respectively;
or, HCDR1, HCDR2 and HCDR3 with sequences as set forth in SEQ ID NOs: 18, 57 and 95, respectively;
or, HCDR1, HCDR2 and HCDR3 with sequences as set forth in SEQ ID NOs: 19, 58 and 96, respectively;
or, HCDR1, HCDR2 and HCDR3 with sequences as set forth in SEQ ID NOs: 18, 61 and 95, respectively;
or, HCDR1, HCDR2 and HCDR3 with sequences as set forth in SEQ ID NOs: 14, 43 and 81, respectively;

Preferably, the 4-1BB-targeting antigen-binding domain comprises one or more heavy chain variable regions with a sequence as set forth in SEQ ID NO: 143-157, 139, 284 or 161.

More preferably, the 4-1BB-targeting antigen-binding domain comprises a heavy chain with a sequence as set forth in SEQ ID NO: 175-189, 193, 285 or 171.

Even more preferably, the 4-1BB-targeting antigen-binding domain further comprises LCDR1, LCDR2 and LCDR3 with sequences as set forth in SEQ ID NOs: 109, 118, and 128, respectively; preferably comprises a light chain variable region with a sequence as set forth in SEQ ID NO: 163; and more preferably comprises a light chain with a sequence as set forth in SEQ ID NO: 195.

In the present invention, the 4-1BB-targeting antigen-binding domain described above may be in a conventional form in the art, e.g., in the form of single VH or tandem VH, HCAb or ScFv; the tandem VH preferably comprises two or more, e.g., 2, 3 or 4, VHs connected in series.

In one specific embodiment of the present invention, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 17, 47 and 85, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 87, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 50 and 88, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 20, 51 and 89, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 52 and 90, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 53 and 91, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 54 and 92, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 55 and 93, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 22, 56 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 17, 47 and 85, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 61 and 95, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 19, 58 and 96, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 49 and 90, respectively.

With respect to the bispecific antibody described herein, in a specific embodiment of the present invention:
the B7-H4-targeting antigen domain is in the form of IgG and the 4-1BB-targeting antigen-binding domain is in the form of single VH;
or, the B7-H4-targeting antigen domain is in the form of IgG and the 4-1BB-targeting antigen-binding domain is in the form of ScFv;
or, the B7-H4-targeting antigen domain is in the form of IgG and the 4-1BB-targeting antigen-binding domain is in the form of a tandem of 2 or 3 VHs;
or, the B7-H4-targeting antigen domain is in the form of Fab and the 4-1BB-targeting antigen-binding domain is in the form of HCAb or HCAb and VH (in the form of HCAb-VH);
or, the B7-H4-targeting antigen domain is in the form of Fab and the 4-1BB-targeting antigen-binding domain is in the form of VH, wherein the form of VH is preferably the form of single VH, or a tandem of 2 or 3 VHs .

More specifically:
the bispecific antibody is in a form where:
   (1) the C-terminus of an IgG is connected with an ScFv, and an VH of the ScFv is connected to the C-terminus; preferably, the bispecific antibody in this form comprises a polypeptide chain 1 shown as a formula of VL_{B7-H4}-CL, and a polypeptide chain 2 shown as a formula of VH_{B7-H4}-CH1- hinge-CH2-CH3-linker-VH_{4-1BB}-linker-VL_{4-1BB};
   (2) the C-terminus of an IgG is connected with a VH or a tandem VH; preferably, the bispecific antibody in this form comprises a polypeptide chain 1 shown as a formula of VL_{B7-H4}-CL, and a polypeptide chain 2 shown as a formula of VH_{B7-H4}-CH1-hinge-CH2-CH3-linker-(VH₄-1_{B}B₎ₙ-;
   (3) the N-terminus of an HCAb is connected with an Fab; preferably, the bispecific antibody in this form comprises a polypeptide chain 1 shown as a formula of VH_{B7-H4}-CH1, and a polypeptide chain 2 shown as a formula of VL_{B7-H4}-CL-linker-VH_{4-1BB}-linker-CH2-CH3;
   (4) the N-terminus of an HCAb is connected with an Fab, and the C-terminus of the Fab is connected with a VH; preferably, the bispecific antibody in this form comprises a polypeptide chain 1 shown as a formula of VH_{B7-H4}-CH1, and a polypeptide chain 2 shown as a formula of VL_{B7-H4}-CL-linker-VH_{4-1BB}-linker-CH2-CH3- VH_{4-1BB};
or (5) the N-termini of two CH2s of an Fc are each connected with an Fab, and a VH or a tandem VH; preferably, the bispecific antibody in this form comprises three polypeptide chains:
   a polypeptide chain 1 shown as a formula of VL_{B7-H4}-CL;
   a polypeptide chain 2 shown as a formula of VH_{B7-H4}-CH1-hinge-CH2-CH3; and
   a polypeptide chain 3 shown as a formula of VH_{4-1BB}-linker-CH2-CH3 or (VH_{4-1BB})ₙ-linker-CH2-CH3;
   wherein the linkers linking different domains comprise identical or different sequences;
   wherein the IgG or HCAb comprises an Fc comprising a mutation, preferably one of the following mutations:

1) L234A and L235A, optionally further P329G, YTE or DHS, according to the EU numbering, when being IgG1, wherein YTE refers to M252Y/S254T/T256E, and DHS refers to L at position 309 being mutated into D, Q at position 311 being mutated into H, and N at position 434 being mutated into S;
2) deletions at positions 233-235 according to the EU numbering, when being IgG1;
3) L234A and G237A according to the EU numbering, when being IgG1;
4) S228P and FALA according to the EU numbering, when being IgG4, wherein FALA refers to F at position 234 being mutated into A, and L at position 235 being mutated into A; and
5) N297A according to the EU numbering, when being IgG1.

The hinges and the linkers described above may be conventional in the art; preferably, the linkers are selected from the group consisting of SEQ ID NOs: 241-261, 282 and 288-289; wherein
the linker in form (1) preferably comprises a sequence as set forth in SEQ ID NO: 245;
the linker in form (2) preferably comprises a sequence as set forth in any one of SEQ ID NOs: 243 and 245-247 and SEQ ID NOs: 288-289;
the linker in form (3) preferably comprises a sequence as set forth in SEQ ID NO: 250;
the linker in form (4) preferably comprises a sequence as set forth in SEQ ID NO: 282; and
the linker in form (5) preferably comprises a sequence as set forth in SEQ ID NO: 245.

In one preferred embodiment of the present invention, the amino acid sequence of polypeptide chain 1 is as set forth in SEQ ID NO: 198, and the amino acid sequence of polypeptide chain 2 is as set forth in any one of SEQ ID NOs: 202-215;
or, the amino acid sequence of polypeptide chain 1 is as set forth in SEQ ID NO: 201, and the amino acid sequence of polypeptide chain 2 is as set forth in SEQ ID NO: 217, 230, 238, 240, 226, 262 or 239;
or, the amino acid sequence of polypeptide chain 1 is as set forth in SEQ ID NO: 200, and the amino acid sequence of polypeptide chain 2 is as set forth in any one of SEQ ID NOs: 218-225, 235-237, 263-268, 274-281, 286 and 287;
or, the amino acid sequence of polypeptide chain 1 is as set forth in SEQ ID NO: 201, the amino acid sequence of polypeptide chain 2 is as set forth in SEQ ID NO: 227, and the amino acid sequence of polypeptide chain 3 is as set forth in SEQ ID NO: 228, 229, 231 or 232;
or, the amino acid sequence of polypeptide chain 1 is as set forth in SEQ ID NO: 233, and the amino acid sequence of polypeptide chain 2 is as set forth in any one of SEQ ID NOs: 234 and 269-273.

A second aspect of the present invention provides an isolated nucleic acid encoding the bispecific antibody described above according to the first aspect.

A third aspect of the present invention provides an expression vector comprising the isolated nucleic acid according to the second aspect.

A fourth aspect of the present invention provides a host cell comprising the expression vector according to the third aspect; preferably, the host cell is a prokaryotic cell or a eukaryotic cell.

A fifth aspect of the present invention provides a method for preparing a bispecific antibody comprising culturing the host cell according to the fourth aspect and obtaining the bispecific antibody from the culture.

A sixth aspect of the present invention provides a pharmaceutical composition comprising the bispecific antibody according to the first aspect of the present invention.

A seventh aspect of the present invention provides use of the bispecific antibody according to the first aspect and the pharmaceutical composition according to the sixth aspect in the manufacture of a medicament for the prevention and/or treatment of a 4-1BB and/or B7-H4-associated disease.

The disease is preferably cancer. The cancer is preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer. Preferably, the cancer is breast cancer, ovarian cancer, endometrial cancer, renal cancer or cholangiocarcinoma. More preferably, the cancer is breast cancer.

An eighth aspect of the present invention provides a chimeric antigen receptor comprising the bispecific antibody according to the first aspect.

A ninth aspect of the present invention provides an antibody-drug conjugate comprising a cytotoxic agent, and the bispecific antibody according to the first aspect; preferably, the cytotoxic agent is MMAF or MMAE.

A tenth aspect of the present invention provides a kit comprising the bispecific antibody according to the first aspect, the chimeric antigen receptor according to the eighth aspect, the antibody-drug conjugate according to the ninth aspect and/or the pharmaceutical composition according to the sixth aspect;
preferably, the kit further comprises (i) a device for administering the antibody or an antigen-binding fragment thereof or the antibody-drug conjugate or the pharmaceutical composition; and/or (ii) instructions for use.

An eleventh aspect of the present invention provides a combination of kits comprising a kit A and a kit B, wherein:
the kit A comprises the bispecific antibody according to the first aspect, the chimeric antigen receptor according to the eighth aspect, the antibody-drug conjugate according to the ninth aspect and/or the pharmaceutical composition according to the sixth aspect;
the kit B comprises another anti-tumor antibody or a pharmaceutical composition comprising anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

A twelfth aspect of the present invention relates to a method for diagnosing, treating and/or preventing a 4-1BB and/or B7-H4-mediated disease or disorder comprising administering to a patient in need thereof a therapeutically effective amount of the bispecific antibody according to the first aspect, the chimeric antigen receptor according to the eighth aspect, the antibody-drug conjugate according to the ninth aspect and/or the pharmaceutical composition according to the sixth aspect, or treating a patient in need thereof using the combination of kits according to the eleventh aspect.

The disease or disorder is preferably a tumor, preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer; preferably, the cancer is breast cancer, ovarian cancer, endometrial cancer, renal cancer or cholangiocarcinoma; more preferably, the cancer is breast cancer.

A thirteenth aspect of the present invention relates to a method for immuno-detection or determination of 4-1BB or B7-H4 comprising using the bispecific antibody according to the first aspect, the chimeric antigen receptor according to the eighth aspect, the antibody-drug conjugate according to the ninth aspect and/or the pharmaceutical composition according to the sixth aspect; preferably, the detection is for non-diagnostic and/or therapeutic purposes.

A fourteenth aspect of the present invention relates to a combination therapy comprising administering to a patient in need thereof the bispecific antibody according the first aspect, the chimeric antigen receptor according to the eighth aspect, the antibody-drug conjugate according to the ninth aspect and/or the pharmaceutical composition according to the sixth aspect, and a second therapeutic agent; the second therapeutic agent preferably comprises another anti-tumor antibody or a pharmaceutical composition comprising anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

It should be noted that: the numerals in the "variant 1", "variant 2" and "variant 3" have no special meaning, and are only used for distinguishing identical terms.

The B7-H4×4-1BB bispecific antibody of the present invention is an exclusive B7-H4×4-1BB bispecific antibody, having one or two or three sites for binding to 4-1BB; both protein functional regions of the bispecific antibody have good binding activity with respect to cynomolgus monkeys.

The beneficial effects of the present invention are as follows:
1. The 4-1BB antibody of the present invention is a novel fully human antibody comprising only a "heavy chain", having binding activity to human 4-1BB and cynomolgus monkey 4-1BB. The 4-1BB heavy-chain antibody is only half the size of conventional IgG antibodies. Due to the absence of a light chain, the antibody can be used for bispecific antibodies, and the problems of light chain mismatching and heterodimerization are solved.
2. The B7-H4 antibody of the present invention is a novel fully human antibody, having binding activity to human B7-H4 and cynomolgus monkey B7-H4.
3. The bispecific antibodies of the present invention have one or two or three sites for binding to 4-1BB, so that the activity of the 4-1BB end is optimized.
4. The present invention reduces the toxicity arising from 4-1BB activation by targeting B7-H4 to specifically bind to tumor cells.
5. The present invention is a bispecific antibody structure with a human Fc fragment, retaining the binding of Fc to FcRn and thereby having a long half-life.

### BRIEF DESCRIPTION OF THE DRAWINGS

FIG. 1 (A) shows the *in vitro* binding of B7-H4 monoclonal antibodies on an SK-BR-3 cell line highly expressing B7-H4 determined by FACS; (B) shows the *in vitro* binding of B7-H4 monoclonal antibodies on a CHO-K1-cynomolgus monkey B7-H4 cell line highly expressing cynomolgus monkey B7-H4 determined by FACS; and (C) shows the *in vitro* binding of B7-H4 monoclonal antibodies on a CHO-K1-mouse B7-H4 cell line highly expressing mouse B7-H4 determined by FACS.
FIGs. 2-(A)-(I) show the *in vitro* binding of 4-1BB monoclonal antibodies on a CHO-K1-human 4-1BB cell line highly expressing human 4-1BB determined by FACS.
FIGs. 3-(A)-(I) show the *in vitro* binding of 4-1BB monoclonal antibodies on a CHO-K1-cynomolgus monkey 4-1BB cell line highly expressing cynomolgus monkey 4-1BB determined by FACS.
FIGs. 4-(A)-(E) show the functions of 4-1BB monoclonal antibodies to activate the 4-1BB pathway and induce T cell activation.
FIGs. 5-(A)-(I) are schematic structural diagrams of B7-H4×4-1BB bispecific molecules.
FIGs. 6-(A)-(L) show the *in vitro* binding of B7-H4×4-1BB bispecific antibodies on a CHO-K1 cell strain overexpressing human 4-1BB determined by FACS.
FIGs. 7-(A)-(C) show the *in vitro* binding of B7-H4×4-1BB bispecific antibodies on a CHO-K1 cell strain overexpressing cynomolgus monkey 4-1BB determined by FACS.
FIGs. 8-(A)-(K) show the *in vitro* binding of B7-H4×4-1BB bispecific antibodies on a SK-BR-3 cell line highly expressing B7-H4 determined by FACS.
FIGs. 9-(A)-(E) show T cell activation experiments for B7-H4×4-1BB bispecific antibodies in the presence of a SK-BR-3 cell line highly expressing B7-H4, and the release of cytokine IFN-γ.
FIGs. 10-(A)-(E) show T cell activation experiments for B7-H4×4-1BB bispecific antibodies in the presence of a SK-BR-3 cell line highly expressing B7-H4, and the release of cytokine IL-2.
FIGs. 11-(A)-(B) show the activation of T cells by B7-H4×4-1BB bispecific antibodies being independent of the presence of FcyR.
FIGs. 12-(A)-(B) show the expression levels of B7-H4 on tumor cell surfaces determined by FACS.
FIGs. 13-(A)-(H) show the activation of T cells by B7-H4×4-1BB bispecific antibodies being specifically dependent on the expression of B7-H4.
FIGs. 14-(A)-(H) show the serum stability of B7-H4×4-1BB bispecific antibodies PR003334, PR003335 and PR004282.
FIGs. 15-(A)-(C) show the *in vitro* binding of B7-H4×4-1BB bispecific antibody PR004282 to human, monkey and mouse 4-1BB.
FIGs. 16-(A)-(C) show the *in vitro* binding of B7-H4×4-1BB bispecific antibody PR004282 to human, monkey and mouse B7H4.
FIG. 17 shows the *in vitro* binding of B7-H4×4-1BB bispecific antibody PR004282 to both SK-BR-3 and CHO-K1/4-1BB.
FIGs. 18-(A)-(B) show the *in vitro* binding of B7-H4×4-1BB bispecific antibody PR004282 to human and monkey Pan T cells activated *in vitro.*
FIGs. 19-(A)-(B) show the cross-reactivity of B7-H4×4-1BB bispecific antibody PR004282 with members of the B7 family or other members of the TNFR family.
FIGs. 20-(A)-(C) show the ADCC effect of B7-H4×4-1BB bispecific antibody PR004282.
FIGs. 21-(A)-(C) show the determination results of the non-specific release of cytokines caused by B7-H4×4-1BB bispecific antibody PR004282.
FIGs. 22-(A)-(F) show the pharmacokinetics of B7-H4×4-1BB bispecific antibodies PR003334, PR003335 and PR004282 in wild-type mice.
FIG. 23 shows the pharmacokinetics of B7-H4×4-1BB bispecific antibody PR004282 (in single doses) in normal monkeys.
FIGs. 24-(A)-(B) show the anti-tumor effect of B7-H4×4-1BB bispecific antibody PR003334 in a BALB/c-hCD137/CT26-B7-H4 mouse model.
FIGs. 25-(A)-(B) show the anti-tumor effect of B7-H4×4-1BB bispecific antibody PR003338 in a MDA-MB-468 xenograft mouse model.
FIGs. 26-(A)-(B) show the anti-tumor effect of B7-H4×4-1BB bispecific antibody PR004282 in an OVCAR3 xenograft mouse model.
FIGs. 27-(A)-(I) show the anti-tumor effect and memory immune effect of B7-H4×4-1BB bispecific antibody PR004282 in a BALB/c-hCD137/CT26-B7-H4 mouse model.
FIGs. 28-(A)-(B) show the specificity of the anti-tumor effect of B7-H4×4-1BB bispecific antibody PR004282.

### DETAILED DESCRIPTION

The present invention is further illustrated by the following examples, which are not intended to limit the present invention. Experimental procedures without specified conditions in the following examples are performed in accordance with conventional procedures and conditions, or in accordance with instructions.

In the present application, the term "antibody" generally refers to a protein comprising a moiety that binds to an antigen, and optionally a scaffold or framework moiety that allows the antigen-binding moiety to adopt a conformation that facilitates binding of the antibody to the antigen. An antibody may typically comprise an antibody light chain variable region (VL) or an antibody heavy chain variable region (VH), or both. For example, the "heavy-chain antibody" in the present application comprises no VL regions but comprises VH regions only. The VH or VL regions can be further divided into hypervariable regions termed complementarity determining regions (CDRs), which are scattered over more conserved regions termed framework regions (FRs). Each VH or VL can consist of three CDRs and four FRs arranged from amino-terminus to carboxyl-terminus in the following order: FR1, CDR1, FR2, CDR2, FR3, CDR3 and FR4. The variable regions of the heavy and light chains comprise binding domains that interact with antigens. Examples of the antibody include, but are not limited to, full-length antibodies, heavy-chain antibodies (HCAbs), antigen-binding fragments (Fab, Fab', F(ab)2, Fv fragments, F(ab')₂, scFv, di-scFv and/or dAb), immunoconjugates, multispecific antibodies (e.g., bispecific antibodies), antibody fragments, antibody derivatives, antibody analogs, fusion proteins, etc., provided that they exhibit the desired antigen-binding activity.

In the present application, the term "variable" generally refers to the fact that certain portions of the sequences of the variable domains of antibodies vary considerably, resulting in the binding and specificity of various particular antibodies to their particular antigens. However, variability is not evenly distributed throughout the variable region of the antibody. It is concentrated in three segments in the light and heavy chain variable regions called CDRs or hypervariable regions (HVRs). FRs are more highly conserved portions of the variable domains. The variable domains of native heavy and light chains each comprise four FRs largely in a β-sheet configuration. The FRs are connected by three CDRs to form a loop connection, and in some cases to form part of a β-sheet structure. The CDRs in each chain are held in close proximity by the FRs and form, together with the CDRs from the other chain, antigen-binding sites of the antibody. The constant regions are not directly involved in the binding of the antibody to antigens, but they exhibit different effector functions, for example, being involved in antibodydependent cytotoxicity of the antibody.

In this application, the term "fully human antibody" generally refers to an antibody that is expressed by a genetically engineered antibody gene-deleted animal into which the entire gene that encodes an antibody in human is transferred. All parts of the antibody (including the variable and constant regions of the antibody) are encoded by genes of human origin. The fully human antibody can greatly reduce the immune side effects caused in the human body by the heterologous antibody. Methods for obtaining fully human antibodies in the art can include phage display, transgenic mice, and the like.

As used herein, the term "nucleic acid" refers to a DNA molecule and an RNA molecule. It may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

In the present application, the term "specifically bind to" generally refers to that an antibody binds to an epitope via its antigen-binding domain, and that the binding requires some complementarity between the antigen-binding domain and the epitope. According to this definition, an antibody is said to "specifically bind to" an antigen when the antibody more easily binds to an epitope via its antigen-binding domain than binds to a random, unrelated epitope.

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form another polypeptide chain, in which case an Fab (cross VH/VL) structure is formed; in certain embodiments, CH1 of the Fab is not linked to the hinge region, but rather the C-terminus of CL is linked to the hinge region of the heavy chain, in which case an Fab (cross Fd/LC) structure is formed.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced using a Harbour HCAb transgenic mouse.

In the art, the CDRs of an antibody can be defined using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see A1-Lazikani et al., J Mol Biol 273 : 927-948, 1997). In the present application, the Combined scheme comprising the Kabat scheme and the Chothia scheme can also be used to determine the amino acid residues in variable domain sequences and full-length antibody sequences (Table 1). In the present invention, each sequence is determined according to the Chothia scheme.

**Table 1. Definition schemes available for the CDRs of the antibody of the present invention (see http://bioinf.org.uk/abs/)**

| CDR | Kabat scheme | Chothia scheme | Combined scheme |
|---|---|---|---|
| LCDR1 | L24-L34 | L24-L34 | L24-L34 |
| LCDR2 | L50--L56 | L50--L56 | L50--L56 |
| LCDR3 | L89--L97 | L89--L97 | L89--L97 |
| HCDR1 | H31--H35 | H26--H32 | H26--H35 |
| HCDR2 | H50--H65 | H52--H56 | H50--H65 |
| HCDR3 | H95--H 102 | H95--H 102 | H95--H 102 |

Laa-Lbb can refer to an amino acid sequence from position aa (the Chothia scheme) to position bb (the Chothia scheme) beginning at the N-terminus of the light chain of the antibody; and Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H32 can refer to the amino acid sequence from position 26 to position 32 according to the Chothia scheme beginning at the N-terminus of the heavy chain of the antibody. The present invention uses the Chothia scheme to define the CDRs of an antibody.

Antibody Fc domain-mediated effector functions such as ADCC and CDC are also very important biological functions. Different IgG subtypes have different ADCC or CDC functions; for example, IgG1 and IgG3 have strong ADCC and CDC effects, while IgG2 and IgG4 have relatively weak effects. In addition, the inherent effector functions of Fc can be modulated by altering the binding ability of Fc to Fc receptors by amino acid mutation or modification. For example, the "LALA" double mutant (L234A/L235A) in IgG1 can significantly reduce the affinity for FcγRIIIA (CD16A), thereby reducing the ADCC effect. In addition, the P329G mutation can significantly reduce binding to a variety of Fcγ receptors (see Schlothauer T, Herter S, Koller CF, Grau-Richards S, Steinhart V, Spick C, Kubbies M, Klein C, Umaña P, Mössner E. Novel human IgG1 and IgG4 Fc-engineered antibodies with completely abolished immune effector functions. Protein EngDes Sel. 2016 Oct;29(10):457-466. doi: 10.1093/protein/gzw040. Epub 2016 Aug 29. PubMed PMID: 27578889). In the present application, in order to reduce the binding of B7-H4×4-1BB bispecific antibodies to Fcy receptors, the "LALA" double mutant (L234A/L235A) or "LALAPG" triple mutant (L234A/L235A/P329G) is introduced into the Fc of these antibodies.

### Example 1. Sequence Analysis, Expression and Purification, and Characterization and Analysis of Physicochemical Properties of Antibodies

### 1.1 Sequence analysis and optimization of antibodies

The sequences of the heavy chain variable domain of the antibody are derived from events such as gene rearrangements of germline gene V, D and J segments of heavy chain gene clusters and somatic hypermutations on chromosomes; the sequences of the light chain variable domain are derived from the events such as gene rearrangements of germline gene V, D and J segments of light chain gene clusters and somatic hypermutations. Gene rearrangement and somatic hypermutation are major factors in increasing antibody diversity. Antibodies derived from the same germline V gene segment may also produce different sequences, but with relatively high similarity overall. The germline gene segments that are likely to undergo gene rearrangement can be deduced from the antibody variable domain sequences using algorithms such as IMGT/DomainGapAlign (http://imgt.org/3Dstructure-DB/cgi/DomainGapAlign.cgi) or NCBI/IgBLAST (https://www.ncbi.nlm.nih.gov/igblast/).

Chemical modifications, sometimes introduced after amino acid chains of a protein or polypeptide is translated and synthesized in a cell, are called post-translational modifications (PTMs). For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have a greater effect on antigen binding or result in changes in the physicochemical properties of the antibody, if they are present in the variable domains, particularly in the antigen binding regions (e.g., CDRs) of an antibody. For example, glycosylation, deamidation, isomerization, oxidation, and the like may increase the instability or heterogeneity of antibody molecules, thereby increasing the difficulty and risk of antibody development. Thus, it is very important for the development of therapeutic antibodies to avoid some potential PTMs. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, it can be predicted that there is an N-linked glycosylation site from the N-x-S/T sequence pattern (asparagine at the first position, any amino acid other than non-proline at the second position, and serine or threonine at the third position). The amino acid sequence patterns leading to PTMs may be derived from germline gene sequences, e.g., the human germline gene fragment IGHV3-33 naturally having a glycosylation pattern NST in the FR3 region; or they may also be derived from somatic hypermutations. For example, NGS or NLT may be a glycosylation site, NS may be a deamidation site, and DG may cause isomerization of aspartic acid.

The amino acid sequence patterns of PTMs may be disrupted by amino acid mutations, thereby reducing or eliminating the formation of specific PTMs. There are different methods for designing mutations depending on the antibody sequences and PTM sequence patterns. One method is to replace a "hot spot" amino acid (e.g., N or S in the NS pattern) with an amino acid with similar physicochemical properties (e.g., to mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

### 1.2. Expression and purification of antibodies

This example describes a general method of antibody preparation in mammalian host cells (e.g., human embryonic kidney cell HEK293 or Chinese hamster ovary CHO cells and derived cells thereof) by using such techniques as transient transfection and expression, and affinity capture and separation. This method is applicable to an antibody of interest comprising an Fc region. The antibody of interest may consist of one or more protein polypeptide chains, and may be derived from one or more expression plasmids.

The amino acid sequences of the polypeptide chains of the antibody were converted into nucleotide sequences by codon optimization. The encoding nucleotide sequences were synthesized and cloned into expression vectors compatible with the host cell. The mammalian host cells were transfected simultaneously with plasmids encoding the polypeptide chains of the antibody in a particular ratio, and the recombinant antibody with correct folding and assembly of polypeptide chains could be obtained by the conventional recombinant protein expression and purification techniques. Specifically, FreeStyle^{™} 293-F cells (Thermo, #R79007) were expanded in FreeStyle^{™} F17 Expression Medium (Thermo, #A1383504). Before transient transfection, the cells were adjusted to a concentration of 6-8×10⁵ cells/mL, and cultured in a shaker at 37 °C with 8% CO₂ for 24 h at a concentration of 1.2×10⁶ cells/mL. 30 mL of cultured cells were taken. Plasmids encoding the polypeptide chains of the antibody were mixed in a certain ratio, and a total of 30 µg of the plasmids (the ratio of the plasmids to cells was 1 µg : 1 mL) were dissolved in 1.5 mL of Opti-MEM reduced serum medium (Thermo, #31985088). The resulting mixture was filtered through a 0.22 µm filter membrane for sterilization. Then, 1.5 mL of Opti-MEM was dissolved in 120 µL of 1 mg/mL PEI (Polysciences, #23966-2), and the mixture was left to stand for 5 min. PEI was slowly added to the plasmids, and the mixture was incubated at room temperature for 10 min. The mixed solution of plasmids and PEI was slowly added dropwise while shaking the culture flask, and the cells were cultured in a shaker at 37 °C with 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect^{™} (GE Healthcare, #71-5020-91) was equilibrated with a PBS buffer (pH 7.4) and rinsed with 2-5 column volumes of PBS. The column was loaded with the supernatant sample, and rinsed with 5-10 column volumes of PBS buffer, followed by 0.1 M glycine at pH 3.5 to elute the target protein. The eluate was adjusted to neutrality with Tris-HCl at pH 8.0, and concentrated and buffer exchanged into PBS buffer or a buffer with other components with an ultrafiltration tube (Millipore, #UFC901024) to obtain a purified solution of the recombinant antibody. Finally, the purified antibody solution was determined for concentration using NanoDrop (Thermo, NanoDrop^{™} One), subpackaged and stored for later use.

### 1.3 Analysis of protein purity and polymers by SEC-HPLC

In this example, analytical size-exclusion chromatography (SEC) was used to analyze the protein sample for purity and polymer form. An analytical chromatography column TSKgel G3000SWxl (Tosoh Bioscience, #08541, 5 µm, 7.8 mm × 30 cm) was connected to a highpressure liquid chromatograph HPLC (Agilent Technologies, Agilent 1260 Infinity II) and equilibrated with a PBS buffer at room temperature for at least 1 h. A proper amount of the protein sample (at least 10 µg) was filtered through a 0.22 µm filter membrane and then injected into the system, and an HPLC program was set: the sample was passed through the chromatography column with a PBS buffer at a flow rate of 1.0 mL/min for a maximum of 25 min. An analysis report was generated by the HPLC, with the retention time of the components with different molecular sizes in the sample reported.

### 1.4 Determination of thermostability of protein molecules by DSF

Differential scanning fluorimetry (DSF) is a commonly used high-throughput method for determining the thermostability of proteins. In this method, changes in the fluorescence intensity of the dye that binds to unfolded protein molecules were monitored using a real-time quantitative fluorescence PCR instrument to reflect the denaturation process of the protein and thus to reflect the thermostability of the protein. In this example, the thermal denaturation temperature (Tm) of a protein molecule was measured by DSF. 10 µg of protein was added to a 96-well PCR plate (Thermo, #AB-0700/W), followed by the addition of 2 µL of 100× diluted dye SYPROTM (Invitrogen, #2008138), and then the mixture in each well was brought to a final volume of 40 µL by adding buffer. The PCR plate was sealed, placed in a real-time quantitative fluorescence PCR instrument (Bio-Rad CFX96 PCR System), and incubated at 25 °C for 5 min, then at a temperature gradually increased from 25 °C to 95 °C at a gradient of 0.2 °C/0.2 min, and at a temperature decreased to 25 °C at the end of the test. The FRET scanning mode was used and data analysis was performed using Bio-Rad CFX Maestro software to calculate the Tm of the sample.

### Example 2. Acquisition of Anti-B7-H4 Fully Human Antibodies

The Harbour H2L2 mouse (Harbour Antibodies BV) is a transgenic mouse carrying an immune repertoire of human immunoglobulins that produces antibodies with intact human antibody variable domains and rat constant domains.

Harbour H2L2 mice were subjected to multiple rounds of immunization with soluble recombinant human B7-H4-ECD-mFc fusion protein (Sino Biological, #10738-H05H), or with an immunogenic reagent prepared by mixing human B7-H4-overexpressing CHO-K1 cells transfected with mCD40L with an immunoadjuvant.

When the titer of the B7-H4-specific antibody in the serum of mice was detected to reach a certain level, spleen cells of the mice were harvested. Screening was performed using the hybridoma fusion technique, B cell clone technique and phage library technique. The monoclonal antibodies obtained by screening were further identified, and clones 80C8-2E9 and 1025_B-1H11 were selected according to parameters such as the binding ability to human B7-H4 and the binding ability to cynomolgus monkey B7-H4. The nucleotide sequences encoding the variable domains of the antibody molecules and the corresponding amino acid sequences were obtained through conventional sequencing means. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules.

Anti-B7-H4 recombinant fully human IgG antibodies in this example are listed in Table 2.

**Table 2. Sequence numbers of anti-B7-H4 H2L2 antibodies (CDRs are all defined according to the Chothia scheme)**

| **Antibody No.** | **Notes** | **Light chain** | **Heavy chain** | **VL** | **VH** | **LCDR 1** | **LCDR 2** | **LCDR 3** | **HCDR 1** | **HCDR 2** | **HCDR 3** |
|---|---|---|---|---|---|---|---|---|---|---|---|
| PR001476 | 80C8-2E9 clone | 198 | 174 | 166 | 142 | 112 | 118 | 131 | 16 | 46 | 84 |
| PR002037 | 1025_B-1H11 clone | 199 | 191 | 167 | 159 | 113 | 118 | 132 | 23 | 59 | 98 |
| PR002408 | PR001476 variant | 200 | 192 | 168 | 160 | 112 | 118 | 133 | 16 | 60 | 84 |
| PR002410 | PR002037 variant | 201 | 191 | 169 | 159 | 113 | 118 | 132 | 23 | 59 | 98 |

In addition, a control antibody 1, also called 1D11.v1.9varC2, was designed by referring to patent WO2016040724A1 as a control in subsequent experiments.

**Table 3. Sequence numbers of B7-H4 control antibody (SEQ ID NO:)**

| **Name of antibody** | **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|---|
| Positive control 1 | PR000157 | 194 | 170 | 162 | 138 | 108 | 117 | 127 | 13 | 42 | 80 |

### 2.1 FACS assays for the binding ability of anti-human B7-H4 H2L2 monoclonal antibodies at the cellular level

This example is intended to investigate the *in vitro* binding activity of anti-human B7-H4 H2L2 monoclonal antibody to human/cynomolgus monkey/mouse B7-H4. Antibody binding experiments at the cellular level were conducted using a CHO-K1 cell strain overexpressing cynomolgus monkey B7-H4 (CHO-K1/cyno B7-H4, prepared in-house by Harbour Biomed), a CHO-K1 cell strain overexpressing mouse B7-H4 (CHO-K1/m B7-H4, prepared in-house by Harbour Biomed) and an SK-BR-3 cell line highly expressing human B7-H4 (ATCC^{®} HTB-30). Briefly, the CHO-K1/cyno B7-H4 cells, CHO-K1/m B7-H4 cells or SK-BR-3 cells were digested and resuspended with PBS containing 2% BSA. The cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 2 h away from light. Thereafter, the cells in each well were rinsed twice with 100 µL of pre-cooled PBS containing 2% BSA, and centrifuged at 500 g at 4 °C for 5 min, and then the supernatant was discarded. Then 100 µL of a fluorescent secondary antibody (Alexa Fluor 488-conjugated AffiniPure Goat Anti-Human IgG, Fcγ Fragment Specific, Jackson, #109-545-06, 1:500 diluted) was added to each well. The plate was incubated away from light at 4 °C for 1 h. The cells in each well were rinsed twice with 100 µL of pre-cooled PBS containing 2% BSA, and centrifuged at 500 g at 4 °C for 5 min, and then the supernatant was discarded. Finally, the cells in each well were resuspended in 200 µL of pre-cooled PBS containing 2% BSA, and the fluorescence signal values were read using an ACEA Novocyte3000 flow cytometer.

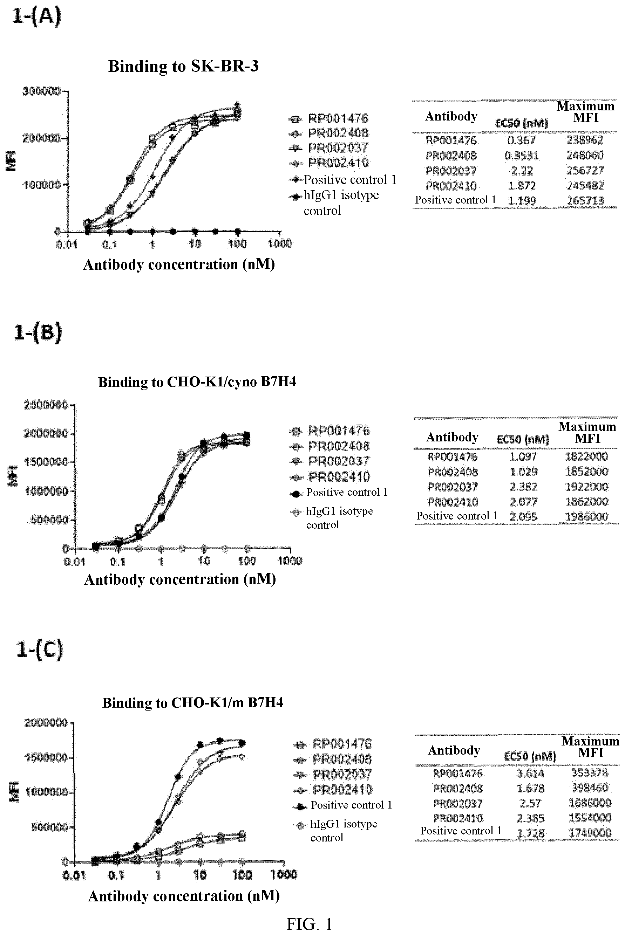

FIG. 1-(A) shows the *in vitro* binding of the B7-H4 antibodies on the SK-BR-3 cell line highly expressing B7-H4; PR001476 and PR002408 showed higher binding activity to the SK-BR-3 cell line than control antibody 1.

FIG. 1-(B) shows the *in vitro* binding of the B7-H4 antibodies on the CHO-K1-cynomolgus monkey B7-H4 cell line highly expressing cynomolgus monkey B7-H4; PR001476 and PR002408 showed higher binding activity to the CHO-K1-cynomolgus monkey B7-H4 cell line than control antibody 1.

FIG. 1-(C) shows the *in vitro* binding of the B7-H4 antibodies on the CHO-K1-mouse B7-H4 cell line highly expressing mouse B7-H4; there was no significant difference in the binding activity to the CHO-K1-mouse B7-H4 cell line between PR002410 and control antibody 1.

### Example 3. Acquisition of 4-1BB Fully Human Antibodies

### 3.1 Acquisition of anti-4-1BB fully human HCAb antibodies

The Harbour HCAb mouse (Harbour Antibodies BV, WO2010/109165A2) is a transgenic mouse carrying an immune repertoire of human immunoglobulins, capable of producing heavy chain-only antibodies that are only half the size of conventional IgG antibodies. The antibodies produced have only human antibody heavy chain variable domains and mouse Fc constant domains.

Harbour HCAb mice were subjected to multiple rounds of immunization with a soluble recombinant human 4-1BB-Fc fusion protein (ChemPartner) or human 4-1BB-overexpressing NIH-3T3 cells (ChemPartner). When the titer of the 4-1BB-specific antibody in the serum of mice was detected to reach a certain level, spleen cells of the mice were taken, from which B cells were isolated, and the CD138-positive plasma cells and human 4-1BB antigen-positive B cell populations were sorted using a BD FACS AriaII cell sorter. The human VH gene was amplified from plasma cells using conventional molecular biology techniques, and the amplified human VH gene fragments were constructed into mammalian cell expression plasmid pCAG vectors encoding the sequence of the heavy chain Fc region of the human IgG1 antibody. Mammal host cells (e.g., human embryonic kidney cell HEK293) were transfected with the plasmids and allowed to express antibodies to obtain a supernatant with fully human HCAb antibodies. Positive HCAb antibodies were identified by testing the supernatant with HCAb antibodies for binding to CHO-K1 cell CHO-K1/hu4-1BB highly expressing human 4-1BB by FACS. These HCAb antibodies were further identified, and several candidate HCAb antibody molecules were preferentially selected according to parameters such as the binding ability to human 4-1BB, the binding ability to cynomolgus monkey 4-1BB, and the T cell activation ability. The candidate HCAb antibody molecules were then subjected to sequence analysis and optimization to obtain several variant sequences. The VH sequence of the HCAb antibody and the Fc sequence of the heavy chain of human IgG1 were fused and expressed to obtain fully human recombinant HCAb antibody molecules. PR004469 is a PTM variant of PR001838. PR007381 is a germline variant of PR004469. The specific mutation sites are shown in Table 5.

**Table 4. Sequence listing for anti-4-1BB recombinant fully human HCAb antibodies**

| **Antibody No.** | **Heavy chain** | **VH** | **HCDR1** | **HCDR2** | **HCDR3** |
|---|---|---|---|---|---|
| PR001758 | 175 | 143 | 17 | 47 | 85 |
| PR001760 | 176 | 144 | 18 | 48 | 86 |
| PR001763 | 177 | 145 | 18 | 49 | 87 |
| PR001764 | 178 | 146 | 19 | 50 | 88 |
| PR001767 | 179 | 147 | 20 | 51 | 89 |
| PR001768 | 180 | 148 | 18 | 52 | 90 |
| PR001771 | 181 | 149 | 18 | 49 | 90 |
| PR001774 | 182 | 150 | 21 | 53 | 91 |
| PR001780 | 183 | 151 | 21 | 54 | 92 |
| PR001781 | 184 | 152 | 19 | 55 | 93 |
| PR001830 | 185 | 153 | 18 | 49 | 86 |
| PR001833 | 186 | 154 | 19 | 49 | 94 |
| PR001836 | 187 | 155 | 22 | 56 | 86 |
| PR001838 | 188 | 156 | 18 | 57 | 95 |
| PR001840 | 189 | 157 | 19 | 58 | 96 |
| PR001842 | 190 | 158 | 19 | 49 | 97 |
| PR004469 | 193 | 161 | 18 | 61 | 95 |
| PR007381 | 285 | 284 | 18 | 61 | 95 |

**Table 5. Mutation site designs for 4-1BB HCAb sequences**

| Initial antibody | Variant | Variable region mutations | Recombinant antibody subtype |
|---|---|---|---|
| PR001838 | PR004469 | G53A | Human IgG1 |
| PR001838 | PR007381 | F37V, P40A, E42G, T43K, K46E, G53A | Human IgG1 |

### 3.2 Acquisition of anti-4-1BB fully human H2L2 antibodies

The Harbour H2L2 mouse (Harbour Antibodies BV) is a transgenic mouse carrying an immune repertoire of human immunoglobulins that produces antibodies with intact human antibody variable domains and rat constant domains. Harbour H2L2 mice were subjected to multiple rounds of immunization with a soluble recombinant human 4-1BB-Fc fusion protein. When the titer of the 4-1BB-specific antibody in the serum of mice was detected to reach a certain level, spleen cells of the mice were taken and fused with a myeloma cell line to obtain hybridoma cells. After multiple rounds of screening and cloning of the hybridoma cells, hybridoma cell strains expressing anti-4-1BB monoclonal antibody molecules were separated. The nucleotide sequences encoding the variable domains of the antibody molecules and the corresponding amino acid sequences were obtained through conventional sequencing means for hybridomas. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules.

**Table 6. Sequence listing for anti-4-1BB recombinant fully human IgG antibodies**

| **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR000448 | 195 | 171 | 163 | 139 | 109 | 118 | 128 | 14 | 43 | 81 |

In addition, control antibodies were designed as controls in subsequent experiments.

**Table 7. Sequence listing for control antibodies**

| **Name of antibody** | **Antibody No.** | **Light chain** | **Heavy chain** | **VL** | **VH** | **LCDR 1** | **LCDR 2** | **LCDR 3** | **HCDR 1** | **HCDR 2** | **HCDR 3** |
|---|---|---|---|---|---|---|---|---|---|---|---|
| Urelumab analog | PR000628 | 197 | 173 | 165 | 141 | 111 | 120 | 130 | 14 | 45 | 83 |
| Utomilumab analog | PR000483 | 196 | 172 | 164 | 140 | 110 | 119 | 129 | 15 | 44 | 82 |

### 3.3 FACS assays for the binding ability of anti-human 4-1BB monoclonal antibodies at the cellular level

This example is intended to investigate the *in vitro* binding activity of anti-human 4-1BB HCAb and H2L2 monoclonal antibodies to human and cynomolgus monkey 4-1BB. Antibody binding experiments at the cellular level were conducted using a CHO-K1 cell strain overexpressing human 4-1BB (CHO-K1-hu 4-1BB, Genescript) and a CHO-K1 cell strain overexpressing cynomolgus monkey 4-1BB (CHO-K1-cyno 4-1BB, Genescript). Briefly, the CHO-K1-hu 4-1BB and CHO-K1-cyno 4-1BB cells were digested and resuspended in DMEM complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor 488-conjugated AffiniPure Goat Anti-Human IgG, Fcγ Fragment Specific, Jackson, #: 109-545-06, 1:500 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII.

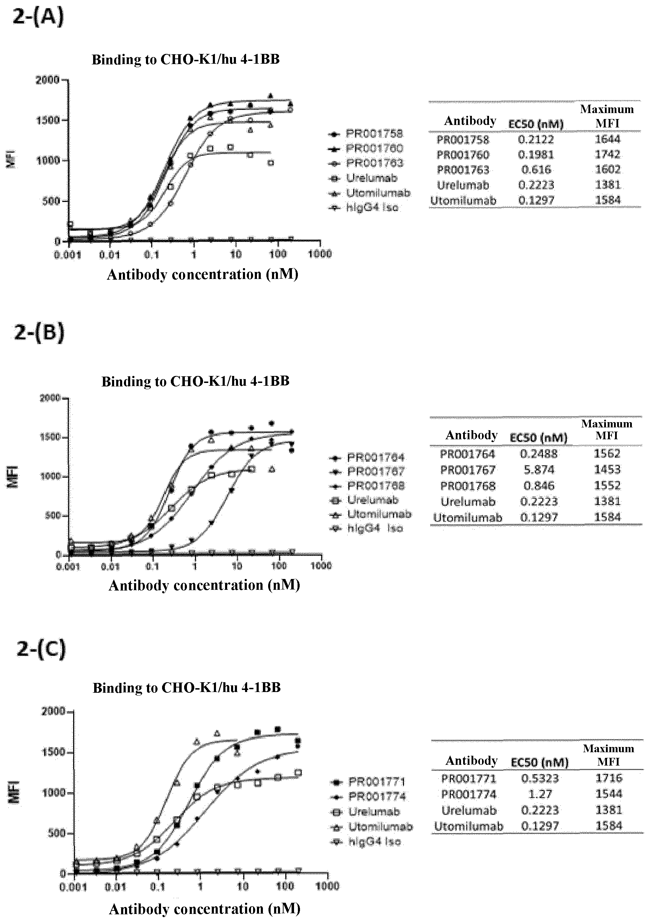

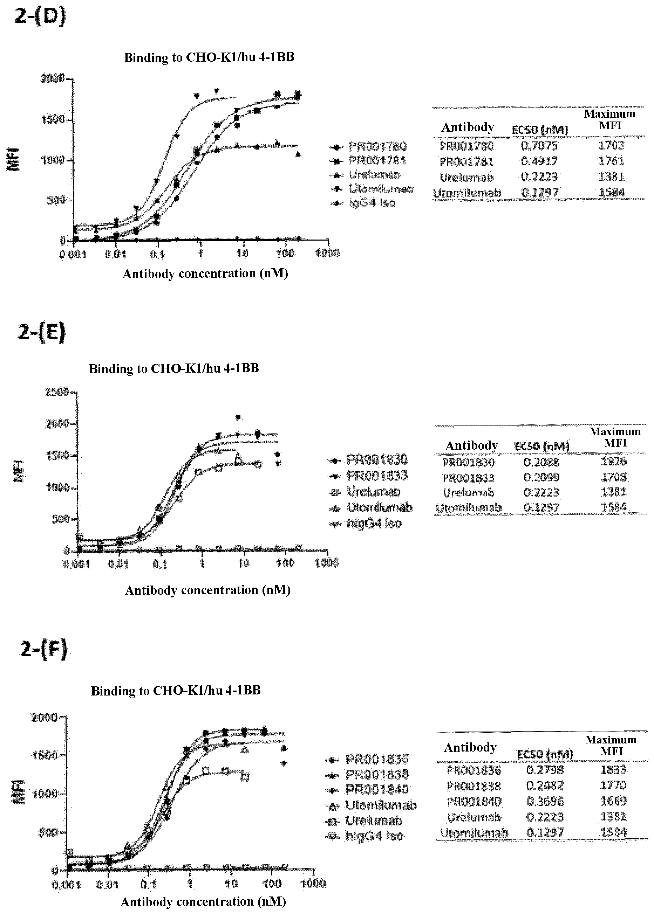

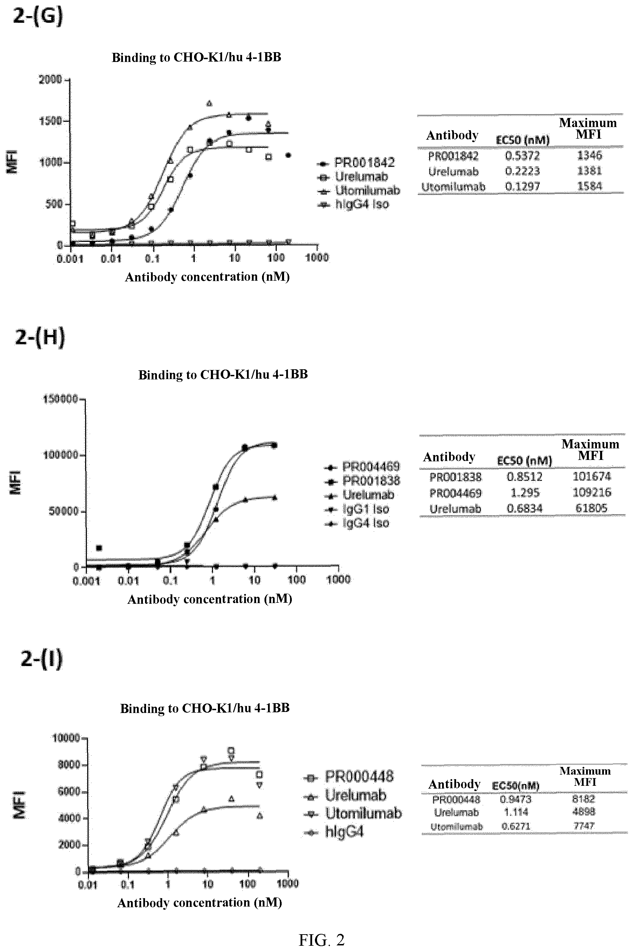

FIGs. 2-(A)-(I) show the *in vitro* binding of 4-1BB antibodies on the CHO-K1-human 4-1BB cell line highly expressing human 4-1BB.

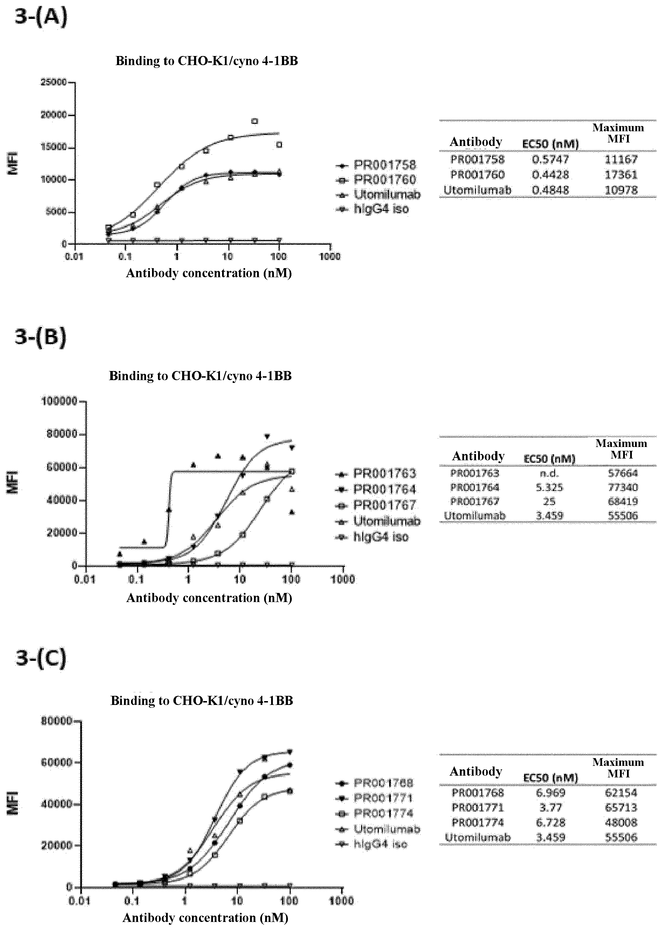

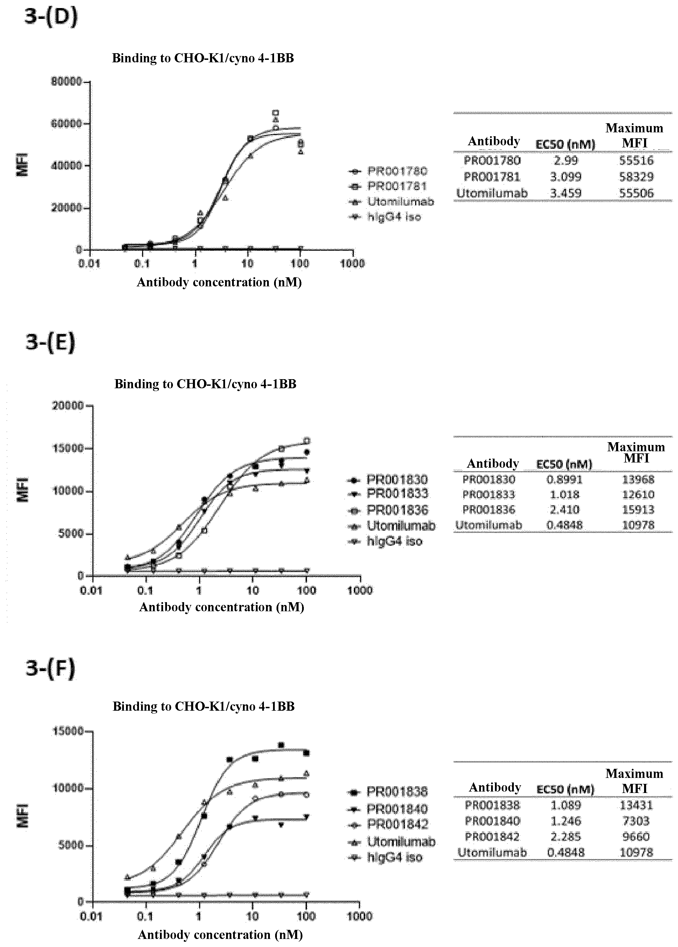

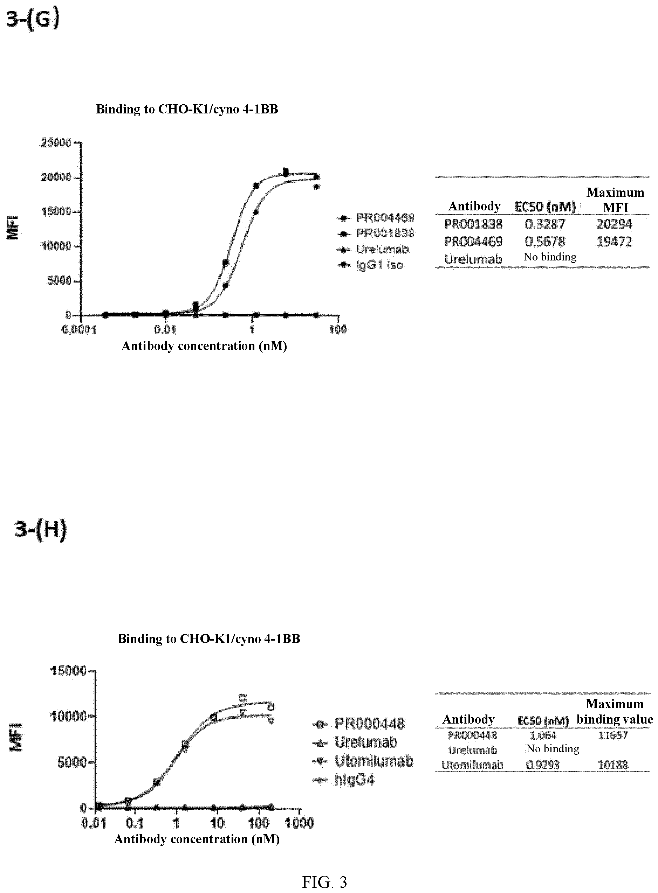

FIGs. 3-(A)-(I) show the *in vitro* binding of 4-1BB antibodies on the CHO-K1-cynomolgus monkey 4-1BB cell line highly expressing cynomolgus monkey 4-1BB.

As shown in FIGs. 2-(A)-(I), the anti-4-1BB antibodies of the present invention can all bind to human 4-1BB, and the binding ability of the antibody determined increases with the antibody concentration in a positive correlation. These antibodies can more sensitively bind to human 4-1BB at lower concentrations than the control antibodies (urelumab and utomilumab), with an EC₅₀ value comparable to those of urelumab and utomilumab.

As shown in FIGs. 3-(A)-(H), the anti-4-1BB antibodies of the present invention can all bind to monkey 4-1BB, and the binding ability of the antibody determined increases with the antibody concentration in a positive correlation. These antibodies can more sensitively bind to monkey 4-1BB at lower concentrations than the control antibody Tab (utomilumab), with an EC₅₀ value comparable to or better than that of utomilumab. However, the control antibody urelumab had no cross-binding activity to monkey 4-1BB.

### 3.4 Antigen-binding proteins can activate the 4-1BB pathway in vitro

CHO-K1 cells overexpressing human CD32b (CHO-K1/CD32b, Genscript, #M00600) were treated with 10 µg/mL mitomycin (Beijing Ruitaibio, #10107409001) at 37 °C for 30 min and then washed 4 times with F-12K culture medium containing 10% FBS. The treated cells were placed in a 96-well plate at 1.5×10⁴ cells/well and incubated in an incubator at 37 °C overnight. The next day, human CD3 positive T cells were isolated from human PBMCs using an MACS kit (Miltenyi Biotec, #130-096-535). The number of the cells was determined firstly, and then corresponding amounts of MACS buffer and Pan-T cell biotin antibody were added according to the number of the cells. The mixture was well mixed and left to stand at 4 °C for 5 min. Then, a corresponding amount of magnetic microbeads was added, and the mixture was left to stand at 4 °C for 10 min. What passed through the LS column were CD3 positive T cells. The previous day's culture medium was washed off the 96-well plate, and purified T cells were added at 1×10⁵ cells/well. Then a 4-1BB antibody or a control antibody was added at a corresponding concentration, and OKT3 (eBiosciences, #16-0037-85) was added at a final concentration of 0.3 µg/mL. The wells were cultured in an incubator at 37 °C for 72 h. After 72 hours, the supernatant was collected and assayed for IFN-γ content using an ELISA kit (Invitrogen, #88-7316-88). A coating antibody was added to a 96-well flat-bottom plate, and the plate was incubated at 4 °C overnight. The next day, ELISA buffer was added, and the plate was incubated at room temperature for 1 h. The collected supernatant was added, and the plated was incubated at room temperature for 2 h. The plate was washed twice, and test antibodies were added. The plate was incubated at room temperature for 1 h. The plate was washed twice, and HRP-streptavidin was added. The plate was incubated at room temperature for 1 h. Then TMB substrate was added, and ELISA stop buffer (BBI, #E661006-0200) was added later. The absorbance values at 450 nm and 570 nm (OD₄₅₀-OD₅₇₀) were read using a microplate reader (PerkinElemer, #Enspire), and the IFN-γ concentration was calculated.

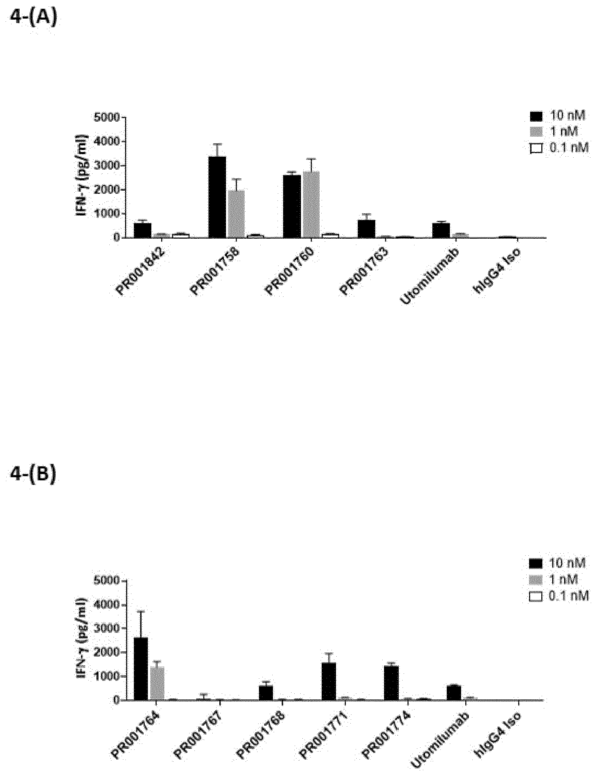

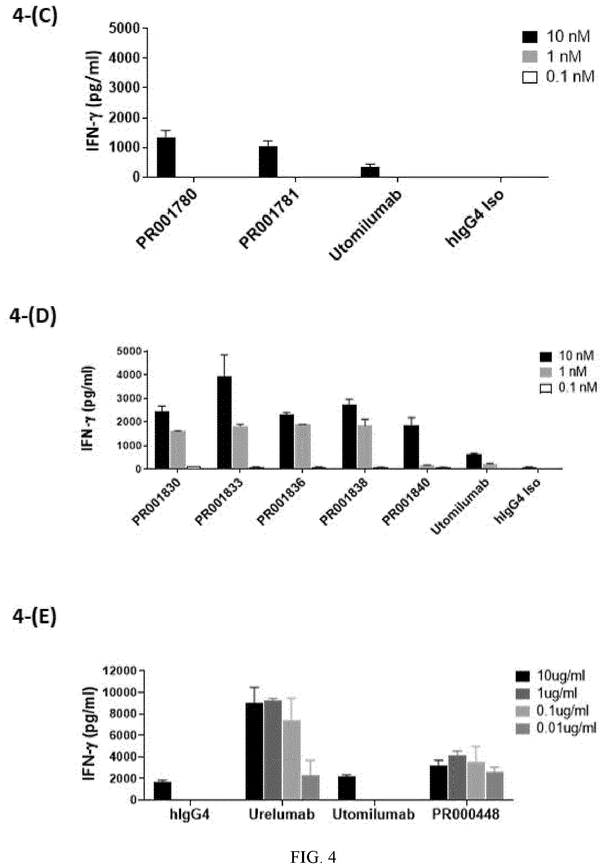

The results are shown in FIGs. 4-(A)-(E): the 4-1BB monoclonal antibodies have the function of activating the 4-1BB pathway and inducing T cell activation. PR001758, PR001760, PR001764, PR001771, PR001774, PR001780, PR001781, PR001830, PR001833, PR001836, PR001838, PR001840 and PR000448 all induced greater activation characterized by stronger IFN-γ signals than utomilumab.

### Example 4: Preparation of B7-H4×4-1BB Bispecific Antibodies

The anti-B7-H4 antibodies selected in Example 2 and the anti-4-1BB antibodies selected in Example 3 were combined and used for the preparation of bispecific antibodies, which could bind to two targets simultaneously, with one terminus being capable of recognizing B7-H4 specifically expressed on tumor cell surfaces and the other terminus being capable of binding to a 4-1BB molecule on T cells. After binding to the surface of a tumor cell, the B7-H4×4-1BB bispecific antibody molecule can recruit and activate T cells in the vicinity of the tumor cell, thereby killing the tumor cell.

The B7-H4×4-1BBbispecific antibodies prepared in this example include a variety of molecular structures.

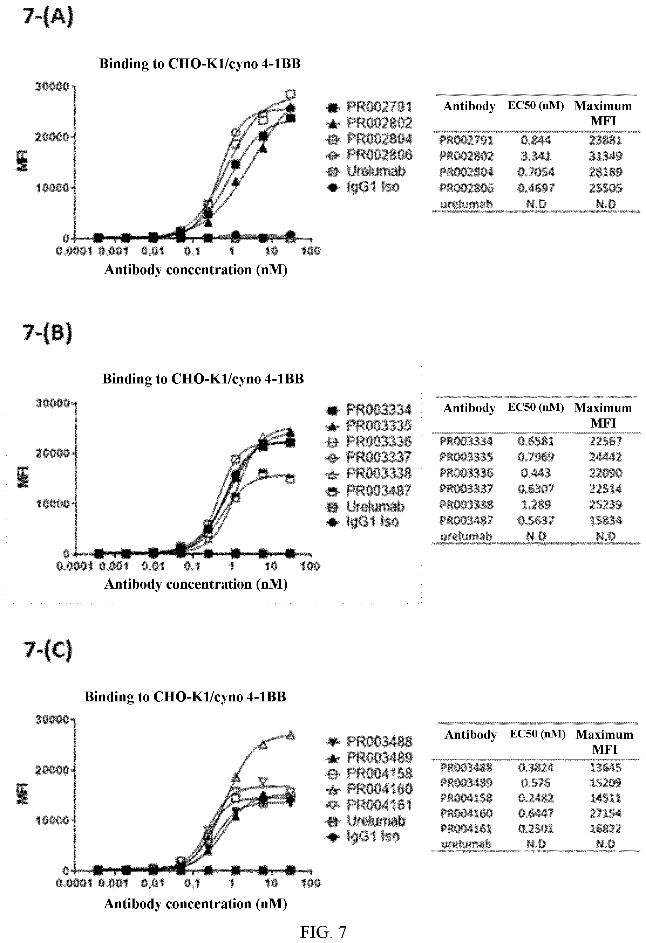

**FIGs. 7****-(A)-(I) are schematic structural diagrams of the bispecific molecules of the present invention.**

### 4.1 Construction of bispecific antibodies of IgG-scFv tetravalent symmetric structure

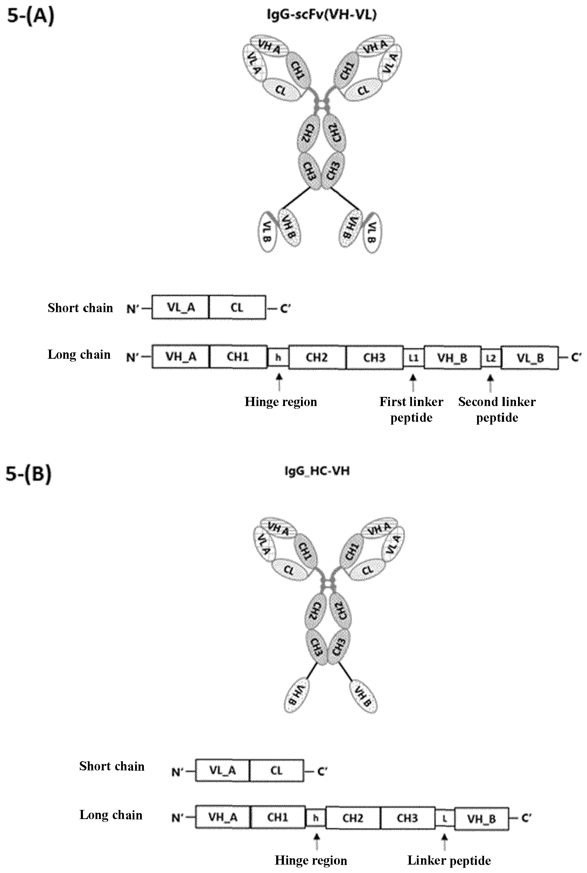

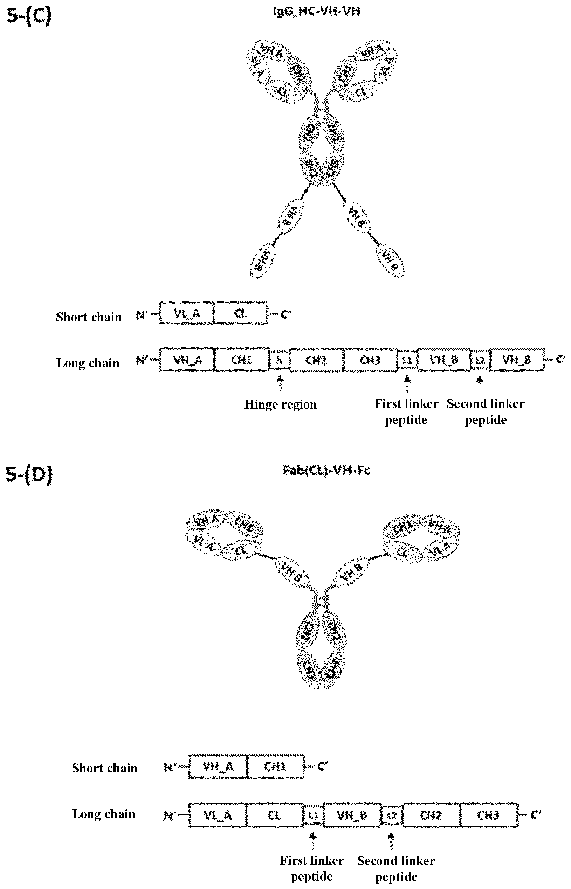

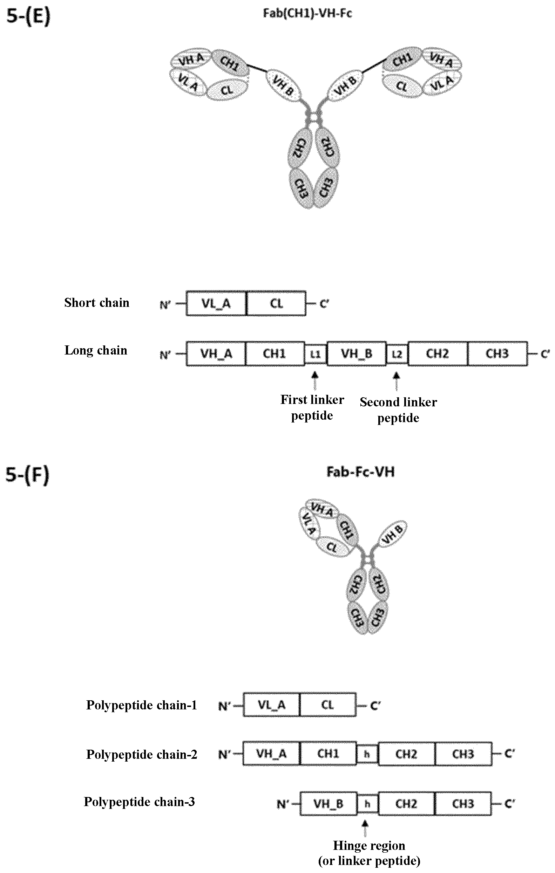

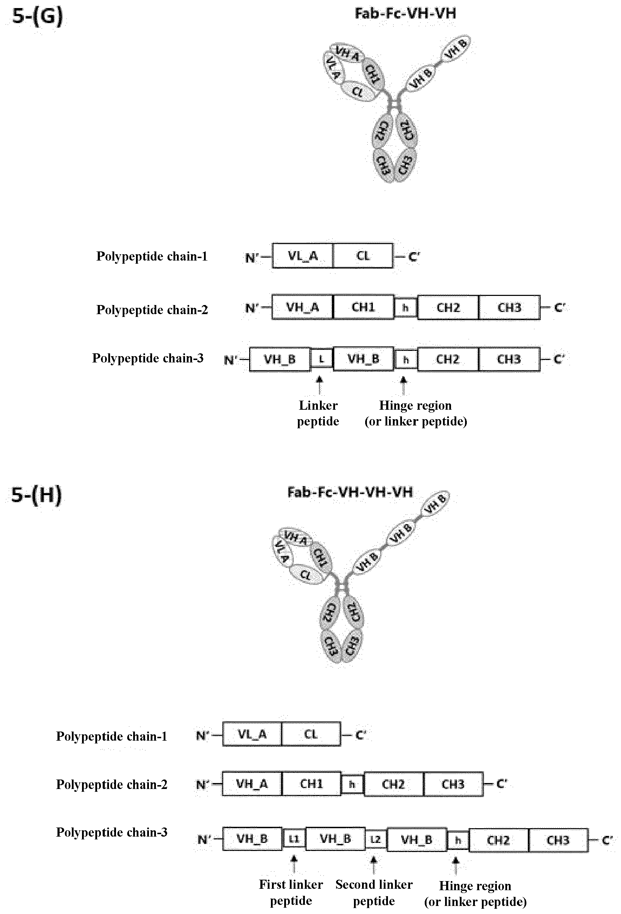

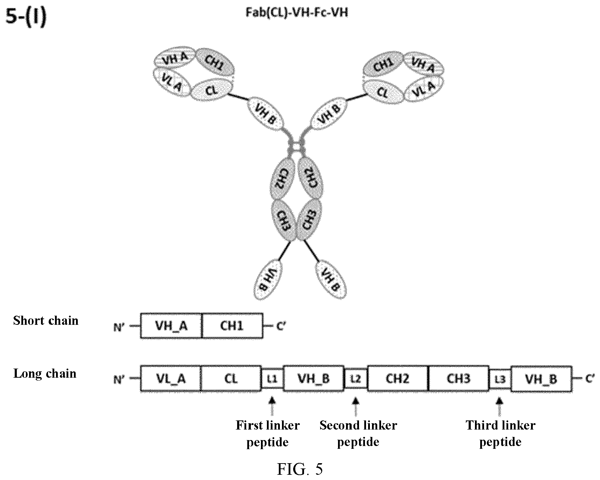

Bispecific antibodies of an IgG-scFv tetravalent symmetric structure were constructed using anti-B7-H4 H2L2 antibodies and anti-4-1BB H2L2 antibodies. A binding protein of an IgG-scFv tetravalent symmetric structure (as shown in FIG. 5-(A)) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-h-CH2-CH3-L1-VH_B L2-VL_B. h is a hinge region or derived sequence of an IgG antibody. The linker peptide L1 and the linker peptide L2 of polypeptide chain 2 may be the sequences listed in Table 8.

### 4.2 Construction of bispecific antibodies of IgG-VH tetravalent symmetric structure

Bispecific antibodies of an IgG-VH tetravalent symmetric structure were constructed using anti-B7-H4 H2L2 antibodies and anti-4-1BB HCAb antibodies. A binding protein of an IgG-VH tetravalent symmetric structure (as shown in FIG. 5-(B)) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-h-CH2-CH3-L-VH_B. h is a hinge region or derived sequence of an IgG antibody. In one embodiment, CH3 of the polypeptide chain 2 is fusion-linked directly to VH_B, i.e., L is 0 in length. In another embodiment, CH3 of the polypeptide chain 2 is linked to VH_B via the linker peptide L; L may be the sequence listed in Table 8.

### 4.3 Construction of bispecific antibodies of IgG-VH-VH hexavalent symmetric structure

Bispecific antibodies of an IgG-VH-VH hexavalent symmetric structure were constructed using anti-B7-H4 H2L2 antibodies and anti-4-1BB HCAb antibodies. A binding protein of an IgG-VH-VH hexavalent symmetric structure (as shown in FIG. 5-(C)) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-h-CH2-CH3-L1-VH_B-L2-VH_B. h is a hinge region or derived sequence of an IgG antibody. In one embodiment, CH3 of the polypeptide chain 2 is fusion-linked directly to VH_B, i.e., L is 0 in length. In another embodiment, the linker peptides L1 and L2 of polypeptide chain 2 may be the sequences listed in Table 8.

### 4.4 Construction of bispecific antibody molecules of Fab-HCAb structure

Bispecific antibodies of an Fab-HCAb symmetric structure were constructed using anti-B7-H4 H2L2 antibodies and anti-4-1BB HCAb antibodies. Two different structures were included: Fab(CL)-VH-Fc (as shown in FIG. 5-(D)) and Fab(CH)-VH-Fc (as shown in FIG. 5-(E)). A binding protein of an Fab(CL)-VH-Fc structure comprises two polypeptide chains: polypeptide chain 1, also known as short chain, from amino-terminus to carboxyl-terminus, comprising VH_A-CH1; and polypeptide chain 2, also known as long chain, from amino-terminus to carboxyl-terminus, comprising VL_A-CL-L1-VH_B-L2-CH2-CH3. The linker peptides L1 and L2 of polypeptide chain 2 may be the sequences listed in Table 8. A binding protein of an Fab(CH)-VH-Fc structure comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-L1-VH_B-L2-CH2-CH3. The linker peptides L1 and L2 of polypeptide chain 2 may be the sequences listed in Table 8.

### 4.5 Construction of Fab-Fc-VH(n) asymmetric structure

Bispecific antibodies of an Fab-Fc-VH(n) asymmetric structure were constructed using anti-B7-H4 H2L2 antibodies and anti-4-1BB HCAb antibodies. A binding protein of an Fab-Fc-VH(n) asymmetric structure (as shown in FIGs. 5-(F), (G) and (H)) comprises three polypeptide chains: polypeptide chain 1, also known as short chain, from amino-terminus to carboxyl-terminus, comprising VL_A-CL; polypeptide chain 2, also known as long chain, from amino-terminus to carboxyl-terminus, comprising VH_A-CH1-h-CH2-CH3; and polypeptide chain 3, from amino-terminus to carboxyl-terminus, comprising VH_B-h-CH2-CH3; or polypeptide chain 3, from amino-terminus to carboxyl-terminus, comprising VH_B-L-VH_B-h-CH2-CH3; or polypeptide chain 3, from amino-terminus to carboxyl-terminus, comprising VH_B-L1-VH_B-L2-VH_B-h-CH2-CH3. h is a hinge region or derived sequence of an IgG antibody. The linker peptides L1 and L2 of polypeptide chain 3 may be the sequences listed in Table 8.

To minimize the formation of byproducts with mismatched heavy chains (e.g., mismatching of two heavy chains of the anti-4-1BB antibody or two heavy chains of the anti-B7-H4 antibody), a mutant heterodimeric Fc region carrying a "knob-hole" mutation and a modified disulfide bond was used, as described in WO2009080251 and WO2009080252.

### 4.6 Construction of Fab-VH-Fc-VH symmetric structures

Bispecific antibodies of an Fab-VH-Fc-VH symmetric structure were constructed using anti-B7-H4 H2L2 antibodies and anti-4-1BB HCAb antibodies. A binding protein of an Fab-VH-Fc-VH symmetric structure (as shown in FIG. 5-(I)) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-L1-VH_B-CH1-h-CH2-CH3-VHB. h is a hinge region or derived sequence of an IgG antibody. The linker peptides L1 and L2 of polypeptide chain 3 may be the sequences listed in Table 8. In PR007379 in Table 11-(B), PR002408(H:gm) and PR002408 involved have identical CDRs, and FR2 of the variable region VH comprises 2 mutations: T69I and E101G.

**Table 8. Sequence listing for linker peptides**

| **Name of linker peptide** | **Length of linker peptide** | **Sequence of linker peptide** | **Sequence No. SEQ ID NO:** |
|---|---|---|---|
| GS_4 | 4 | GSGS | 241 |
| GS_5 | 5 | GGGGS | 242 |
| GS_6 | 6 | GGSGGS | 243 |
| GS_7 | 7 | GGGGSGS | 244 |
| GS_15 | 15 | GGGGSGGGGSGGGGS | 245 |
| GS_20 | 20 | GGGGSGGGGSGGGGSGGGGS | 246 |
| GS_25 | 25 | GGGGSGGGGSGGGGSGGGGSGG GGS | 247 |
| Human IgG1 hinge | 15 | EPKSCDKTHTCPPCP | 248 |
| Human IgG1 hinge (C220S) | 15 | EPKSSDKTHTCPPCP | 249 |
| H1_15 | 15 | EPKSSDKTHTPPPPP | 250 |
| G5-LH | 15 | GGGGGDKTHTCPPCP | 251 |
| H1_15-RT | 17 | EPKSSDKTHTPPPPPRT | 252 |
| L-GS_15-RT | 18 | LGGGGSGGGGSGGGGSRT | 253 |
| L-H1_15-RT | 18 | LEPKSSDKTHTPPPPPRT | 254 |
| KL-H1_15-RT | 19 | KLEPKSSDKTHTPPPPPRT | 255 |
| KL-H1_15-AS | 19 | KLEPKSSDKTHTPPPPPAS | 256 |
| RT-GS_5-KL | 9 | RTGGGGSKL | 257 |
| RT-GS_15-KL | 19 | RTGGGGSGGGGSGGGGSKL | 258 |
| RT-GS_25-KL | 29 | RTGGGGSGGGGSGGGGSGGGGS GGGGSKL | 259 |
| EPKSSD | 6 | EPKSSD | 260 |
| AS-GS_15 | 17 | ASGGGGSGGGGSGGGGS | 261 |
| H2_13 | 13 | ERKCCVECPPCP | 282 |
| GS_2 | 2 | GS | |
| (G2S)2 | 6 | GGSGGS | 288 |
| (G4S)2 | 10 | GGGGSGGGGS | 289 |

The amino acid sequences of the polypeptide chains of the bispecific antibody molecules obtained in the present invention are shown in the sequence listing below.

**Table 9. Sequence listing for the bispecific antibody molecules obtained in the present invention**

| **Antibody No.** | **Polypeptide chain 1** | **Polypeptide chain 2** | **Polypeptide chain 3** |
|---|---|---|---|
| PR002789 | 198 | 202 | |
| PR002972 | 201 | 217 | |
| PR002790 | 198 | 203 | |
| PR002791 | 198 | 204 | |
| PR002792 | 198 | 205 | |
| PR002793 | 198 | 206 | |
| PR002794 | 198 | 207 | |
| PR002795 | 198 | 208 | |
| PR002798 | 198 | 209 | |
| PR002800 | 198 | 210 | |
| PR002801 | 198 | 211 | |
| PR002802 | 198 | 212 | |
| PR002804 | 198 | 213 | |
| PR002805 | 198 | 214 | |
| PR002806 | 198 | 215 | |
| PR002808 | 198 | 216 | |
| PR003334 | 200 | 218 | |
| PR003335 | 200 | 219 | |
| PR003336 | 200 | 220 | |
| PR003337 | 200 | 221 | |
| PR003338 | 200 | 222 | |
| PR004162 | 201 | 230 | |
| PR004357 | 201 | 238 | |
| PR004359 | 201 | 240 | |
| PR004280 | 200 | 235 | |
| PR004281 | 200 | 236 | |
| PR004282 | 200 | 237 | |
| PR003487 | 200 | 223 | |
| PR003488 | 200 | 224 | |
| PR003489 | 200 | 225 | |
| PR004158 | 201 | 226 | |
| PR004358 | 201 | 239 | |
| PR004160 | 201 | 227 | 228 |
| PR004161 | 201 | 227 | 229 |
| PR004181 | 201 | 227 | 231 |
| PR004182 | 201 | 227 | 232 |
| PR004279 | 233 | 234 | |
| PR004995 | 201 | 262 | |
| PR005183 | 200 | 263 | |
| PR005184 | 200 | 264 | |
| PR005185 | 200 | 265 | |
| PR005186 | 200 | 266 | |
| PR005187 | 200 | 267 | |
| PR005188 | 200 | 268 | |
| PR005189 | 233 | 269 | |
| PR005190 | 233 | 270 | |
| PR005827 | 200 | 274 | |
| PR005828 | 200 | 275 | |
| PR005829 | 200 | 276 | |
| PR005830 | 200 | 277 | |
| PR005649 | 233 | 271 | |
| PR005650 | 233 | 272 | |
| PR005651 | 233 | 273 | |
| PR005838 | 200 | 278 | |
| PR005839 | 200 | 279 | |
| PR005866 | 200 | 280 | |
| PR007165 | 200 | 281 | |
| PR007379 | 200 | 286 | |
| PR007380 | 200 | 287 | |

The CDR sequence numbers of the antigen domains of the B7-H4×4-1BB bispecific antibodies are shown in the table below. In Table 10, the number 1# refers to a B7-H4-binding antigen domain, and the number 2# refers to a 4-1BB-binding antigen domain.

**Table 10. CDR sequence numbers of antigen-binding domains of bispecific antibodies (SEQ ID NO:)**

| **Structure No.** | **Antibody No.** | **Antigen-binding domain No.** | **LCDR 1** | **LCDR 2** | **LCDR 3** | **HCDR 1** | **HCDR 2** | **HCDR 3** |
|---|---|---|---|---|---|---|---|---|
| A | PR002789 | #1 | 112 | 118 | 131 | 16 | 46 | 84 |
| | | #2 | 109 | 118 | 128 | 14 | 43 | 81 |
| A | PR002972 | #1 | 113 | 118 | 132 | 23 | 59 | 98 |
| | | #2 | 109 | 118 | 128 | 14 | 43 | 81 |
| B | PR002790 | #1 | 112 | 118 | 131 | 16 | 46 | 84 |
| | | #2 | | | | 17 | 47 | 85 |
| B | PR002791 | #1 | 112 | 118 | 131 | 16 | 46 | 84 |
| | | #2 | | | | 18 | 48 | 86 |
| B | PR002792 | #1 | 112 | 118 | 131 | 16 | 46 | 84 |
| | | #2 | | | | 18 | 49 | 87 |
| B | PR002793 | #1 | 112 | 118 | 131 | 16 | 46 | 84 |
| | | #2 | | | | 19 | 50 | 88 |
| B | PR002794 | #1 | 112 | 118 | 131 | 16 | 46 | 84 |
| | | #2 | | | | 20 | 51 | 89 |
| B | PR002795 | #1 | 112 | 118 | 131 | 16 | 46 | 84 |
| | | #2 | | | | 18 | 52 | 90 |
| B | PR002798 | #1 | 112 | 118 | 131 | 16 | 46 | 84 |
| | | #2 | | | | 21 | 53 | 91 |
| B | PR002800 | #1 | 112 | 118 | 131 | 16 | 46 | 84 |
| | | #2 | | | | 21 | 54 | 92 |
| B | PR002801 | #1 | 112 | 118 | 131 | 16 | 46 | 84 |
| | | #2 | | | | 19 | 55 | 93 |
| B | PR002802 | #1 | 112 | 118 | 131 | 16 | 46 | 84 |
| | | #2 | | | | 18 | 49 | 86 |
| B | PR002804 | #1 | 112 | 118 | 131 | 16 | 46 | 84 |
| | | #2 | | | | 19 | 49 | 94 |
| B | PR002805 | #1 | 112 | 118 | 131 | 16 | 46 | 84 |
| | | #2 | | | | 22 | 56 | 86 |
| B | PR002806 | #1 | 112 | 118 | 131 | 16 | 46 | 84 |
| | | #2 | | | | 18 | 57 | 95 |
| B | PR002808 | #1 | 112 | 118 | 131 | 16 | 46 | 84 |
| | | #2 | | | | 19 | 49 | 97 |
| B | PR003334 | #1 | 112 | 118 | 133 | 16 | 60 | 84 |
| | | #2 | | | | 17 | 47 | 85 |
| B | PR003335 | #1 | 112 | 118 | 133 | 16 | 60 | 84 |
| | | #2 | | | | 18 | 48 | 86 |
| B | PR003336 | #1 | 112 | 118 | 133 | 16 | 60 | 84 |
| | | #2 | | | | 18 | 49 | 86 |
| B | PR003337 | #1 | 112 | 118 | 133 | 16 | 60 | 84 |
| | | #2 | | | | 19 | 49 | 94 |
| B | PR003338, PR004280, PR004281, PR004282, PR005183, PR005184, PR005185, PR005186, PR005187, PR005188, PR005827, PR005828, PR005829, PR005830, PR005838, PR005839, PR005866 | #1 | 112 | 118 | 133 | 16 | 60 | 84 |
| | | #2 | | | | 18 | 61 | 95 |
| B | PR004162 | #1 | 113 | 118 | 132 | 23 | 59 | 98 |
| | | #2 | | | | 18 | 48 | 86 |
| B | PR004357 | #1 | 113 | 118 | 132 | 23 | 59 | 98 |
| | | #2 | | | | 19 | 58 | 96 |
| B | PR004359 | #1 | 113 | 118 | 132 | 23 | 59 | 98 |
| | | #2 | | | | 18 | 57 | 95 |
| B | PR004995 | #1 | 113 | 118 | 132 | 23 | 59 | 98 |
| | | #2 | | | | 18 | 61 | 95 |
| C | PR003487, PR003488, PR003489 | #1 | 112 | 118 | 133 | 16 | 60 | 84 |
| | | #2 | | | | 18 | 48 | 86 |
| C | PR004158 | #1 | 113 | 118 | 132 | 23 | 59 | 98 |
| | | #2 | | | | 18 | 48 | 86 |
| C | PR004358 | #1 | 113 | 118 | 132 | 23 | 59 | 98 |
| | | #2 | | | | 19 | 58 | 96 |
| D | PR004279, PR005189, PR005649 | #1 | 112 | 118 | 133 | 16 | 60 | 84 |
| | | #2 | | | | 18 | 48 | 86 |
| D | PR005190 | #1 | 112 | 118 | 133 | 16 | 60 | 84 |
| | | #2 | | | | 18 | 61 | 95 |
| E | PR007165 | #1 | 112 | 118 | 133 | 16 | 60 | 84 |
| | | #2 | | | | 18 | 48 | 86 |
| G | PR004160 | #1 | 113 | 118 | 132 | 23 | 59 | 98 |
| | | #2 | | | | 18 | 48 | 86 |
| H | PR004161 | #1 | 113 | 118 | 132 | 23 | 59 | 98 |
| | | #2 | | | | 18 | 48 | 86 |
| H | PR004181 | #1 | 113 | 118 | 132 | 23 | 59 | 98 |
| | | #2 | | | | 18 | 49 | 90 |
| H | PR004182 | #1 | 113 | 118 | 132 | 23 | 59 | 98 |
| | | #2 | | | | 19 | 58 | 96 |
| I | PR005650 | #1 | 112 | 118 | 133 | 16 | 60 | 84 |
| | | #2 | | | | 18 | 48 | 86 |
| I | PR005651 | #1 | 112 | 118 | 133 | 16 | 60 | 84 |
| | | #2 | | | | 18 | 61 | 95 |

**Tables 11-(A)-(F) show the structures of the bispecific antibody molecules of the present invention.**

**Table 11-(A): B7-H4×4-1BB bispecific antibody molecules of an IgG-scFv symmetric structure**

| **Bispecific antibody molecules** | **B7-H4 antibody (IgG)** | **4-1BB antibody (scFv)** | **Structure of scFv end** | **First linker peptide (between Fc and scFv)** | **Second linker peptide (between VH_B and VL_B)** | **Fc type (mutation)** | **Length of first linker peptide** | **Length of second linker peptide** |
|---|---|---|---|---|---|---|---|---|
| PR002789 | PR001476 | PR000448 | VH-linker peptide-VL | H1_15-RT | GS_15 | Human IgG1 (L234A, L235A, P329G) | 17 | 15 |
| PR002972 | PR002410 | PR000448 | VH-linker peptide-VL | H1_15-RT | GS_15 | Human IgG1 (L234A, L235A, P329G) | 17 | 15 |

**Table 11-(B): B7-H4×4-1BB bispecific antibody molecules of an IgG-VH tetravalent symmetric structure**

| **Bispecific antibody molecule** | **B7-H4 antibody (IgG)** | **4-1BB antibody (VH_B)** | **VH_B position relative to IgG** | **Linker peptide (between VH_B and IgG)** | **Fc type (mutation)** | **Length of linker peptide** |
|---|---|---|---|---|---|---|
| PR002790 | PR001476 | PR001758 | C-terminus of heavy chain | H1_15-RT | Human IgG1 (L234A, L235A, P329G) | 17 |
| PR002791 | PR001476 | PR001760 | C-terminus of heavy chain | H1_15-RT | Human IgG1 (L234A, L235A, P329G) | 17 |
| PR002792 | PR001476 | PR001763 | C-terminus of heavy chain | H1_15-RT | Human IgG1 (L234A, L235A, P329G) | 17 |
| PR002793 | PR001476 | PR001764 | C-terminus of heavy chain | H1_15-RT | Human IgG1 (L234A, L235A, P329G) | 17 |
| PR002794 | PR001476 | PR001767 | C-terminus of heavy chain | H1_15-RT | Human IgG1 (L234A, L235A, P329G) | 17 |
| PR002795 | PR001476 | PR001768 | C-terminus of heavy chain | H1_15-RT | Human IgG1 (L234A, L235A, P329G) | 17 |
| PR002798 | PR001476 | PR001774 | C-terminus of heavy chain | H1_15-RT | Human IgG1 (L234A, L235A, P329G) | 17 |
| PR002800 | PR001476 | PR001780 | C-terminus of heavy chain | H1_15-RT | Human IgG1 (L234A, L235A, P329G) | 17 |
| PR002801 | PR001476 | PR001781 | C-terminus of heavy chain | H1_15-RT | Human IgG1 (L234A, L235A, P329G) | 17 |
| PR002802 | PR001476 | PR001830 | C-terminus of heavy chain | H1_15-RT | Human IgG1 (L234A, L235A, P329G) | 17 |
| PR002804 | PR001476 | PR001833 | C-terminus of heavy chain | H1_15-RT | Human IgG1 (L234A, L235A, P329G) | 17 |
| PR002805 | PR001476 | PR001836 | C-terminus of heavy chain | H1_15-RT | Human IgG1 (L234A, L235A, P329G) | 17 |
| PR002806 | PR001476 | PR001838 | C-terminus of heavy chain | H1_15-RT | Human IgG1 (L234A, L235A, P329G) | 17 |
| PR002808 | PR001476 | PR001842 | C-terminus of heavy chain | H1_15-RT | Human IgGl (L234A, L235A, P329G) | 17 |
| PR003334 | PR002408 | PR001758 | C-terminus of heavy chain | H1_15-RT | Human IgG1 (L234A, L235A) | 17 |
| PR003335 | PR002408 | PR001760 | C-terminus of heavy chain | H1_15-RT | Human IgG1 (L234A, L235A) | 17 |
| PR003336 | PR002408 | PR001830 | C-terminus of heavy chain | H1_15-RT | Human IgG1 (L234A, L235A) | 17 |
| PR003337 | PR002408 | PR001833 | C-terminus of heavy chain | H1_15-RT | Human IgG1 (L234A, L235A) | 17 |
| PR003338 | PR002408 | PR004469 | C-terminus of heavy chain | H1_15-RT | Human IgG1 (L234A, L235A) | 17 |
| PR004162 | PR002410 | PR001760 | C-terminus of heavy chain | H1_15-RT | Human IgG1 (L234A, L235A) | 17 |
| PR004357 | PR002410 | PR001840 | C-terminus of heavy chain | H1_15-RT | Human IgG1 (L234A, L235A, P329G) | 17 |
| PR004359 | PR002410 | PR001838 | C-terminus of heavy chain | H1_15-RT | Human IgG1 (L234A, L235A) | 17 |
| PR004280 | PR002408 | PR004469 | C-terminus of heavy chain | EPKSSD | Human IgG1 (L234A, L235A) | 6 |
| PR004281 | PR002408 | PR004469 | C-terminus of heavy chain | None | Human IgG1 (L234A, L235A) | 0 |
| PR004282 | PR002408 | PR004469 | C-terminus of heavy chain | GS_6 | Human IgG1 (L234A, L235A) | 6 |
| PR004995 | PR002410 | PR004469 | C-terminus of heavy chain | None | Human IgG1 (L234A, L235A) | 0 |
| PR005183 | PR002408 | PR004469 | C-terminus of heavy chain | None | Human IgG1 (L234A, L235A, P329G) | 0 |
| PR005184 | PR002408 | PR004469 | C-terminus of heavy chain | None | Human IgG1 (de1233-235) | 0 |
| PR005185 | PR002408 | PR004469 | C-terminus of heavy chain | None | Human hIgG4 (S228P, FALA) | 0 |
| PR005186 | PR002408 | PR004469 | C-terminus of heavy chain | None | Human IgG1 (L234A, G237A) | 0 |
| PR005187 | PR002408 | PR004469 | C-terminus of heavy chain | None | Human IgG1 (LALA, YTE) | 0 |
| PR005188 | PR002408 | PR004469 | C-terminus of heavy chain | None | Human IgG1 (LALA, DHS) | 0 |
| PR005827 | PR002408 | PR004469 | C-terminus of heavy chain | (G4S)2 | Human IgG1 (L234A, L235A) | 10 |
| PR005828 | PR002408 | PR004469 | C-terminus of heavy chain | GS_15 | Human IgG1 (L234A, L235A) | 15 |
| PR005829 | PR002408 | PR004469 | C-terminus of heavy chain | GS_20 | Human IgG1 (L234A, L235A) | 20 |
| PR005830 | PR002408 | PR004469 | C-terminus of heavy chain | GS_25 | Human IgG1 (L234A, L235A) | 25 |
| PR005838 | PR002408 | PR004469 | C-terminus of heavy chain | GS_6 | Human IgG1 (L234A, L235A, G237A) | 6 |
| PR005839 | PR002408 | PR004469 | C-terminus of heavy chain | GS_6 | Human IgG1 (L234A, G237A) | 6 |
| PR005866 | PR002408 | PR004469 | C-terminus of heavy chain | (G4S)2 | Human IgG1 (N297A) | 10 |
| PR007379 | PR002408 (H:gm) | PR004469 VH | C-terminus of heavy chain | (G2S)2 | Human IgG1 (L234A, L235A) | 6 |
| PR007380 | PR002408 (H:gm) | PR004469_ VH_gm | C-terminus of heavy chain | (G2S)2 | Human IgG1 (L234A, L235A) | 6 |

**Table 11-(C): B7-H4×4-1BB bispecific antibody molecules of an IgG-VH-VH hexavalent symmetric structure**

| **Bispecific antibody molecules** | **B7-H4 antibody (IgG)** | **4-1BB antibody (VH_B)** | **VH_B position relative to IgG** | **First linker peptide (between VH_B and IgG)** | **Second linker peptide (between VH_B and VH_B)** | **Fc type (mutation)** | **Length of first linker peptide** | **Length of second linker peptide** |
|---|---|---|---|---|---|---|---|---|
| PR003487 | PR002408 | PR001760 | C-terminus of heavy chain | H1_15-RT | None | Human IgG1 (L234A, L235A) | 17 | 0 |
| PR003488 | PR002408 | PR001760 | C-terminus of heavy chain | H1_15-RT | GS_5 | Human IgG1 (L234A, L235A) | 17 | 5 |
| PR003489 | PR002408 | PR001760 | C-terminus of heavy chain | H1_15-RT | GS_15 | Human IgG1 (L234A, L235A) | 17 | 15 |
| PR004158 | PR002410 | PR001760 | C-terminus of heavy chain | H1_15-RT | GS_5 | Human IgG1 (L234A, L235A) | 17 | 5 |
| PR004358 | PR002410 | PR001840 | C-terminus of heavy chain | H1_15-RT | GS_5 | Human IgG1 (L234A, L235A, P329G) | 17 | 5 |

**Table 11-(D): B7-H4×4-1BB bispecific antibody molecules of an Fab-HCAb tetravalent symmetric structure**

| **Bispecific antibody molecules** | **B7-H4 antibody (IgG)** | **4-1BB antibody (VH_B)** | **First linker peptide (between Fab and VH_B)** | **Second linker peptide (between VH_B and CH2)** | **Fc type (mutation)** | **Length of first linker peptide** | **Length of second linker peptide** |
|---|---|---|---|---|---|---|---|
| PR004279 | PR002408 | PR001760 | H1_15 | Human IgG1 hinge (C220S) | Human IgG1 (L234A, L235A) | 15 | 15 |
| PR005189 | PR002408 | PR001760 | None | Human IgG1 hinge (C220S) | Human IgG1 (L234A, L235A) | 0 | 15 |
| PR005190 | PR002408 | PR004469 | H1_15 | Human IgG1 hinge (C220S) | Human IgG1 (L234A, L235A) | 15 | 15 |
| PR005649 | PR002408 | PR001760 | None | Human IgG1 hinge (C220S) | Human IgG1 (L234A, L235A) | 0 | 15 |
| PR007165 | PR002408 | PR001760 | None | Human IgG1 hinge (C220S) | Human IgG1 (L234A, L235A) | 0 | 15 |

**Table 11-(E): B7-H4×4-1BB bispecific antibody molecules of an Fab-Fc-VH(n) asymmetric structure**

| **Bispecific antibody molecules** | **B7-H4 antibody (IgG)** | **4-1BB antibody (VH_B)** | **Number of repetition n for VH_B** | **First linker peptide** | **Second linker peptide** | **Fc type of Fab end (mutation)** | **Fc type of VH_B end (mutation)** | **Length of first linker peptide** | **Length of second linker peptide** |
|---|---|---|---|---|---|---|---|---|---|
| PR004160 | PR002410 | PR001760 | 2 | GS_15 | None | Human IgG1 (knob, LALA) | Human IgG1 (hole, LALA) | 15 | 0 |
| PR004161 | PR002410 | PR001760 | 3 | GS_15 | AS-GS_15 | Human IgG1 (knob, LALA) | Human IgG1 (hole, LALA) | 15 | 17 |
| PR004181 | PR002410 | PR001771 | 3 | GS_15 | GS_15 | Human IgG1 (knob, LALA) | Human IgG1 (hole, LALA) | 15 | 15 |
| PR004182 | PR002410 | PR001840 | 3 | GS_15 | GS_15 | Human IgG1 (knob, LALA) | Human IgG1 (hole, LALA) | 15 | 15 |

**Table 11-(F): B7-H4×4-1BB bispecific antibody molecules of an Fab-VH-Fc-VH structure**

| **Bispecific antibody molecules** | **B7-H4 antibody (IgG)** | **4-1BB antibody (VH_B)** | **First linker peptide** | **Second linker peptide** | **Fc type (mutation)** | **Length of first linker peptide** | **Length of second linker peptide** |
|---|---|---|---|---|---|---|---|
| PR005650 | PR002408 | PR001760 | None | None | Human IgG1 (L234A, L235A) | 0 | 0 |
| PR005651 | PR002408 | PR004469 | None | None | Human IgG1 (L234A, L235A) | 0 | 0 |

**Tables 12-(A)-(F) show the protein expression and physicochemical properties of the bispecific antibody molecules of the present invention.**

**Table 12-(A): Expression and physicochemical properties of bispecific antibody molecule proteins of an IgG-scFv symmetric structure**

| **Bispecific antibody molecules** | **Expression system and volume** | **Plasmid transfection ratio (short chain:long chain)** | **Yield (mg/L) after first purification** | **HPLC-SEC purity (%)** | **DSF Tm1 (°C)** |
|---|---|---|---|---|---|
| PR002789 | HEK293F (30ml) | 3:2 | 23 | 59 | 51.4 |
| PR002972 | HEK293F (30ml) | 3:2 | | 59 | 50.6 |

**Table 12-(B): Expression and physicochemical properties of bispecific antibody molecule proteins of an IgG-VH symmetric structure**

| **Bispecific antibody molecules** | **Expression system and volume** | **Plasmid transfection ratio (short chain:long chain)** | **Yield (mg/L) after first purification** | **HPLC-SEC purity (%)** |
|---|---|---|---|---|
| PR002790 | Expi293F (10ml) | 3:2 | 74 | 95 |
| PR002791 | Expi293F (10ml) | 3:2 | 60 | 95 |
| PR002792 | Expi293F (10ml) | 3:2 | 52 | 92 |
| PR002793 | Expi293F (10ml) | 3:2 | 49 | 92 |
| PR002794 | Expi293F (10ml) | 3:2 | 47 | 94 |
| PR002795 | Expi293F (10ml) | 3:2 | 69 | 93 |
| PR002798 | Expi293F (10ml) | 3:2 | 67 | 94 |
| PR002800 | Expi293F (10ml) | 3:2 | 73 | 85 |
| PR002801 | Expi293F (10ml) | 3:2 | 57 | 89 |
| PR002802 | Expi293F (10ml) | 3:2 | 33 | 76 |
| PR002804 | Expi293F (10ml) | 3:2 | 26 | 83 |
| PR002805 | Expi293F (10ml) | 3:2 | 3 | 83 |
| PR002806 | Expi293F (10ml) | 3:2 | 20 | 88 |
| PR002808 | Expi293F (10ml) | 3:2 | 16 | 71 |
| PR003334 | Expi-CHOs(200ml) | 3:2 | 38.2 | 98 |
| PR003335 | Expi-CHOs(200ml) | 3:2 | 68.9 | 99 |
| PR003336 | HEK293-F (30ml) | 3:2 | 66.47 | 91 |
| PR003337 | HEK293-F (30ml) | 3:2 | 91.41 | 93 |
| PR003338 | HEK293-F (30ml) | 3:2 | 74.6 | 98 |
| PR004162 | HEK293-F (30ml) | 3:2 | 57 | 44.81 |
| PR004357 | HEK293-F (30ml) | 3:2 | 66 | 47.53 |
| PR004359 | HEK293-F (30ml) | 3:2 | 81 | 96.82 |
| PR004280 | HEK293-F (30ml) | 3:2 | 39.5 | 98.63 |
| PR004281 | HEK293-F (30ml) | 3:2 | 28.5 | 98.73 |
| PR004282 | HEK293-F (30ml) | 3:2 | 34.25 | 99.09 |
| PR004995 | 293-6E(40ml) | 3:2 | 42 | 95.69 |
| PR005183 | Expi-CHOs(1000ml) | 3:2 | 89.6 | 99.916 |
| PR005184 | 293-6E(40ml) | 3:2 | 7.15 | 90.77 |
| PR005185 | 293-6E(40ml) | 3:2 | 7.425 | 89.1 |
| PR005186 | 293-6E(40ml) | 3:2 | 10.5 | 83.89 |
| PR005187 | 293-6E(40ml) | 3:2 | 8 | 97.86 |
| PR005188 | 293-6E(40ml) | 3:2 | 10.15 | 94.19 |
| PR005827 | HEK293-F (100ml) | 3:2 | 35.2 | 99.526 |
| PR005828 | HEK293-F (100ml) | 3:2 | 38.4 | 99.311 |
| PR005829 | HEK293-F (100ml) | 3:2 | 31.8 | 99.457 |
| PR005830 | HEK293-F (100ml) | 3:2 | 27.3 | 99.680 |
| PR005838 | Expi-CHOs(2000ml) | 3:2 | 11.5 | 98.833 |
| PR005839 | Expi-CHOs(2000ml) | 3:2 | 33.2 | 98.104 |
| PR005866 | Expi-CHOs(2000ml) | 3:2 | 5.3 | 98.241 |
| PR007379 | HEK293-F (40ml) | 3:2 | 33.5 | 98.625 |
| PR007380 | HEK293-F (40ml) | 3:2 | 38.0 | 98.453 |

**Table 12-(C): Expression and physicochemical properties of bispecific antibody molecule proteins of an IgG-VH-VH hexavalent symmetric structure**

| **Bispecific antibody molecules** | **Expression system and volume** | **Plasmid transfection ratio (short chain:long chain)** | **Yield (mg/L) after first purification** | **HPLC-SEC purity (%)** |
|---|---|---|---|---|
| PR003487 | HEK293-F (30ml) | 3:2 | 19 | 84 |
| PR003488 | HEK293-F (30ml) | 3:2 | 17.39 | 85 |
| PR003489 | HEK293-F (30ml) | 3:2 | 16.03 | 76 |
| PR004158 | HEK293-F (30ml) | 3:2 | 31.92 | 63 |
| PR004358 | HEK293-F (30ml) | 3:2 | 18.9 | 98.72 |

**Table 12-(D): Expression and physicochemical properties of bispecific antibody molecule proteins of an Fab-HCAb tetravalent symmetric structure**

| **Bispecific antibody molecules** | **Expression system and volume** | **Plasmid transfection ratio (short chain:long chain)** | **Yield (mg/L) after first purification** | **HPLC-SEC purity (%)** |
|---|---|---|---|---|
| PR004279 | HEK293-F (30ml) | 3:2 | 11.375 | 79.18 |
| PR005189 | HEK293-F (100ml) | 3:2 | 4 | 97.59 |
| PR005649 | HEK293-F (100ml) | 3:2 | 30.4 | 91.928 |
| PR007165 | HEK293-F (100ml) | 3:2 | 39 | 94.70 |

**Table 12-(E): Expression and physicochemical properties of bispecific antibody molecule proteins of an Fab-Fc-VH(n) asymmetric structure**

| **Bispecific antibody molecules** | **Expression system and volume** | **Plasmid transfection ratio (short chain:long chain)** | **Yield (mg/L) after first purification** | **HPLC-SEC purity (%)** | **DSF Tm1 (°C)** |
|---|---|---|---|---|---|
| PR004160 | HEK293-F (30ml) | 1:1:1 | 29.36 | 89.41 | 64.4 |
| PR004161 | HEK293-F (30ml) | 1:1:1 | 31.62 | 85.47 | 63.0 |
| PR004181 | HEK293-F (30ml) | 1:1:1 | 87.25 | 87.19 | 64.2 |
| PR004182 | HEK293-F (30ml) | 1:1:1 | 24.75 | 79.14 | 51 |

**Table 12-(F): Expression and physicochemical properties of bispecific antibody molecule proteins of an Fab-VH-Fc-VH symmetric structure**

| **Bispecific antibody molecules** | **Expression system and volume** | **Plasmid transfection ratio (short chain:long chain)** | **Yield (mg/L) after first purification** | **HPLC-SEC purity (%)** |
|---|---|---|---|---|
| PR005650 | HEK293-F (100ml) | 3:2 | 21.5 | 97.476 |
| PR005651 | HEK293-F (100ml) | 3:2 | 18.2 | 98.129 |

### Example 5. FACS Assays for In Vitro Binding of B7-H4×4-1BB Bispecific Antibodies on CHO-K1 Cell Strain Overexpressing Human and Cynomolgus Monkey 4-1BB

This example is intended to investigate the *in vitro* binding activity of the 4-1BB arms of B7-H4×4-1BB bispecific antibodies to human and cynomolgus monkey 4-1BB. Antibody binding experiments at the cellular level were conducted using a CHO-K1 cell strain overexpressing human 4-1BB (CHO-K1/hu 4-1BB, Genscript, #M00538) and a CHO-K1 cell strain overexpressing cynomolgus monkey 4-1BB (CHO-K1/cyno 4-1BB, Genscript, #M00569). Briefly, the CHO-K1-hu 4-1BB and CHO-K1/cyno 4-1BB cells were digested, resuspended in F12K complete medium, and washed once with PBS. The cell density was adjusted to 1×10⁶ cells/mL with PBS. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor 488-conjugated AffiniPure Goat Anti-Human IgG, Fcy Fragment Specific, Jackson ImmunoResearch, #: 109-545-06, 1:500 diluted) or (Alexa Fluor^{®} 647, Goat Anti-Human IgG, Fcy fragment specific, Jackson ImmunoResearch, #: 109-605-098, 1: 1000 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII or an ACEA NovoCyte flow cytometer.

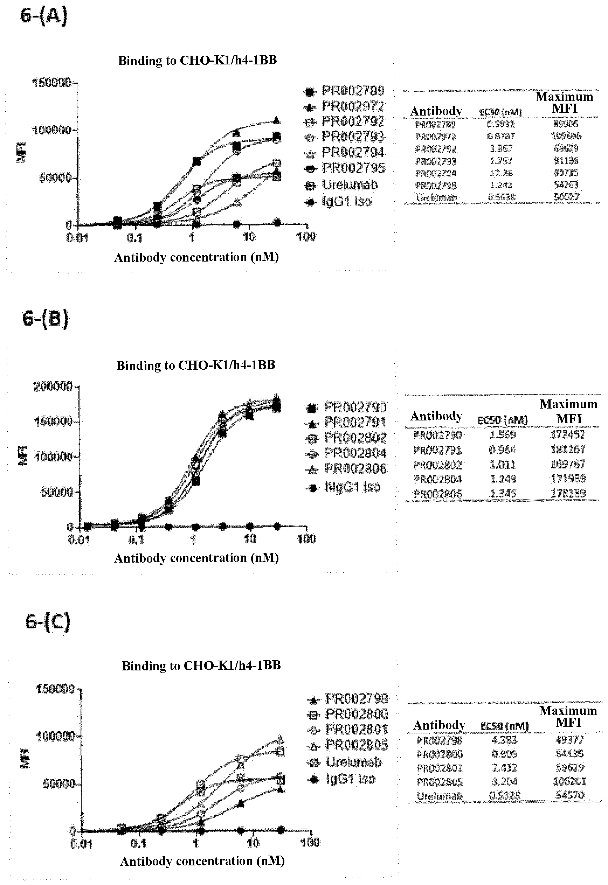

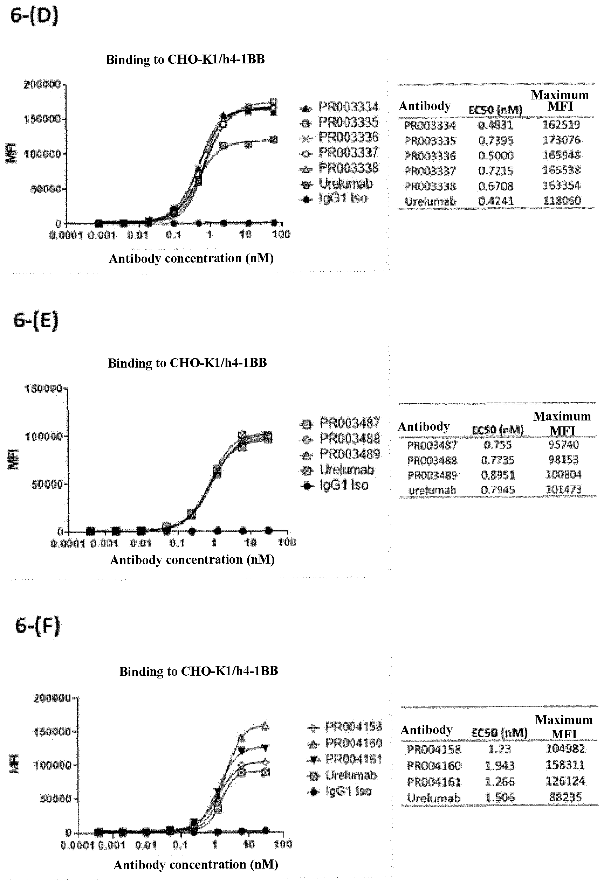

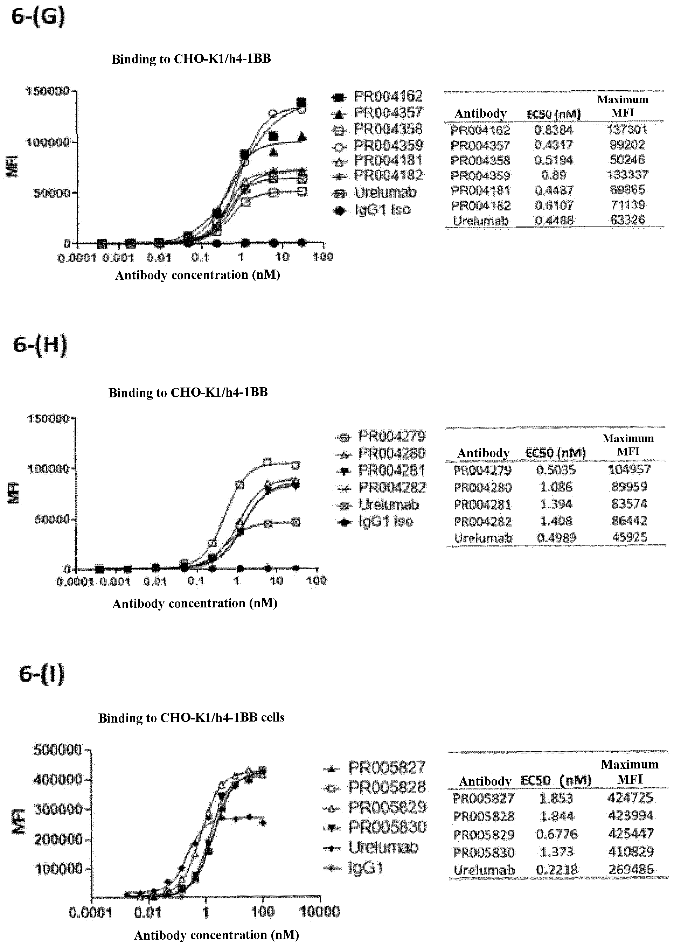

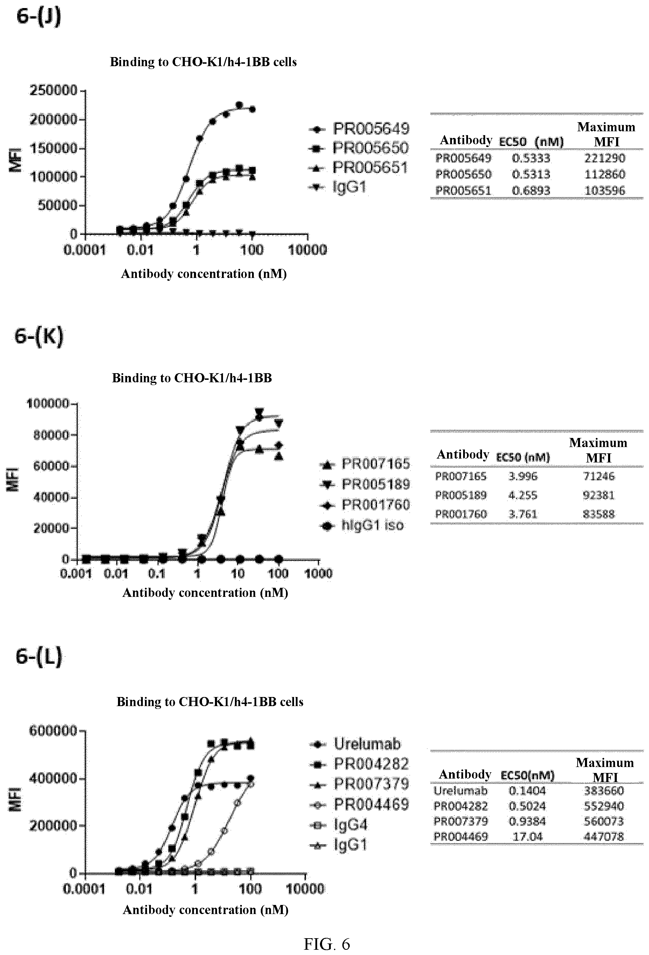

FIGs. 6-(A)-(L) show the *in vitro* binding of B7-H4×4-1BB bispecific antibodies on the CHO-K1 cell strain overexpressing human 4-1BB.

FIGs. 7-(A)-(C) show the *in vitro* binding of B7-H4×4-1BB bispecific antibodies on the CHO-K1 cell strain overexpressing cynomolgus monkey 4-1BB.

As shown in FIGs. 6-(A)-(L), all the B7-H4×4-1BB bispecific antibodies in this example specifically bound to the CHO-K1 cells overexpressing human 4-1BB. PR002789, PR002972, PR003334, PR003335, PR003336, PR003337, PR003338, PR004160, PR004161, PR004158, PR004162, PR004357, PR004359, PR004279, PR004280, PR004281, PR004282, PR005829, etc., bound to human 4-1BB more strongly than the positive control urelumab. Specifically, the Span value of the binding curve of the antibody PR003335 was about 1.5 times that of urelumab, and the maximum MFI was greater than that of urelumab.

As shown in FIGs. 7-(A)-(C), all the B7-H4×4-1BB bispecific antibodies in this example specifically bound to the CHO-K1 cells overexpressing monkey 4-1BB. However, the control antibody urelumab had no cross-binding activity to monkey 4-1BB.

### Example 6. FACS Assays for In Vitro Binding of B7-H4×4-1BB Bispecific Antibodies on SK-BR-3 Cell Line Highly Expressing B7-H4

This example is intended to investigate the binding activity of the B7-H4 arms of B7-H4×4-1BB bispecific antibodies to human B7-H4. Experiments of binding to human B7-H4 at the cellular level were conducted using an SK-BR-3 cell line highly expressing B7-H4. Briefly, SK-BR-3 cell suspensions were collected and the cell densities were adjusted to 2×10⁶ cells/mL. The cells were seeded in a 96-well V-bottom plate (Corning, #3894) at 50 µL/well,followed by the addition of test antibodies diluted in a 3-fold gradient at a concentration that was 2 times the final concentration, each at 50 µL/well. The cells were incubated at 4 °C for 2 h away from light. Thereafter, the cells in each well were rinsed twice with 100 µL of pre-cooled PBS, and centrifuged at 500g at 4 °C for 5 min, and then the supernatant was discarded. Then 100 µL of a fluorescent secondary antibody (Alexa Fluor 647-conjugated AffiniPure Goat Anti-Human IgG, Fcy Fragment Specific, Jackson ImmunoResearch, #: 109-605-098, 1:1000 diluted) was added to each well. The plate was incubated away from light at 4 °C for 1 h. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled FACS, and the fluorescence signal values were read using ACEA Novocyte3000.

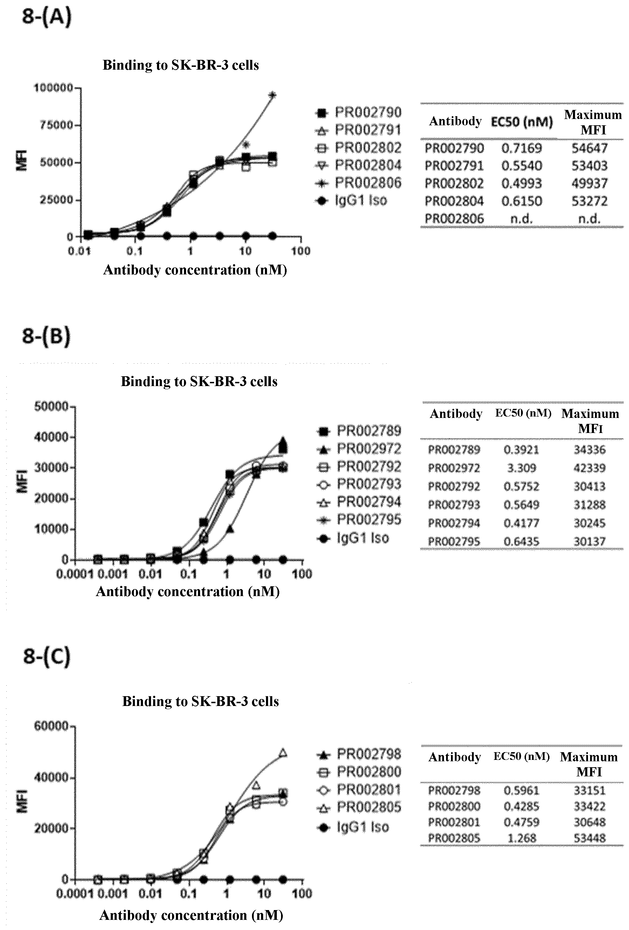

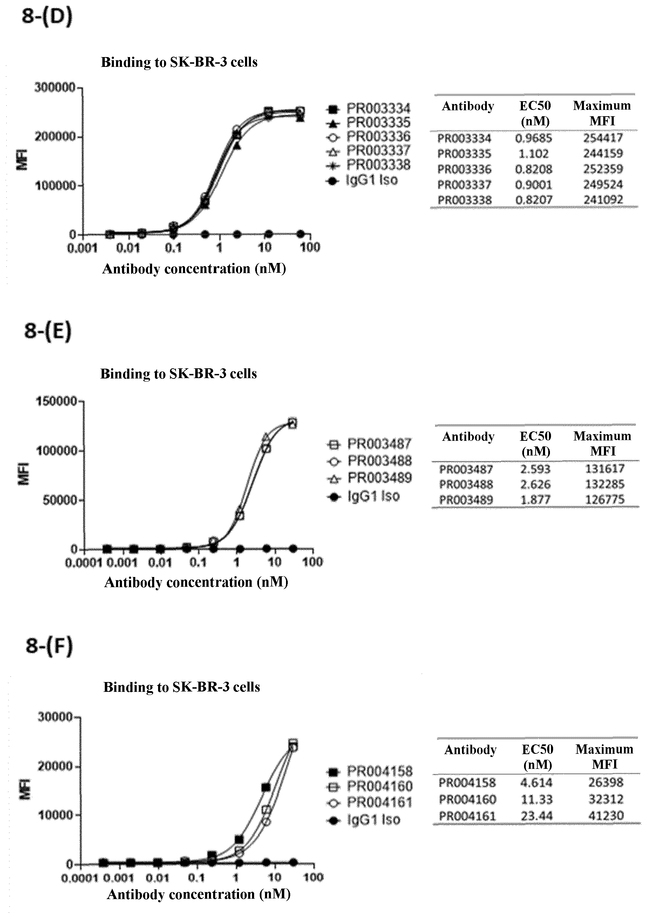

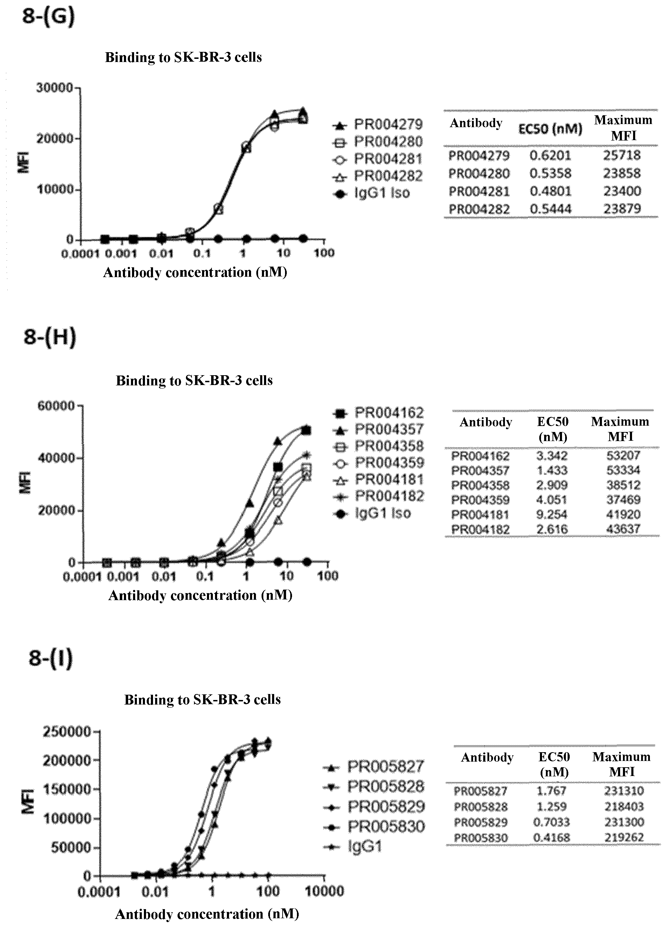

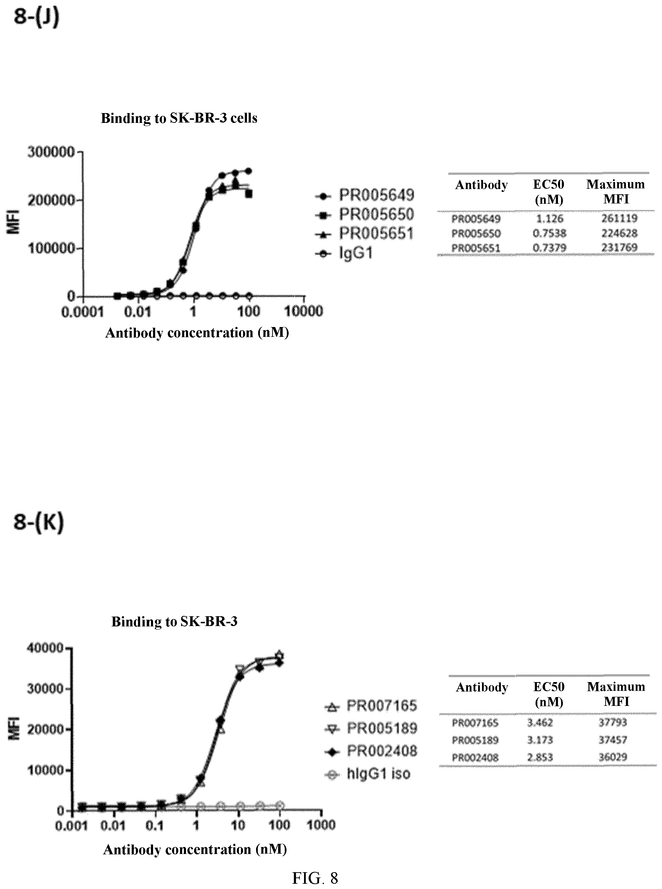

FIGs. 8-(A)-(K) show the *in vitro* binding of B7-H4×4-1BB bispecific antibodies on the SK-BR-3 cell line highly expressing B7-H4.

As shown in FIGs. 8-(A)-(K), all the B7-H4×4-1BB bispecific antibodies in this example specifically bound to human B7-H4 on the SK-BR-3 cell line, with binding curve EC₅₀ values of mostly 0.1-10 nM.

### Example 7. T Cell Activation Experiments for B7-H4×4-1BB Bispecific Antibodies in the Presence of SK-BR-3 Cell Line Highly Expressing B7-H4, and Release of Cytokines.

To study the ability of B7-H4×4-1BB bispecific antibodies to mediate activation of target T cells *in vitro,* activation experiments were conducted *in vitro* using human pan T cells as effector cells and an SK-BR-3 cell line highly expressing B7-H4 as crosslinking-mediating cells, and the release of cytokines was determined. Specifically, a 96-well flat-bottom plate (Corning, #3599) was coated with OKT3; the density of pan T cells was adjusted to 2×10⁶ cells/mL, and the density of SK-BR-3 cells was adjusted to 2×10⁵ cells/mL; each of the two cell suspensions was seeded into the 96-well flat-bottom plate (Corning, #3599) at 50 µL/well,and then 50 µL of test antibodies serially diluted 3-fold with concentrations that were 3 times the final concentrations were added to each well, with the highest final concentration of the antibodies being 100 nM, 30 nM, 20 nM or 6 nM; 6, 3 or 2 concentrations were set for each antibody, the final effector-to-target ratio was 10:1, and two replicates were set. Meanwhile, an isotype IgG control group was set in the plate. The 96-well plate was incubated in a carbon dioxide incubator at 37 °C for 2 or 3 days. After incubation, the supernatant was collected and added to a 96-well plate (Corning, #3599), and centrifuged at 500 g at 4 °C for 5 min. The supernatant was incubated for 2 days and then analyzed for the release of cytokine IL-2; or incubated for 3 days and then analyzed for the release of IFN-gamma. The instructions of the IL-2 (IL-2 Human Uncoated ELISA Kit, Thermo, #88-7025-88) kit and IFN gamma (IFN gamma Human Uncoated ELISA Kit, Thermo, #88-7316-88) were referred to for the ELISA method.

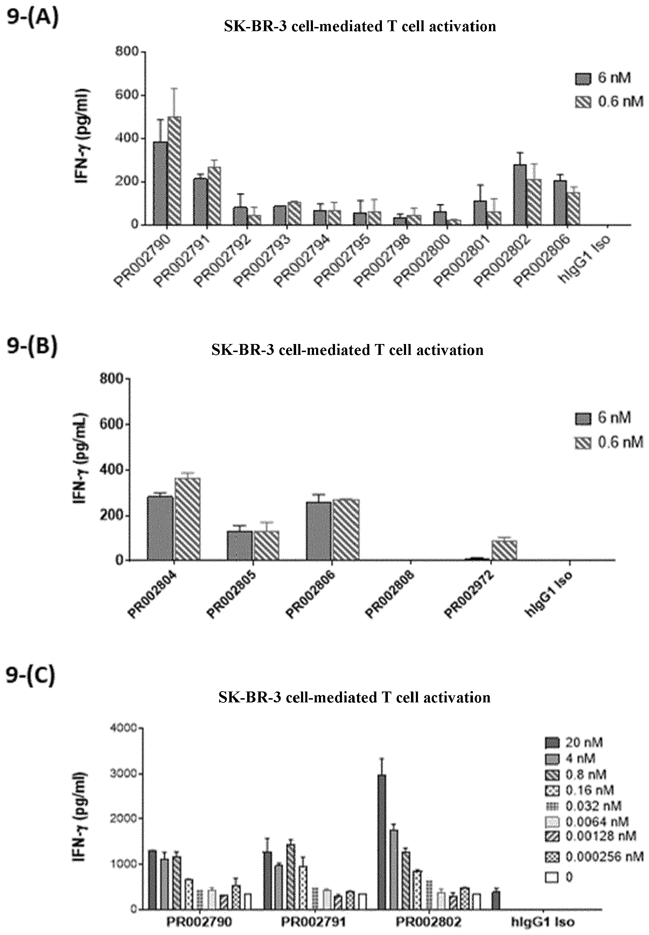

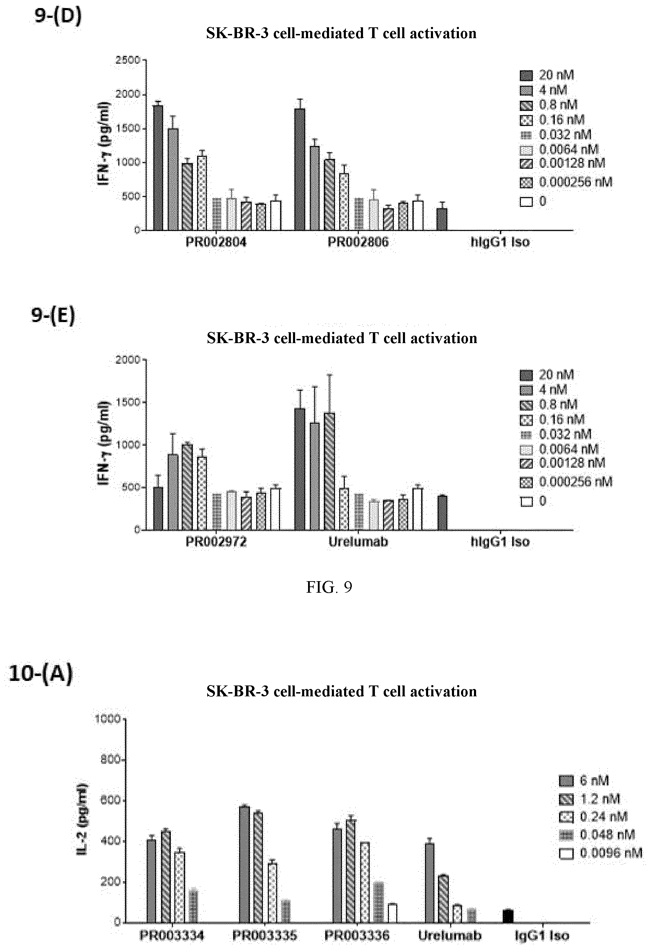

FIGs. 9-(A)-(E) show the activation of the 4-1BB pathway and the induction of T cell activation to release IFN-γ by B7-H4×4-1BB bispecific antibodies in the presence of the SK-BR-3 cell line highly expressing B7-H4.

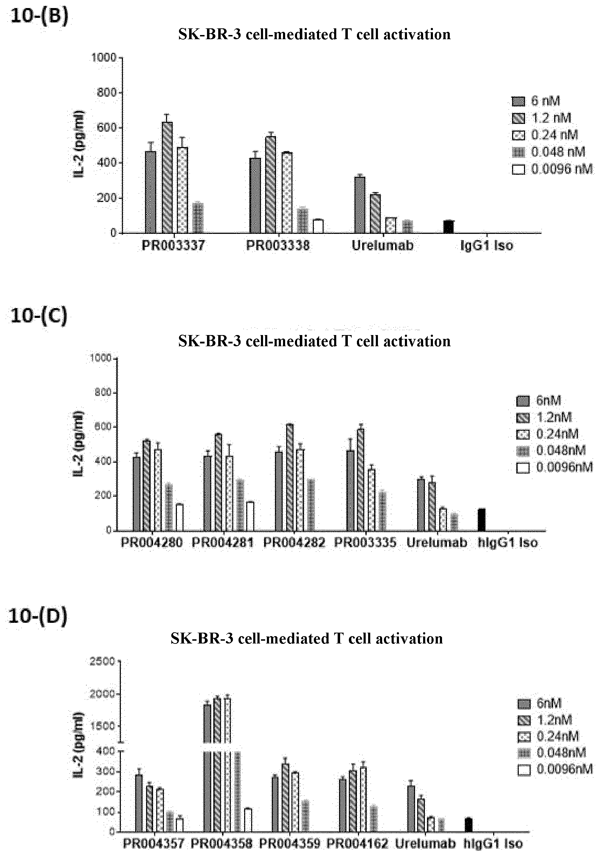

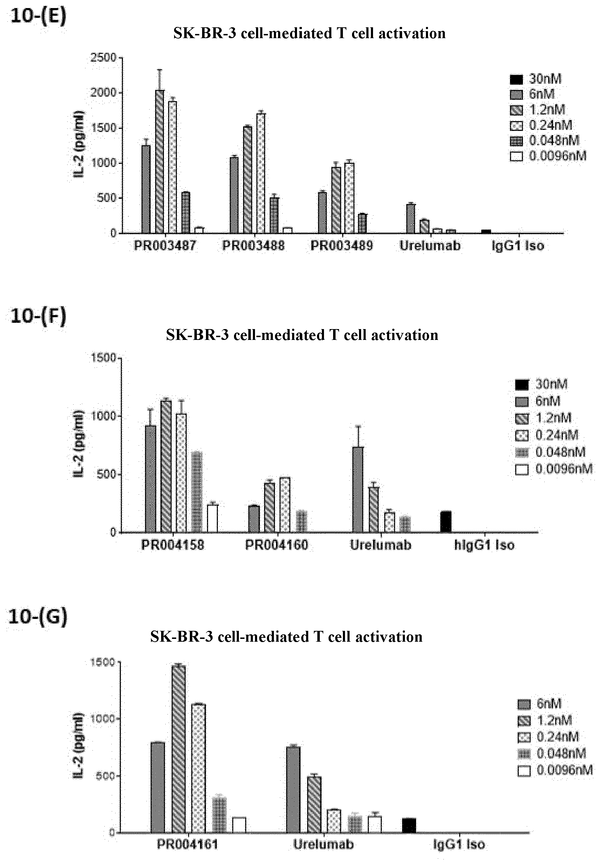

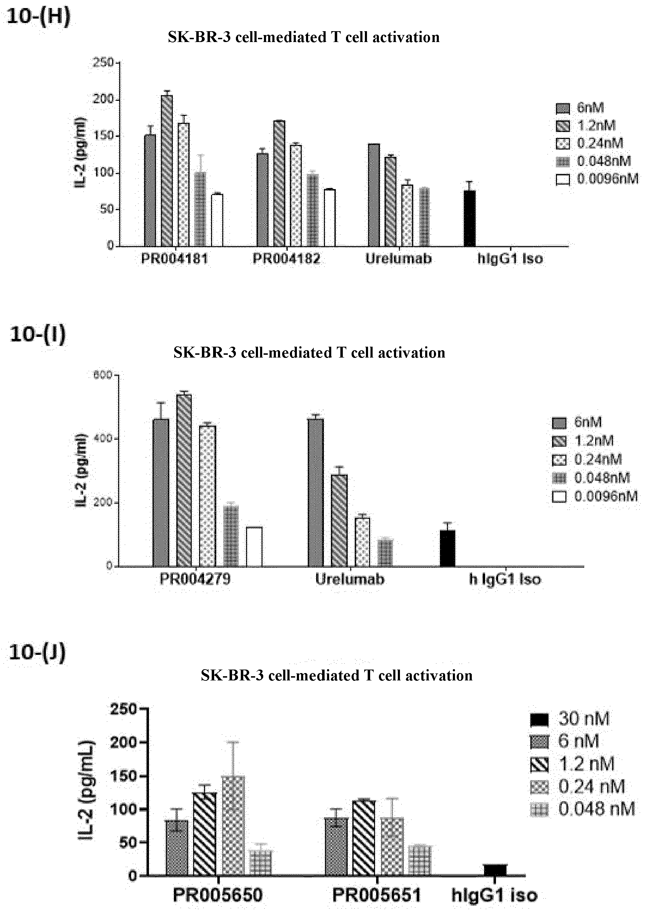

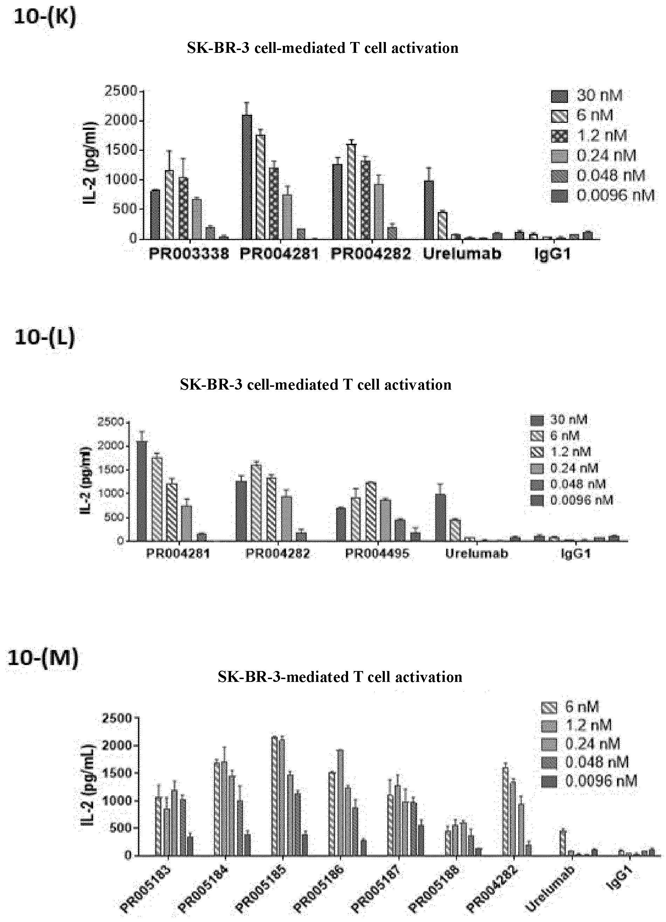

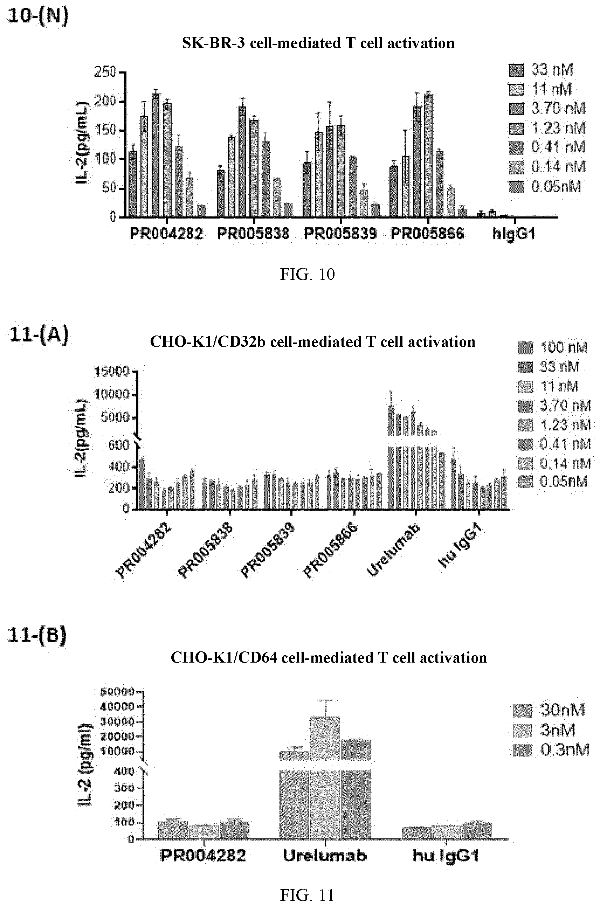

FIGs. 10-(A)-(N) show the activation of the 4-1BB pathway and the induction of T cell activation to release IL-2 by B7-H4×4-1BB bispecific antibodies in the presence of the SK-BR-3 cell line highly expressing B7-H4.

As shown in FIGs. 9-(A)-(E) and 10-(A)-(N), the B7-H4×4-1BB bispecific antibodies can mediate T cell activation in the presence of the SK-BR-3 cell line highly expressing B7-H4. In addition, the activation of the 4-1BB pathway by PR002790, PR002791, PR002802, PR002804, PR002806, PR003334, PR003335, PR003336, PR003337, PR003338, PR003487, PR003488, PR003489, PR004158, PR004160, PR004161, PR004162, PR004181, PR004182, PR004279, PR004280, PR004281, PR004282, PR004357, PR004358 and PR004359 was greater than or equivalent to that by urelumab. Specifically, more cytokine IFN-γ or IL-2 was produced under the action of these bispecific antibodies than under the action of urelumab.

The linker also affected the bispecific antibodies' activation of T cells when varying in length; for example, PR004281 and PR004282 performed better than PR003338 and urelumab (FIG. 10-K).

The B7H4 arm affected the bispecific antibodies' activation of T cells when comprising different antigenic epitopes; for example, PR004281 and PR004282 performed better than PR004995 and urelumab (FIG. 10-L).

The bispecific antibodies' activation of T cells was affected when different Fc mutation sites were comprised; for example, PR005185, PR005186 and PR004282 were superior to urelumab (FIG. 10-M).

### Example 8. Activation of T Cells by B7-H4×4-1BB Bispecific Antibodies Being Independent of FcγR Cells

To investigate whether the activity of the B7-H4×4-1BB bispecific antibodies was dependent on the expression of FcyR, activation experiments were conducted *in vitro* using human pan T cells as effector cells and a CHO-K1/CD32b cell line highly expressing FcyRIIb or a CHO-K1/CD64 cell line highly expressing FcyRI as crosslinking-mediating cells, and the release of cytokines was determined. Specifically, a 96-well flat-bottom plate (Corning, #3599) was coated with OKT3; the density of pan T cells was adjusted to 2×10⁶ cells/mL, and the density of FcγR cells was adjusted to 2×10⁵ cells/mL; each of the two cell suspensions was seeded into the 96-well flat-bottom plate (Corning, #3599) at 50 µL/well,and then 100 µL of test antibodies serially diluted 2-fold with a concentration that was twice the final concentration were added to each well; the final effector-to-target ratio was 10:1, and two replicates were set. Meanwhile, an isotype IgG control group was set in the plate. The 96-well plate was incubated in a carbon dioxide incubator at 37 °C for 2 days. After incubation, 100 µL of supernatant was collected and added to a 96-well plate (Corning, #3894), and centrifuged at 500 g at 4 °C for 5 min. The supernatant was collected and analyzed for the release of cytokine IL-2. The instructions of the IL-2 (IL-2 Human Uncoated ELISA Kit, Thermo, #88-7025-88) kit were referred to for the ELISA method.

FIGs. 11-(A)-(B) show the activation of T cells by B7-H4×4-1BB bispecific antibodies being specifically independent of the expression of FcyR. (A) The B7-H4×4-1BB bispecific antibody PR004282 was comparable to the isotype IgG control in mediating T cell activation in the presence of the cell line CHO-K1/CD32b cells highly expressing FcyRIIb. (B) The B7-H4×4-1BB bispecific antibody PR004282 was comparable to the isotype IgG control in mediating T cell activation in the presence of the cell line CHO-K1/ CD64 cells highly expressing FcyRI.

### Example 9. Activation of T Cells by B7-H4×4-1BB Bispecific Antibodies Being Dependent on Expression of B7-H4

To investigate whether the activity of the B7-H4×4-1BB bispecific antibodies was dependent on the expression of B7-H4, activation experiments were conducted *in vitro* using human pan T cells as effector cells and cells of cell lines SK-BR-3 and MDA-MB-468 highly expressing B7-H4 and COV644, JIMT-1 and MDA-MB-231 cells not expressing B7-H4 as crosslinking-mediating cells, and the release of cytokines was determined. Specifically, a 96-well flat-bottom plate (Corning, #3599) was coated with OKT3; the density of pan T cells was adjusted to 2×10⁶ cells/mL, and the density of tumor cells was adjusted to 2×10⁵ cells/mL; each of the two cell suspensions was seeded into the 96-well flat-bottom plate (Corning, #3599) at 50 µL/well, and then 50 µL of test antibodies with a concentration that was 3 times the final concentration were added to each well; the final effector-to-target ratio was 10:1, and two replicates were set. Meanwhile, an isotype IgG control group was set in the plate. The 96-well plate was incubated in a carbon dioxide incubator at 37 °C for 2 days. After incubation, 100 µL of supernatant was collected and added to a 96-well plate (Corning, #3894), and centrifuged at 500 g at 4 °C for 5 min. The supernatant was collected and analyzed for the release of cytokine IL-2. The instructions of the IL-2 (IL-2 Human Uncoated ELISA Kit, Thermo, #88-7025-88) kit were referred to for the ELISA method.

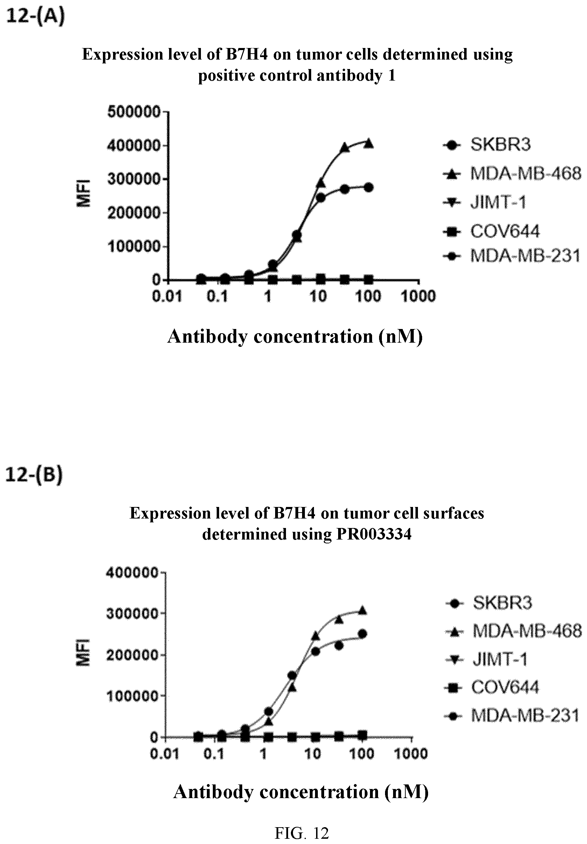

FIG. 12 shows the expression levels of B7-H4 on tumor cell surfaces determined by FACS. FIG. 12-(A) shows the expression levels of B7-H4 on tumor cell surfaces determined using B7-H4 positive control antibody 1. FIG. 12-(B) shows the expression levels of B7-H4 on tumor cell surfaces determined using a B7-H4×4-1BB bispecific antibody. As shown in the drawing, SK-BR-3 and MDA-MB-468 were tumor cells that highly expressed B7-H4, and JIMT-1, COV644 and MDA-MB-231 were tumor cells that did not express B7-H4.

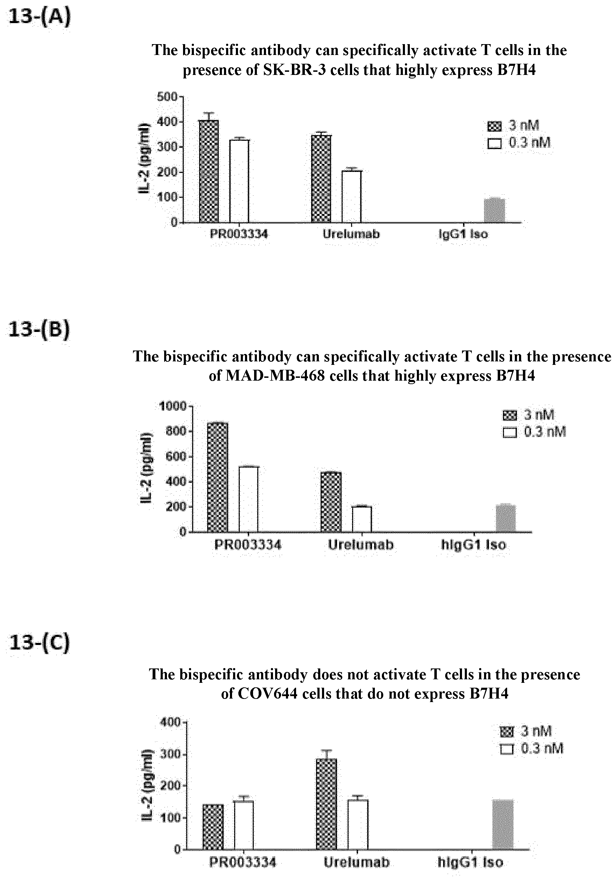

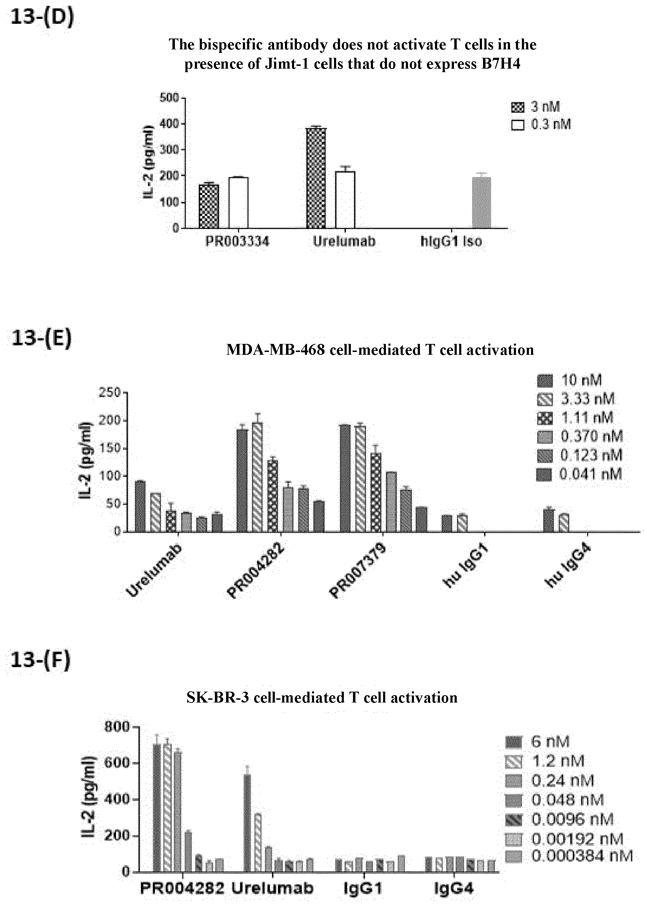

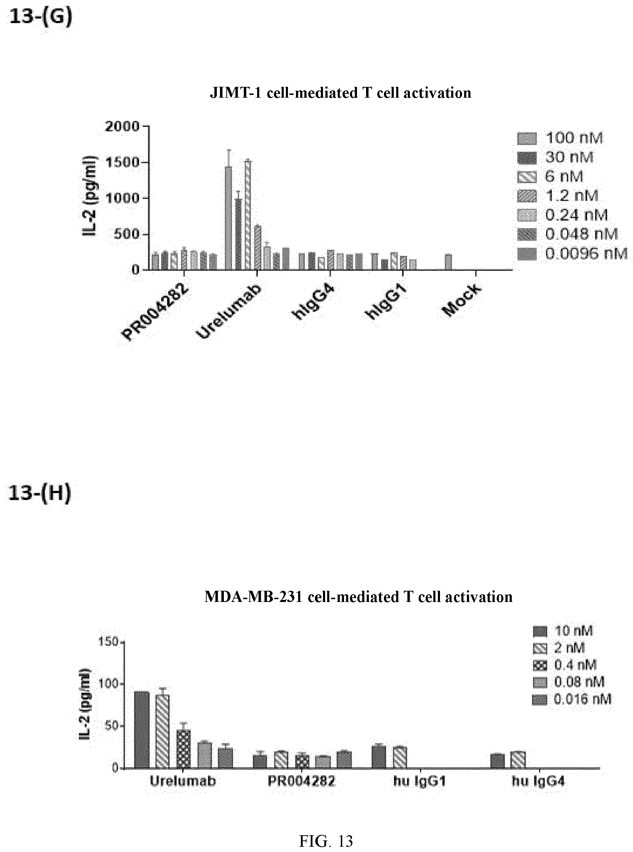

FIGs. 13-(A)-(H) show the activation of T cells by B7-H4×4-1BB bispecific antibodies being specifically dependent on the expression of B7-H4. (A) and (B) The B7-H4×4-1BB bispecific antibody PR003334 was superior to the positive control in mediating T cell activation in the presence of the cell line SK-BR-3 or MDA-MB-468 cells highly expressing B7-H4. (C) and (D) PR003334 was comparable to the isotype IgG control in mediating T cell activation in the presence of the cell line COV644 or JIMT-1 cells not expressing B7-H4. (E) and (F) The B7-H4×4-1BB bispecific antibody PR004282 was superior to the positive control in mediating T cell activation in the presence of the cell line SK-BR-3 cells highly expressing B7-H4. (G) and (H) PR004282 was comparable to the isotype IgG control in mediating T cell activation in the presence of the cell line JIMT-1 or MDA-MB-231 cells not expressing B7-H4.

### Example 10. Study on Serum Stability of B7-H4×4-1BB Bispecific Antibodies

To investigate the stability of B7-H4×4-1BB bispecific antibodies in human serum with high concentration, PR0003334 and PR0003335 were serially diluted 1:3 with 90% human serum from an initial concentration of 100 nM to obtain 8 concentration points. Six samples were prepared, incubated at 37 °C for 0 days, 1 day, 2 days, 4 days, 7 days and 14 days, respectively, then snap frozen in liquid nitrogen, and stored at -70 °C. After the B7-H4×4-1BBbispecific antibodies were stored in high-concentration serum at 37 °C for different periods of time, their *in vitro* binding to the SK-BR-3 cell line and CHO-K1-hu4-1BB cells highly expressing B7-H4 was determined by FACS, as in Example 3.3 and Example 6.

PR0004282 was serially diluted with 95% human serum and divided into 6 tubes, which were incubated at 37 °C for 0 days, 1 day, 2 days, 4 days, 7 days and 14 days, respectively, then snap frozen in liquid nitrogen, and stored at -80 °C. After PR004282 was stored in high-concentration human serum at 37 °C for different periods of time, its *in vitro* binding to B7H4+ cells and 4-1BB+ cells was determined by FACS as follows.

CHO-K1/h4-1BB, CHO-K1/cyno4-1BB, CHO-K1/hB7H4 and CHO-K1/cynoB7H4 cells were digested, resuspended in DMEM complete medium, and washed with PBS. Then the cell density was adjusted to 1×10⁶ cells/mL. The cells were seeded in a 96-well V-bottom plate (Corning, #3894) at 100 µL/well, followed by the addition of the antibody PR004282 serially diluted 3-fold with a concentration that was twice the final concentration and treated with test human serum, each at 100 µL/well (each dose of the antibody treated for a different period of time contained 4.5% human serum). The cells were incubated at 4 °C for 2 h away from light. Then, the cells in each well were rinsed twice with 100 µL of pre-cooled PBS containing 2% FBS, and centrifuged at 500 g at 4 °C for 5 min, and then the supernatant was discarded. Then 100 µL of a fluorescent secondary antibody (Alexa Fluor 647-conjugated AffiniPure Goat Anti-Human IgG, Fcγ Fragment Specific, Jackson, #109-545-06, 1:1000 diluted) was added to each well. The plate was incubated away from light at 4 °C for 1 h. The cells in each well were rinsed twice with 100 µL of pre-cooled PBS containing 2% FBS, and centrifuged at 500 g at 4 °C for 5 min, and then the supernatant was discarded. Finally, the cells in each well were resuspended in 200 µL of pre-cooled PBS containing 2% FBS, and the fluorescence signal values were read using an ACEA NovoCyte flow cytometer.

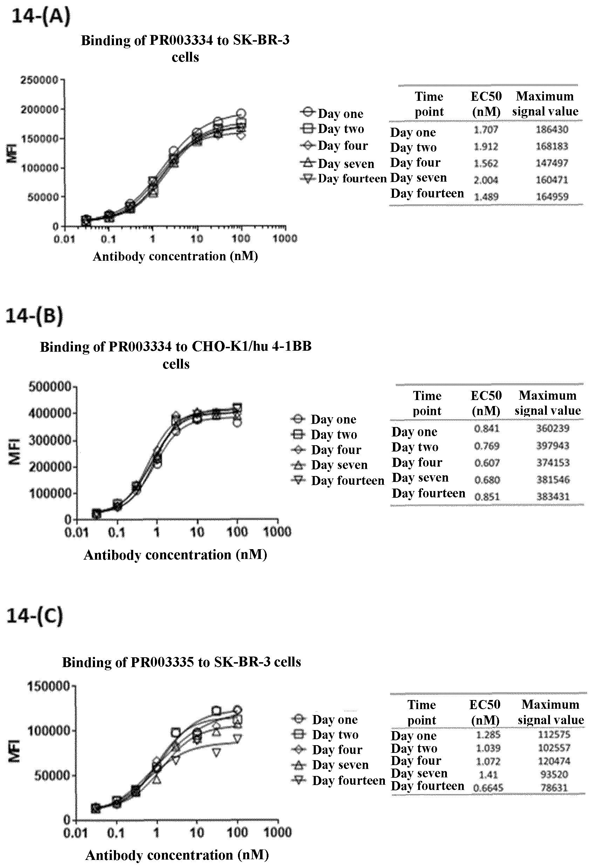

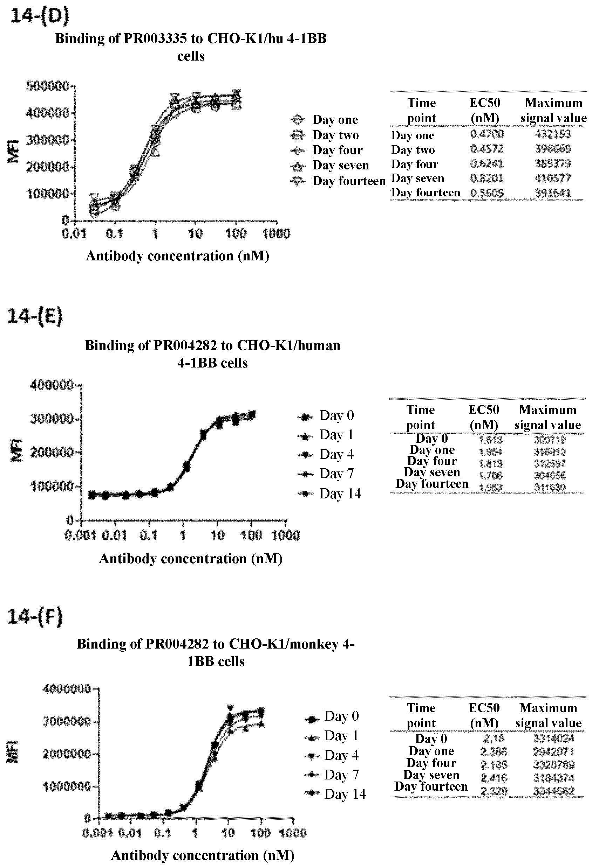

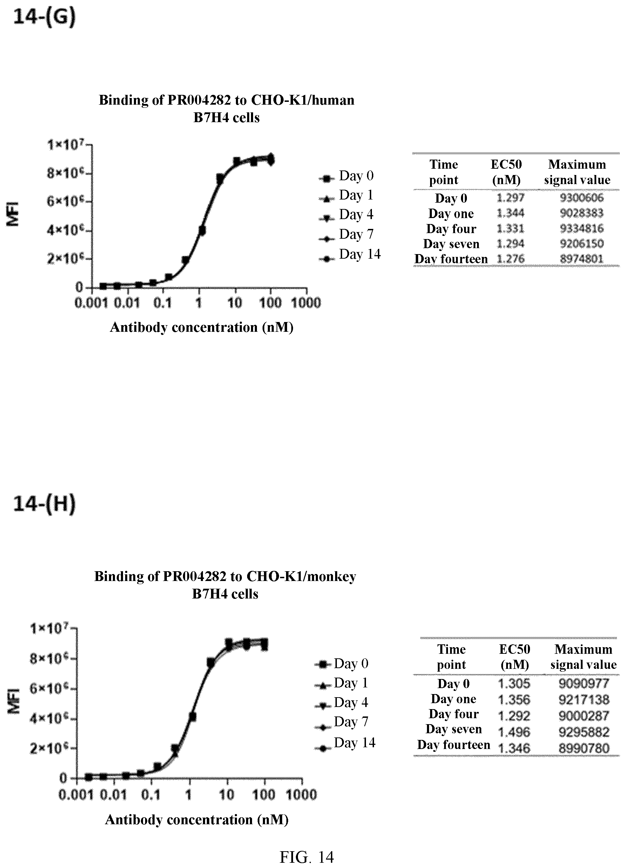

FIGs. 14-(A-D) show the serum stability of B7-H4×4-1BB bispecific antibodies PR003334 and PR003335. After PR003334 and PR003335 were incubated in human serum for different periods of time, there were no changes in their binding to the SK-BR-3 cells and CHO-K1-human 4-1BB cells highly expressing B7-H4 in 90% serum, indicating that PR003334 and PR003335 have good serum stability.

FIGs. 14-(E-H) show the serum stability of B7-H4×4-1BB bispecific antibody PR004282. After PR004282 was incubated in human serum for different periods of time, there were no changes in its binding to the CHO-K1 cells highly expressing B7H4 or 4-1BB in 95% serum, indicating that PR004282 has good serum stability.

### Example 11. FACS Assays for Binding Ability of B7-H4×4-1BB Bispecific Antibody PR004282 at 4-1BB+ Cell Level

This example is intended to investigate the *in vitro* binding activity of the B7-H4×4-1BB bispecific antibody PR004282 to human/cynomolgus monkey/mouse 4-1BB. Antibody binding experiments at the cellular level were conducted using a CHO-K1 cell strain overexpressing human 4-1BB (CHO-K1/h4-1BB, Gensript, #M00538), a CHO-K1 cell strain expressing cynomolgus monkey 4-1BB (CHO-K1/cyno4-1BB, Genscript, #M00569) and a CHO-K1 cell strain overexpressing mouse 4-1BB (CHO-K1/m4-1BB, Genscript, #M00568). Briefly, the CHO-K1/h4-1BB cells, CHO-K1/cyno 4-1BB cells and CHO-K1/m 4-1BB cells were digested and resuspended with PBS containing 2% FBS. The cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 2 h away from light. Thereafter, the cells in each well were rinsed twice with 100 µL of a pre-cooled FACS buffer, PBS containing 2% FBS, and centrifuged at 500 g at 4 °C for 5 min, and then the supernatant was discarded. Then 100 µL of a fluorescent secondary antibody (Alexa Fluor^{®} 647, Goat Anti-Human IgG, Fcγ fragment specific, Jackson ImmunoResearch, #109-605-098, 1: 1000 diluted) was added to each well. The plate was incubated away from light at 4 °C for 1 h. The cells in each well were rinsed twice with 100 µL of pre-cooled FACS buffer, and centrifuged at 500 g at 4 °C for 5 min, and then the supernatant was discarded. Finally, the cells in each well were resuspended in 200 µL of pre-cooled FACS buffer, and the fluorescence signal values were read using an ACEA Novocyte3000 flow cytometer.

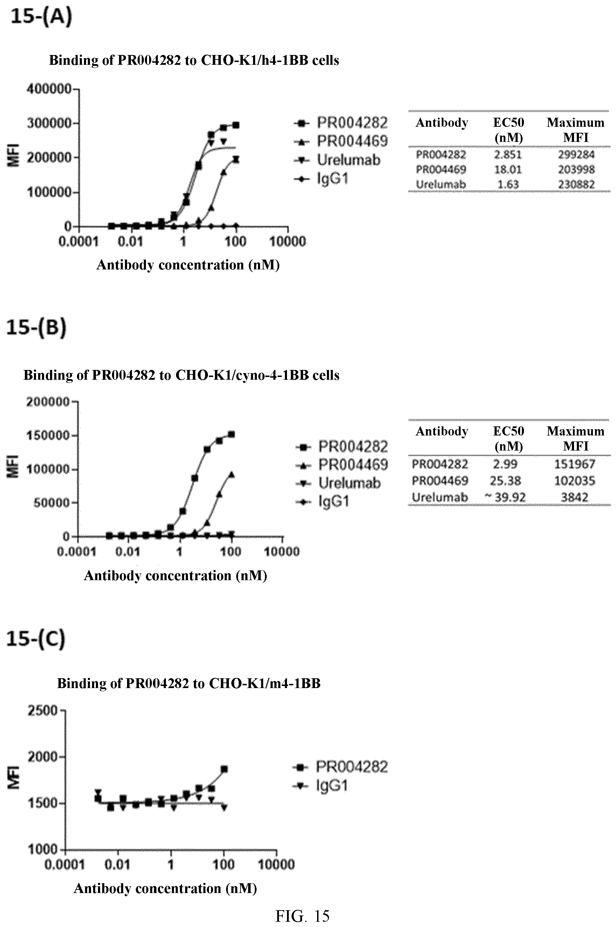

FIG. 15-(A) shows the *in vitro* binding of the B7-H4/4-1BB bispecific antibody PR004282 on the CHO-K1 cell line highly expressing human 4-1BB; PR004282 showed higher binding activity to the CHO-K1/h4-1BB cell line than the control antibody urelumab.

FIG. 15-(B) shows the *in vitro* binding of PR004282 on the CHO-K1 cell line highly expressing cynomolgus monkey 4-1BB; PR004282 can bind to monkey 4-1BB, while urelumab has no cross-reactivity with monkeys.

FIG. 15-(C) shows the *in vitro* binding of PR004282 on the CHO-K1 cell line highly expressing mouse 4-1BB; PR004282 does not have the ability to bind to mouse 4-1BB.

### Example 12. FACS Assays for Binding Ability of B7-H4×4-1BB Bispecific Antibody PR004282 at B7H4+ Cell Level

This example is intended to investigate the *in vitro* binding activity of the B7-H4×4-1BB bispecific antibody PR004282 to human/cynomolgus monkey/mouse B7-H4. Antibody binding experiments at the cellular level were conducted using a CHO-K1 cell strain overexpressing human B7-H4 (CHO-K1/hB7-H4, prepared in-house by Harbour Biomed), a CHO-K1 cell strain expressing cynomolgus monkey B7-H4 (CHO-K1/cynoB7-H4, prepared in-house by Harbour Biomed) and a CHO-K1 cell strain overexpressing mouse B7-H4 (CHO-K1/mB7-H4, prepared in-house by Harbour Biomed). Briefly, the CHO-K1/hB7H4 cells, CHO-K1/cyno B7-H4 cells and CHO-K1/mB7-H4 cells were digested and resuspended with PBS containing 2% FBS. The cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 2 h away from light. Thereafter, the cells in each well were rinsed twice with 100 µL of pre-cooled PBS containing 2% FBS, and centrifuged at 500 g at 4 °C for 5 min, and then the supernatant was discarded. Then 100 µL of a fluorescent secondary antibody (Alexa Fluor^{®} 647, Goat Anti-Human IgG, Fcy fragment specific, Jackson ImmunoResearch, #109-605-098, 1: 1000 diluted) was added to each well. The plate was incubated away from light at 4 °C for 1 h. The cells in each well were rinsed twice with 100 µL of pre-cooled PBS containing 2% FBS, and centrifuged at 500 g at 4 °C for 5 min, and then the supernatant was discarded. Finally, the cells in each well were resuspended in 200 µL of pre-cooled PBS containing 2% BSA, and the fluorescence signal values were read using an ACEA Novocyte3000 flow cytometer.

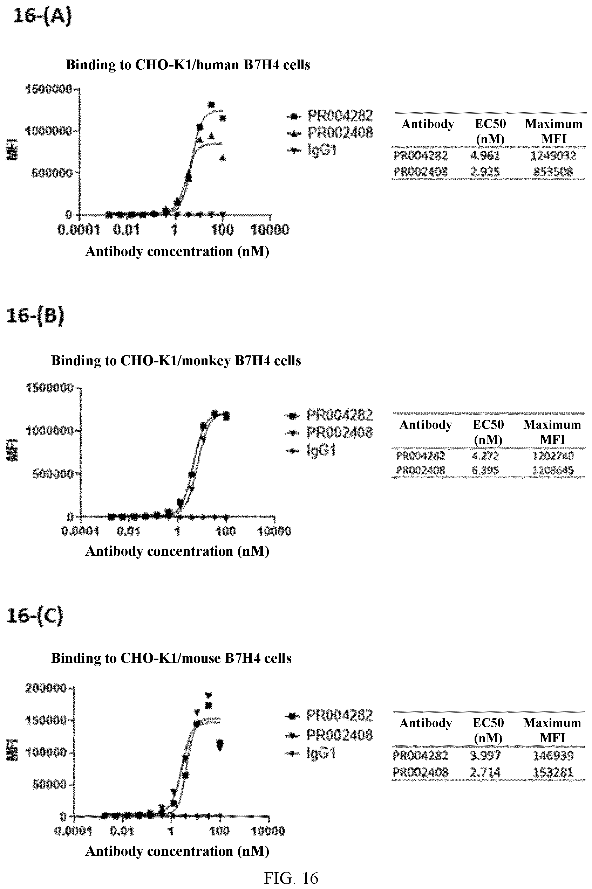

FIG. 16-(A) shows the *in vitro* binding of the B7-H4/4-1BB bispecific antibody PR004282 on the CHO-K1 cell line highly expressing human B7-H4; PR004282 showed higher binding activity to the CHO-K1/hB7H4 cell line than the B7H4 monoclonal antibody PR002408.

FIG. 16-(B) shows the *in vitro* binding of the B7-H4 antibodies on the CHO-K1 cell line highly expressing cynomolgus monkey B7-H4; PR004282 was comparable to PR002408 in the binding activity to the CHO-K1-cynomolgus monkey B7-H4 cell line.

FIG. 16-(C) shows the *in vitro* binding of B7-H4 antibodies on the CHO-K1 cell line highly expressing mouse B7-H4; PR004282 had a slight ability to bind to mouse B7H4.

### Example 13. FACS Assays for Ability of B7-H4×4-1BB Bispecific Antibody PR004282 to Bind to Both CHO-K1/h4-1BB and SK-BR-3

This example is intended to investigate the ability of the B7-H4×4-1BB bispecific antibody PR004282 to bind to both CHO-K1/h4-1BB and SK-BR-3. Briefly, the CHO-K1/h4-1BB cells and SK-BR-3 cells were digested, and the cell density was adjusted to 1×10⁶ cells/mL with PBS. The SK-BR-3 cells and CHO-K1/h4-1BB cells were stained with 0.5 µM Far-red (lifetechnologies, #C34572) and 0.5 µM CFSE (lifetechnologies, #C34544), respectively, at room temperature for 5 min. The stained cells were centrifuged and washed once with >20 mL of medium containing 1% FBS. The washed cells were resuspended in FACS buffer, and the cell density was adjusted to 2×10⁶/mL. To each well of a 96-well V-bottom plate (Corning, #3894) were added 50 µL of SK-BR-3 cells (1×10⁵ cells per well), 25 µL of CHO-K1/h4-1BB cells (1×10⁵ cells per well) and 25 µL of a test antibody serially diluted 4-fold with FACS buffer. The mixtures were well mixed and incubated at 4 °C for 1 h. The percentage of the double positive FITC+CHO-K1/h4-1BB cells and Alexa647+SK-BR-3 was determined using an ACEA Novocyte3000 flow cytometer. Data were analyzed using FlowJo software (Tree Star, Ashland, OR). The percentage of double stained cells was used to determine the bispecific antibody-mediated co-binding rate = (Q2/(Q1 + Q2).

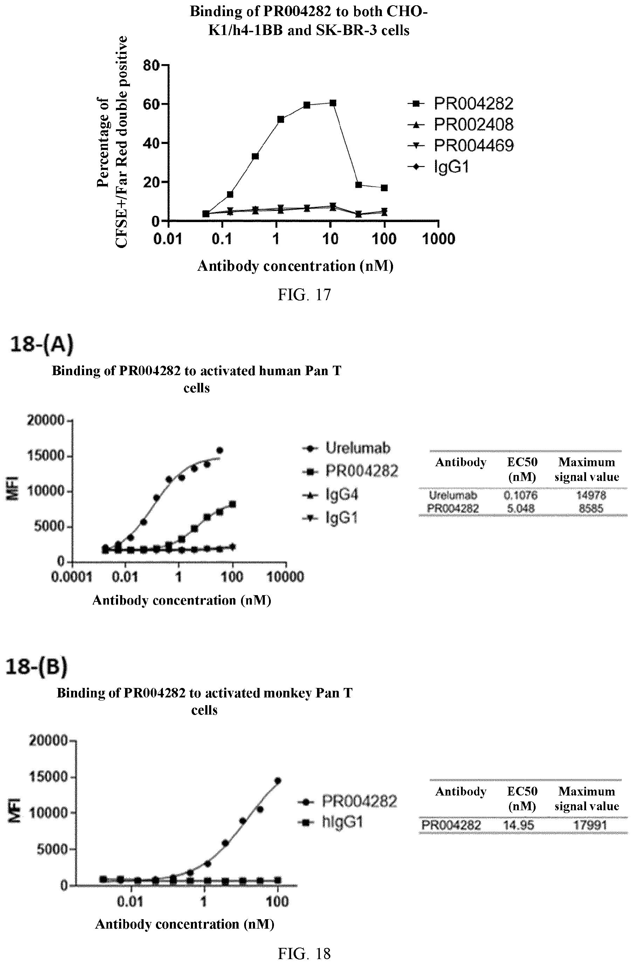

FIG. 17 shows that only PR004282 has the ability to bind both CHO-K1/h4-1BB and SK-BR-3. None of the monoclonal antibodies has this ability.

### Example 14. FACS Assays for Binding Ability of B7-H4×4-1BB Bispecific Antibody PR004282 at Primary Cell Level

This example is intended to investigate the *in vitro* binding activity of the **B7-H4×4-1BB** bispecific antibody PR004282 to activated human/cynomolgus monkey primary T cells. Monkey CD3+ T cells were isolated from cryopreserved monkey PBMCs (PharmaLegacy, #SC1702051) using a non-human primate CD3 isolation kit (Miltenyi, #130-092-012), resuspended to 2×10⁶/mL in medium RPMI1640 + 10% FBS + 1% sodium pyruvate (Thermo, #11360-070) + 1% non-essential amino acid solution (Thermo, #11140-050). 1 mL of the cell suspension was added to a 6-well plate, and 1 mL of medium containing 20 ng/mL PMA (Sigma, #P1585-1MG) and 5 nM Ionomycin (Sigma, #407952-5MG) was added. The plate was incubated in an incubator at 37 °C with CO₂ for 16 h. Human CD3+ T cells were isolated from cryopreserved human PBMCs (Saily, XFB-HP100B) using a human CD3 isolation kit (Miltenyi, #130-096-535), and other procedures were the same as those for the monkey CD3+ T cells.

The activated monkey T cells was adjusted to a density of 1×10⁶ cells/mL with PBS containing 2% FBS and seeded at 100 µL CD3+ T cells/well in a 96-well V-bottom plate (Corning, #3894), followed by the addition of test antibodies PR004282/hIgG1/urelumab/hIgG4 serially diluted 3-fold with a concentration that was twice the final concentration at 100 µL/well. The cells were incubated away from light at 4 °C for 2 h. Thereafter, the cells in each well were rinsed twice with 100 µL of pre-cooled PBS containing 2% FBS, and centrifuged at 500 g at 4 °C for 5 min, and then the supernatant was discarded. Then 100 µL of a fluorescent secondary antibody (Alexa Fluor^{®} 647, Goat Anti-Human IgG, Fcy fragment specific, Jackson ImmunoResearch, #109-605-098, 1:1000 diluted) was added to each well. The plate was incubated away from light at 4 °C for 1 h. The cells in each well were rinsed twice with 100 µL of pre-cooled PBS containing 2% FBS, and centrifuged at 500 g at 4 °C for 5 min, and then the supernatant was discarded. Finally, the cells in each well were resuspended in 100 µL of pre-cooled PBS containing 2% FBS, and the fluorescence signal values were read using an ACEA Novocyte3000 flow cytometer.

The activated human T cells were incubated with Zombie NIR^{™} dye (Biolegend, #77184) away from light at room temperature for 15-30 min and washed once with FACS buffer. The cell density was adjusted to 1×10⁶ cells/mL. The cells were seeded at 100 µL CD3+ T cells/well in a 96-well V-bottom plate (Corning, #3894), followed by the addition of test antibodies PR004282/hIgG1 serially diluted 3-fold with a concentration that was twice the final concentration at 100 µL/well. The cells were incubated away from light at 4 °C for 2 h. Thereafter, the cells in each well were rinsed twice with 100 µL of pre-cooled PBS containing 2% FBS, and centrifuged at 500 g at 4 °C for 5 min, and then the supernatant was discarded. Then 100 µL of a fluorescent secondary antibody (Alexa Fluor^{®} 488, Goat Anti-Human IgG, Fcy fragment specific, Jackson ImmunoResearch, #109-545-098, 1:1000 diluted) was added to each well. The plate was incubated away from light at 4 °C for 1 h. The cells in each well were rinsed twice with 100 µL of pre-cooled PBS containing 2% FBS, and centrifuged at 500 g at 4 °C for 5 min, and then the supernatant was discarded. Finally, the cells in each well were resuspended in 100 µL of pre-cooled PBS containing 2% FBS, and the fluorescence signal values were read using an ACEA Novocyte3000 flow cytometer.

As shown in FIG. 18-(A-B), the B7-H4/4-1BB bispecific antibody PR004282 can bind to the activated human or monkey CD3+ T cells.

### Example 15. ELISA Assays for Cross-Reactivity of B7-H4×4-1BB Bispecific Antibody PR004282 with Other Members of B7 Family or TNFR Family

### 15.1 ELISA assays for cross-reactivity of B7-H4×4-1BB bispecific antibody PR004282 with other members of B7 family

The proteins of the B7 family (Table 13) were each diluted to 1 µg/mL with PBS, added to a 96-well plate (Corning, #9018) at 100 µL per well, and incubated overnight at 4 °C. After the liquid was discarded, the plate was washed 3 times with PBST buffer (pH 7.4, containing 0.05% tween-20), and 250 µL of 2% BSA blocking buffer was added. The plate was incubated at 37 °C for 1 h. The blocking buffer was discarded, and the plate was washed 3 times with PBST buffer. The test antigen-binding protein was diluted to 3 concentrations: 100 nM, 10 nM and 1 nM, and added at 100 µL per well. The plate was incubated at 37 °C for 1 h. After the plate was washed 3 times with PBST buffer, a 5000-fold diluted goat anti-human HRP secondary antibody (Invitrogen, #A18805) was added. The plate was incubated away from light at 37 °C for 1 h. After the plate was washed 3 times with PBST buffer, TMB (Biopanda, #TMB-S-003) was added at 100 µL/well. The plate was left away from light at room temperature for about 30 min. The reactions were terminated by adding 50 µL of stop buffer (BBI life sciences, #E661006-0200) to each well, and the absorbance values at 450 nm (OD450) was measured using a microplate reader (PerkinElemer, #Enspire).

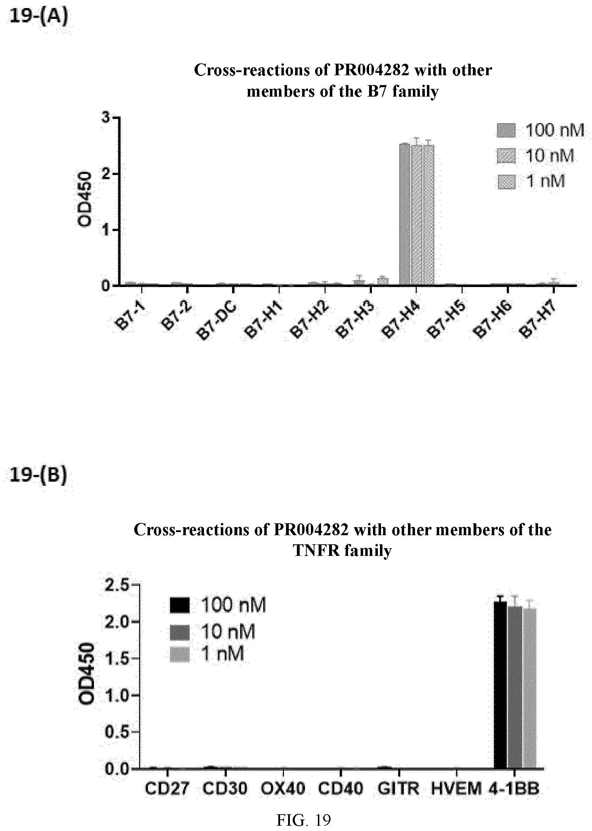

FIG. 19-(A) shows that the antibody PR004282 of the present invention did not cross-react with other member proteins of the B7 family.

**Table 13. Suppliers of other members of the B7 family**

| Other members of B7 family | Supplier | Catalog number | Alias |
|---|---|---|---|
| Human B7-1/CD80 protein, Fc Tag (HPLC-verified) | Acrobiosystem | B71-H5259 | CD80 |
| Human B7-2 / CD86 protein, Fc Tag | Acrobiosystem | CD6-H5257 | CD86 |
| Human B7-DC/PD-L2/CD273 recombinant protein (Fc Tag) | Sino Biological | H10292-H02H | PD-L2 |
| Human PD-L1/B7-H1 protein, His Tag (HPLC verified) | Acrobiosystem | PD1-H5229 | PD-L1 |
| Human CD275/ICOS ligand recombinant protein (Fc Tag) | Sino Biological | 11559-H02H | ICOS-L |
| Human B7-H3/CD276 recombinant protein (ECD, Fc Tag) | Sino Biological | 11188-H02H | |
| Human B7H4 Fc chimeric recombinant protein | R&D | 8870-B7-050 | B7S1 |
| Human VISTA/B7H5/PD-1H Fc chimeric recombinant protein | R&D | 7126-B7-050 | VISTA |
| Human B7-H6/NCR3LG1 chimeric recombinant protein (Fc Tag) | Sino Biological | 16140-H02H | |
| Human B7H7/HHLA2 chimeric recombinant protein (Fc Tag) | R&D | 8084-B7-050 | HHLA2 |

### 15.2 ELISA assays for cross-reactivity of B7-H4×4-1BB bispecific antibody PR004282 with other members of TNFR family

The proteins of the TNFR family (Table 14) were each diluted to 1 µg/mL with PBS, added to a 96-well plate (Corning, #9018) at 100 µL per well, and incubated overnight at 4 °C. After the liquid was discarded, the plate was washed 3 times with PBST buffer (pH 7.4, containing 0.05% tween-20), and 250 µL of 2% BSA blocking buffer was added. The plate was incubated at 37 °C for 1 h. The blocking buffer was discarded, and the plate was washed 3 times with PBST buffer. The test antigen-binding protein was diluted to 3 concentrations: 100 nM, 10 nM and 1 nM, and added at 100 µL per well. The plate was incubated at 37 °C for 1 h. An isotype antibody was used as a control. After the plate was washed 3 times with PBST buffer, a 5000-fold diluted goat anti-human HRP secondary antibody (Invitrogen, #A18805) was added. The plate was incubated away from light at 37 °C for 1 h. After the plate was washed 3 times with PBST buffer, TMB (Biopanda, #TMB-S-003) was added at 100 µL/well. The plate was left away from light at room temperature for about 30 min. The reactions were terminated by adding 50 µL of stop buffer (BBI life sciences, #E661006-0200) to each well, and the absorbance values at 450 nm (OD450) was measured using a microplate reader (PerkinElemer, #Envision).

**Table 14. Suppliers of other members of the TNFR family**

| Other members of TNFR family | Supplier | Catalog No. |
|---|---|---|
| CD30 | Acrobiosystems | CD0-H5229 |
| CD27 | Acrobiosystems | CD7-H522b |
| HVEM | Acrobiosystems | HVM-H52E9 |
| GITR | Acrobiosystems | GIR-H5228 |
| OX40 | Acrobiosystems | OX0-H5224 |
| 4-1BB | Acrobiosystems | 41B-H5227 |
| CD40 | Sino Biological | 10774-H08H |

FIG. 19-(B) shows that the antibody PR004282 of the present invention did not cross-react with other member proteins of the TNFR family.

### Example 16. Determination of ADCC Effect of B7-H4×4-1BB Bispecific Antibody PR004282

16.1 This example used an ADCC reporter gene cell line to determine the ADCC effect of PR004282

The ADCC effect activity of PR004282 for CHO-K1/h4-1BB or SK-BR-3 was determined using Jurkat FcyRIIIa-V158/NFAT-Luc cells (prepared in-house by Harbour Biomed). The CHO-K1/h4-1BB or SK-BR-3 cells were centrifuged at 300 g for 5 min and then resuspended in RPMI1640+4% FBS serum medium. The cell density was adjusted to 6×10⁵ cells/mL, and 50 µL of the cell suspension was added to each well of a 96-well plate. The plate was incubated overnight at 37 °C. The Jurkat FcyRIIIa-V158/NFAT-Luc cells were centrifuged at 400 g for 4 min and then resuspended in RPMI1640+4% FBS serum medium. The cell density was adjusted to 3×10⁶ cells/mL, and 50 µL of the cell suspension was added to each well of a 96-well plate. Antibodies were 5-fold diluted with RPMI1640+4% FBS medium to a maximum final concentration of 100 nM, and a total of 4 concentrations were prepared for each antibody. Two replicates were set. 50 µL of the antibody dilutions was added to each well of the 96-well plate. Meanwhile, an isotype IgG control group and a blank medium control group were set in the plate. The cells were incubated with the antibodies at 37 °C for 5 h. The 96-well plate was left to stand at room temperature for 30 min, and 60 µL of One-Glo chromogenic solution (Promega, #E6110) was added to each well at room temperature. Then, the sample was incubated away from light at room temperature for 10 min. Luminscence readings were obtained using PE Enspire. Fold = Luminscence reading of antibody well/Luminscence reading of blank medium control group. Plotting was performed using Prism 8 software. PR004469 and PR003369 (B7H4 monoclonal antibodies of an affinity mature variant, having ADCC-enhanced effects, comprising a heavy chain with a sequence as set forth in SEQ ID NO: 290 and a light chain with a sequence as set forth in SEQ ID NO: 291) were used as positive controls, and human Iso IgG1 (CrownBio, #C0001-4) was used as a negative control.

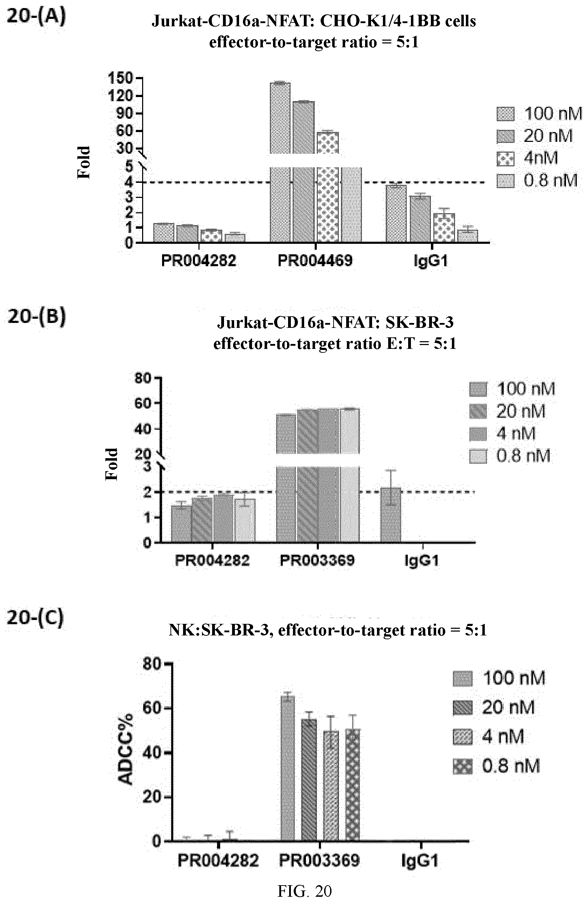

FIGs. 20-(A)-(B) show that PR004282 did not have significant ADCC activity against CHO-K1/h4-1BB and SK-BR-3 cells, while the positive controls PR004469 and PR003369 were able to produce a strong ADCC effect on CHO-K1/4-1BB or SK-BR-3 cells, respectively, in a dose-dependent manner.

### 16.2 This example is intended to investigate the activity of the B7H4/4-1BB bispecific antibody PR004282 in mediating NK cell killing of target cells through the ADCC effect.

In this experiment, the human NK cell was used as an effector cell and an SK-BR-3 cell line highly expressing B7H4 as a target cell. The killing efficiency was reflected by the conductivity of the target cell measured using an RTCA instrument from ACEA. A 96-well E-plate was first equilibrated with 50 µL of complete medium. SK-BR-3 cells were digested, resuspended in RPM1640 complete medium containing 10% fetal bovine serum and diluted to 4× 10⁵/mL. The cell suspension was plated on the 96-well E-plate at 50 µL/well, i.e. 2×10⁴/well, and incubated overnight at 37 °C. The next day NK cells were sorted using an EasySep^{™} Human CD56 positive selection kit (Stem cell, #17855). 50 µL of fresh culture medium containing 1×10⁵ PBMCs was added to each well, followed by the addition of 50 µL of 4× antibodies serially diluted 5-fold with a maximum final concentration of 100 nM. A total of 4 concentrations were set in duplicate for each antibody. PR003369 was used as a positive control and human Iso IgG1 (CrownBio, C0001-4) antibody as a negative control. The conductivity of the target cells was measured in real time. The value at hour 24 was used to calculate the target cell killing efficiency = (1 - sample/iso control) × 100%.

The results in FIG. 20-(C) show that PR004282 did not significantly kill compared to PR003369, indicating that PR004282 does not have ADCC activity against SK-BR-3 target cells.

### Example 17. Determination of Affinity of Antibodies for Human, Mouse and Cynomolgus Monkey Fcy Receptor Proteins by BLI

An Octet Red 96e molecular interaction analyzer was used in the assay. The buffer used in the experiment was a 1× diluted kinetics buffer (ForteBio, #18-1105).

The rotational speed of the instrument was set to 1000 rpm. FAB2G sensors (Fortebio, #18-5125) arranged in a row were equilibrated in a test buffer for 10 min, and then allowed to capture antibodies at a capture height of 1 nm. After being equilibrated in buffer for 120 s, the FAB2G sensors were allowed to associate with Fcy receptor proteins that were serially diluted 2-fold. The association time was set to 60 s, and the dissociation time to 30 s. The protein concentrations are shown in Table 15. Finally, the FAB2G sensor was immersed in a 10 mM glycine-hydrochloric acid solution at pH 1.5 for regeneration to elute the proteins bound to the sensor. The affinity assays of antibodies for FcRn were performed under the conditions of both pH 6.0 buffer and pH 7.4 buffer. For the avi-tagged cynomolgus monkey CD32b and CD64, an SA sensor (Fortebio, 18-5019) was used to capture the receptor proteins. Antibodies were analytes.

**Table 15. Fc receptor proteins and analyte concentrations**

| **Receptor protein** | **Supplier** | **Catalog No.** | **Concentration** |
|---|---|---|---|
| Human CD64 | Novo Protein | CM60 | 800-25 |
| Human CD32a (H) | | CS35 | 8000-500 |
| Human CD32b | | C444 | 8000-250 |
| Human CD16a (V158) | | C441 | 8000-250 |
| Human CD16a (F158) | | CS11 | 8000-250 |
| Human CD 16b | | CB62 | 8000-250 |
| Human FcRn | | CI01 | 800-50 |
| Mouse CD32 (C-6his) | | C767 | 8000-250 |
| Mouse CD16 (C-6his) | | CS29 | 4000-250 |
| Mouse CD64 (his) | Aero Biosystems | CD4-M5227 | 1000-125 |
| Cynomolgus monkey FcRn | | FCM-C52W9 | 800-25 |
| Cynomolgus monkey CD32a | | CDA-C52H7 | 8000-250 |
| Cynomolgus monkey CD32b | | CDB-C82E4 | 3000-187.5 |
| Cynomolgus monkey CD16 | | FC6-C52H9 | 2000-125 |
| Cynomolgus monkey CD64 | | FCA-C82E8 | 400-12.5 |

When data analysis was performed using Octet Data Analysis software (Fortebio, version 11.0), 0 nM was used as a reference hole, and reference subtraction was performed; the "1:1 Global fitting" method was selected to fit the data, and the kinetics parameters of the binding of proteins to antibodies were calculated, with kon(1/Ms) values, kdis(1/s) values and KD(M) values obtained. For the interactions of fast association and fast dissociation, the "steady state" method was selected to fit the data to obtain KD(M) values.

As can be seen from Tables 16-18, PR004282 showed higher KD values than the control antibody urelumab, indicating that it binds weakly to the human, mouse and monkey Fcγ receptor protein.

**Table 16. Affinity of PR004282 for human Fcy receptors**

| **KD(M)** | **Human Fcγ receptor** | | | | | | |
|---|---|---|---|---|---|---|---|
| Name of antibody | CD64 | CD 16a(v) | CD16a(F) | CD 16b | CD32a (H) | CD32b | FcRn (pH 6.0) |
| PR002408 | 2.68E-11 | 1.23E-07 | 1.62E-06 | 3.80E-06 | 4.40E-07 | 3.30E-06 | 2.64E-07 |
| PR004282 | 1.20E-07 | 1.52E-06 | No binding | | >8E-06 | No binding | 2.35E-07 |
| Urelumab | 6.52E-10 | 2.39E-06 | | | 2.80E-06 | 3.20E-06 | 1.94E-07 |

**Table 17. Affinity of PR004282 for monkey Fcy receptors**

| **KD (M)** | **Monkey Fcγ receptor** | | | | | |
|---|---|---|---|---|---|---|
| **Antibody** | **CD64** | **CD32b** | **CD32a** | **CD16** | **FcRn(pH7.4)** | **FcRn(pH6.0)** |
| PR002408 | 3.67E-10 | 1.51E-06 | 2.10E-06 | 1.29E-07 | No binding | 1.59E-07 |
| PR004282 | 9.77E-08 | 5.10E-06 | No binding | 7.47E-07 | | 1.43E-07 |
| Urelumab | 1.74E-10 | 5.97E-07 | 4.82E-06 | 8.92E-07 | | 1.39E-07 |

**Table 18. Affinity of PR004282 for MOUSE Fcy receptors**

| **KD (M)** | **Mouse Fcγ receptor** | | |
|---|---|---|---|
| **Name of antibody** | **CD16** | **CD32** | **CD64** |
| PR002408 | 1.28E-07 | 6.10E-07 | 3.60E-08 |
| PR004282 | 1.41E-06 | >8E-06 | No binding |
| urelumab | 7.81E-07 | 2.10E-06 | 1.63E-07 |

### Example 18. Determination of Non-Specific Release of Cytokines Caused by B7-H4×4-1BB Bispecific Antibody

This example is intended to evaluate whether B7-H4×4-1BB bispecific antibodies cause non-specific release of cytokines. Monocytes were isolated using a human CD14 isolation kit (Meltenyi, #130-050-201). The monocytes or PBMCs of the same donor were resuspended in RPM1640 complete medium containing 10% fetal bovine serum and diluted to 2E6/mL. The cell suspension was plated on a flat-bottom 96-well plate (Costar, #3599) at 100 µL/well, i.e., 2E5/well, and then 100 µL of antibodies serially diluted 10-fold with concentrations that were twice the final concentrations were added. The highest concentration of the antibodies was 300 nM. A total of 3 concentrations were set in duplicate. The plate was incubated at 37 °C overnight. Urelumab was used as a positive control and utomilumab and human Iso IgG1(CrownBio, C0001-4) as negative controls. After 24 h of incubation, the supernatant was collected and analyzed for the release of TNF-a (Thermo, 88-7066-88) and IL-6 (Thermo, 88-7346-77). After 72 h of incubation, the supernatant was collected and analyzed for the release of IFN-gamma (Thermo, 88-7316-77).

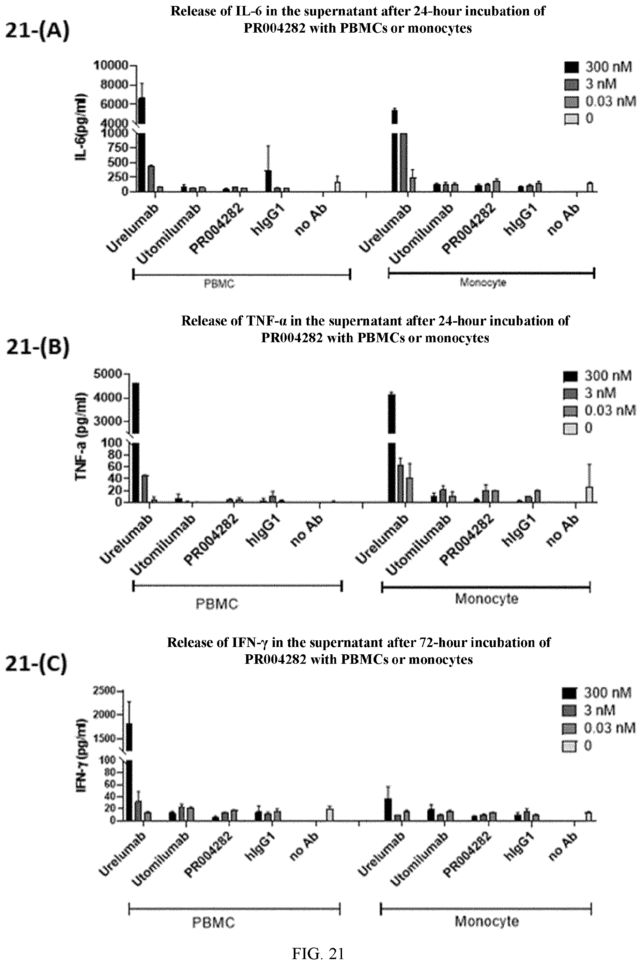

FIGs. 21-(A)-(C) show the release of cytokines after the B7-H4 ×4-1BB bispecific antibody PR004282 was co-incubated with PBMCs or monocytes. Whether being co-incubated with PBMCs or monocytes, urelumab induced great release of TNF-a and IL-6; however, urelumab induced release of IFN-gamma only when being co-incubated with PBMCs. PR004282 showed as good safety as utomilumab, and induced significant release of cytokines whether being co-incubated with PBMCs or monocytes.

### Example 19. Pharmacokinetic Study of B7-H4×4-1BB Bispecific Antibodies in Normal Mice

This example determined the pharmacokinetic properties of fusion proteins as follows. Six female C57BL/6 mice weighing 18-22 g were selected and given a bispecific antibody drug by intravenous injection at a dose of 5 mg/kg PR003334 or 5 mg/kg PR003335 or 5 mg/kg PR004282. The whole blood of 3 mice in one group was collected before the administration and 15 min, 24 h (1 day), 4 days and 10 days after the administration, and the whole blood of 3 mice in the other group was collected before the administration and 5 h, 2 days, 7 days and 14 days after the administration. The whole blood was left to stand for 30 min for coagulation and centrifuged at 2,000 rpm for 5 min at 4 °C, and the isolated serum sample was cryopreserved at -80 °C until it was taken for analysis. In this example, the drug concentration in the serum of mice was quantitatively determined by two ELISA methods. The ELISA method I, namely the overall detection method, was performed by capturing the fusion protein containing human Fc in the serum of mice using a goat anti-human Fc polyclonal antibody coating a 96-well plate, and then adding an HRP-labeled goat anti-human Fc secondary antibody; and the ELISA method II, namely the functional domain detection method, was performed by capturing the bispecific antibody containing human 4-1BB HCAb in the serum of mice using a human 4-1BB protein a 96-well plate was coated with, and then adding an HRP-labeled goat anti-human Fc secondary antibody. The plasma concentration data were analyzed using Phoenix WinNonlin software (version 8.2) by non-compartmental analysis (NCA) to evaluate the pharmacokinetics.

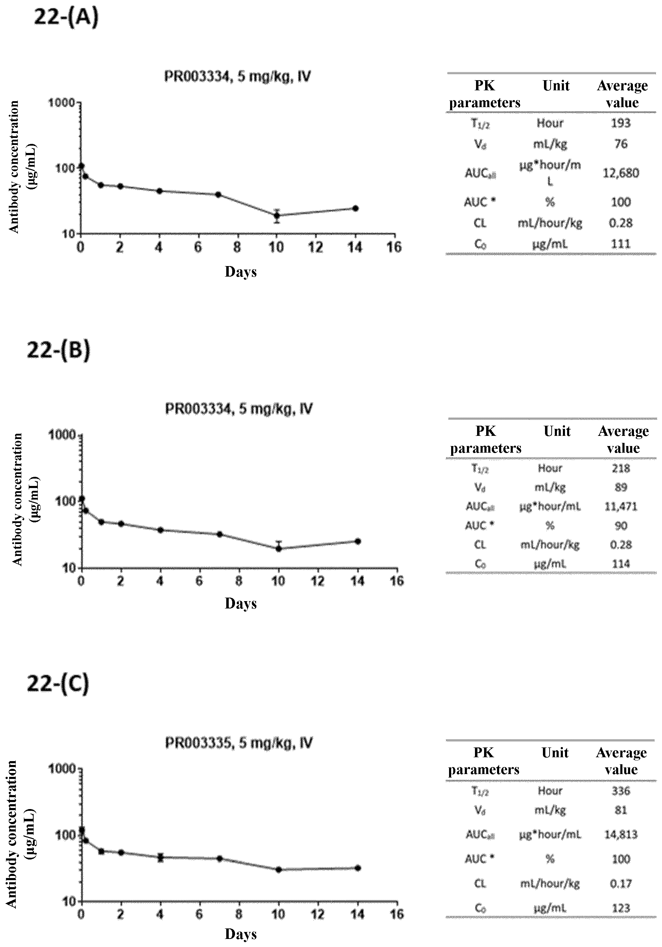

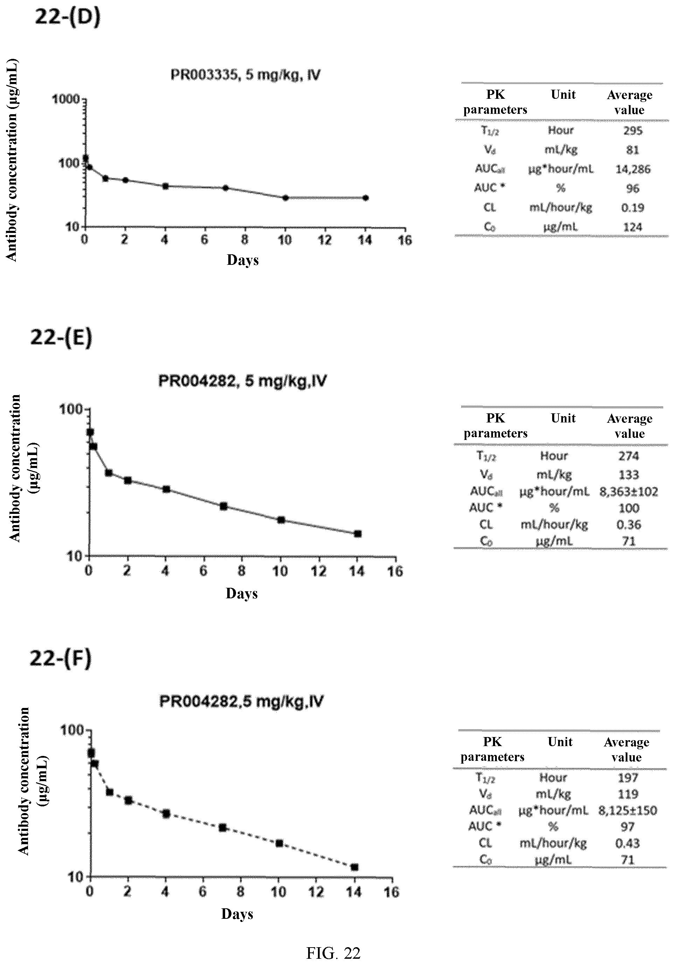

FIGs. 22-(A)-(F) and Table 19 show the pharmacokinetics of B7-H4×4-1BB bispecific antibodies PR003334, PR003335 and PR004282. The results in FIG. 22-(A) show that the half-life of PR003334 in mice determined using the overall detection method is about 8 days. FIG. 22-(A) shows that the half-life of PR003334 in mice determined using the functional domain detection method is about 9 days. FIG. 22-(c) shows that the half-life of PR003335 in mice determined using the overall detection method is about 14 days. FIG. 22-(D) shows that the half-life of PR003335 in mice determined using the functional domain detection method is about 12 days. FIG. 22-(E) shows that the half-life of PR004282 in mice determined using the overall detection method is about 11 days. FIG. 22-(F) shows that the half-life of PR004282 in mice determined using the functional domain detection method is about 8 days.

**Table 19. Pharmacokinetics of PR003334, PR003335 and PR004282**

| | PR003334 | PR003334 | PR003335 | PR003335 | PR004282 | PR004282 |
|---|---|---|---|---|---|---|
| PK parameters | Overall detection | Functional domain detection | Overall detection | Functional domain detection | Overall detection | Functional domain detection |
| T_{1/2}(hr) | 193 | 218 | 336 | 295 | 274 | 197 |
| V_{d} (mL/kg) | 76 | 89 | 81 | 81 | 133 | 119 |
| AUCₐₗₗ (µg/mL hr) | 12,680±428 | 11,471±497 | 14,813±683 | 14,286±560 | 8,363±102 | 8,125±150 |
| AUC (%)* | 100 | 90 | 100 | 96 | 100 | 97 |
| Cl (mL/hr/kg) | 0.28 | 0.28 | 0.17 | 0.19 | 0.36 | 0.43 |
| C₀ (µg/mL) | 111 | 114 | 123 | 124 | 71 | 71 |

### Example 20. Pharmacokinetic Study of B7H4/4-1BB Bispecific Antibody PR004282 in Cynomolgus Monkeys

This example studied the pharmacokinetics of the B7-H4/4-1BB bispecific antibody in cynomolgus monkeys. Specifically, PR004282 was diluted with 0.9% normal saline and intravenously injected into male cynomolgus monkeys at a dose of 1 mg/kg, and blood samples were collected before the administration and 0.5, 1, 2, 4, 8, 12, 24, 48, 72, 168, 336, 504, 672, 840 and 1008 hours after the administration. The blood samples were left to stand at room temperature for 30 min and then centrifuged to obtain serum, and the B7-H4/4-1BB bispecific antibody content in the serum is determined by using the established specific ELISA method (the overall detection method).

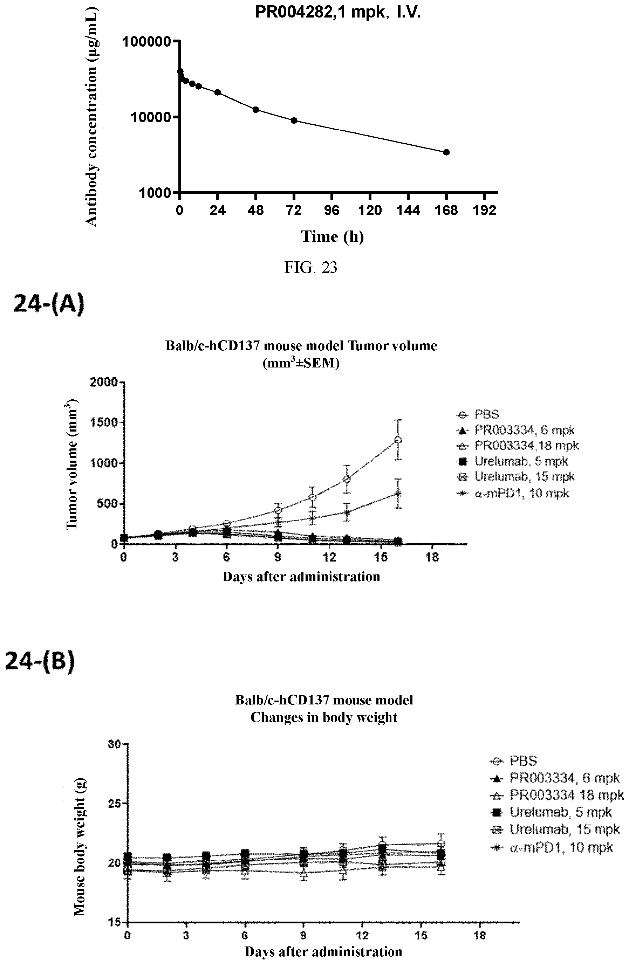

As shown in FIG. 23 and Table 20, the B7-H4/4-1BB bispecific antibody showed a typical IgG-like pharmacokinetic curve after being intravenously administered to cynomolgus monkeys. The half-life of PR004282 in male monkeys is about 3 days.

**Table 20. Pharmacokinetics of PR004282 (in single doses) in normal monkeys**

| PK parameters | | Monkey 1 | Monkey 2 | Average value |
|---|---|---|---|---|
| Cₘₐₓ | µg/mL | 32.1 | 48 | 40 |
| Terminal t_{1/2} | hr | 45.3 | 82.3 | 63.8 |
| AUC₀₋ₗₐₛₜ | hr^{∗}µg/mL | 1472 | 2300 | 1886 |
| AUC_{0-INF} | hr^{∗}µg/mL | 1574 | 2932 | 2253 |
| CL | mL/hr/kg | 0.635 | 0.341 | 0.488 |
| Vₛₛ | mL/kg | 35.1 | 35.5 | 35.3 |

### Example 21. Evaluation of Anti-tumor Effect of B7-H4×4-1BB Bispecific Antibody in BALB/c-hCD137/CT26-B7-H4 Mouse Model

To evaluate the *in vivo* anti-tumor effect of the B7-H4×4-1BB bispecific antibody, 6-8 week-old female BALB/c-hCD137 mice were used, and each was subcutaneously inoculated with 5×10⁵ CT26/hB7-H4 cells on the day of tumor cell inoculation. When the mean tumor volume reached 80 mm³, the mice were randomized into groups of 6. After grouping, drugs diluted with PBS at specific concentrations were administered by intraperitoneal injection (i.p.) twice a week, for a total of 6 doses (BIW×3). PBS was used as a blank control group. Tumor volume and body weight of mice were measured on the day of the first administration and on days 4, 7, 11, 14 and 18 thereafter. Tumor size calculation formula: tumor volume (mm³) = 0.5 × (long diameter of tumor × short diameter of tumor²).

FIG. 24 shows the anti-tumor effect of the B7-H4×4-1BB bispecific antibody in the BALB/c-hCD137/CT26-B7-H4 mouse model. As shown in FIG. 24-(A), the B7-H4×4-1BB bispecific antibody PR003334 produced inhibitory effects against tumor growth in mice at both concentrations of 18 mpk and 6 mpk. As shown in FIG. 24-(B), the change in body weight of mice in each test group is within the normal range.

### Example 22. Evaluation of Anti-tumor Effect of B7-H4×4-1BB Bispecific Antibody in MDA-MB-468 Xenograft Mouse Model

To evaluate the *in vivo* anti-tumor effect of the B7-H4×4-1BB bispecific antibody, 6-8 week-old female NCG mice were used, and each was subcutaneously inoculated with 5×10⁶ MDA-MB-468 cells on the day of tumor cell inoculation. When the mean tumor volume reached 120 mm³, the mice were randomized into groups of 6. After grouping, the mice were inoculated with 5×10⁶ human PBMCs. The next day, drugs diluted with PBS at specific concentrations were administered by intraperitoneal injection (i.p.). The administration was performed twice a week, for a total of 6 doses (BIW×3). An isotype control IgG was used as a control group. Tumor volume and body weight of mice were measured on the day of the first administration and on days 7, 12, 15, 19, 22, 26 and 29 thereafter. Tumor size calculation formula: tumor volume (mm³) = 0.5 × (long diameter of tumor × short diameter of tumor²).

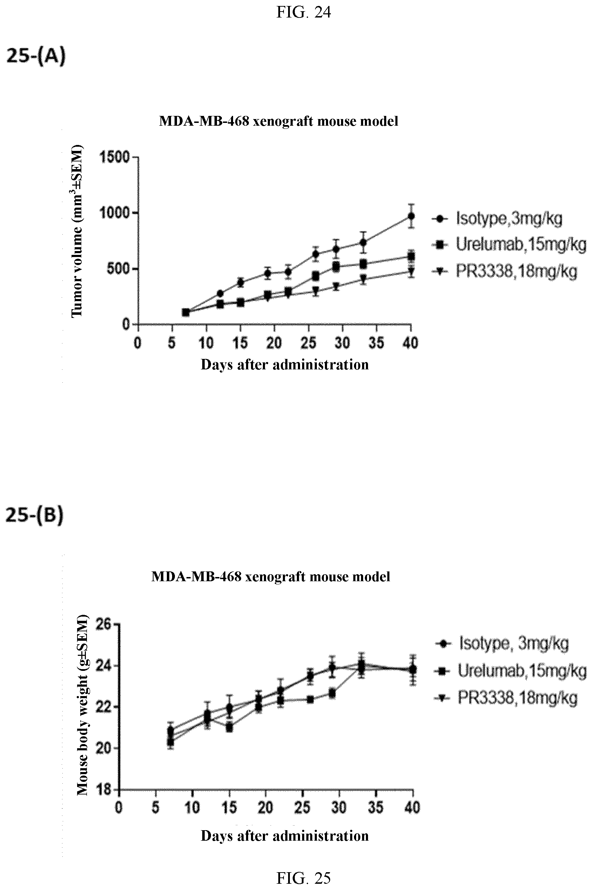

FIG. 25 shows the anti-tumor effect of the B7-H4×4-1BB bispecific antibody in the MDA-MB-468 xenograft mouse model. As shown in FIG. 25-(A), the B7-H4×4-1BB bispecific antibody PR003338 produced an inhibitory effect against tumor growth in mice at concentration 18 mpk, which was superior to that of urelumab at concentration 15 mpk. As shown in FIG. 25-(B), the change in body weight of mice in each test group is within the normal range.

### Example 23. Evaluation of Anti-tumor Effect of B7-H4×4-1BB Bispecific Antibody in OVCAR3 Xenograft Mouse Model

To evaluate the *in vivo* anti-tumor effect of the B7-H4×4-1BB bispecific antibody, 6-8 week-old female NCG mice were used, and each was subcutaneously inoculated with 5×10⁶ OVCAR3 cells on the day of tumor cell inoculation. When the mean tumor volume reached 120 mm³, the mice were randomized into groups of 6. After grouping, the mice were inoculated with 5×10⁶ human PBMCs. The next day, drugs diluted with PBS at specific concentrations were administered by intraperitoneal injection (i.p.). The administration was performed twice a week, for a total of 6 doses (BIW×3). An isotype control IgG was used as a control group. Tumor volume and body weight of mice were measured on the day of the first administration and on days 7, 12, 15, 19, 22, 26 and 29 thereafter. Tumor size calculation formula: tumor volume (mm³) = 0.5 × (long diameter of tumor × short diameter of tumor²).

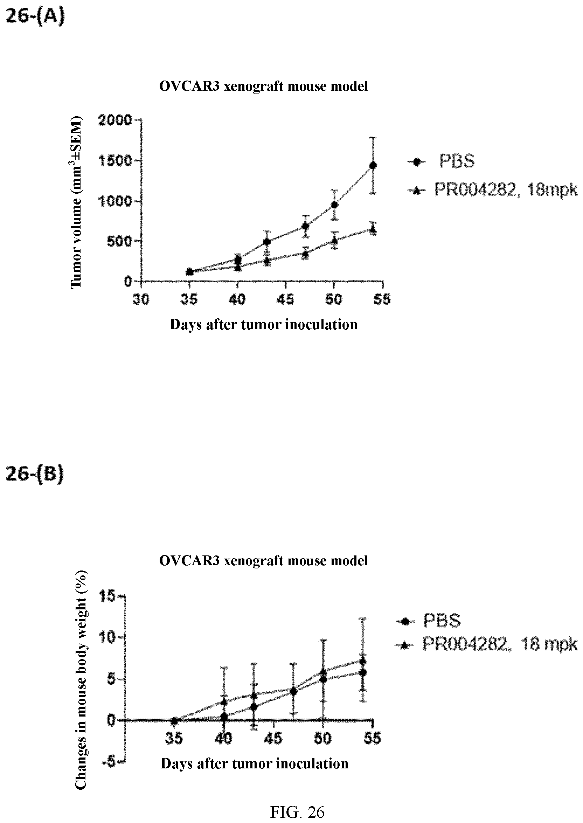

FIG. 26 shows the anti-tumor effect of the B7-H4×4-1BB bispecific antibody PR004282 in the OVCAR3 xenograft mouse model. As shown in FIG. 26-(A), the B7-H4×4-1BB bispecific antibody PR004282 produced inhibitory effects against tumor growth in mice at concentration 18 mpk. As shown in FIG. 26-(B), the change in body weight of mice in each test group is within the normal range.

### Example 24. Evaluation of Anti-tumor Effect and Memory Immune Effect of B7-H4×4-1BB Bispecific Antibody in BALB/c-hCD137/CT26-hB7-H4 Mouse Model

To evaluate the *in vivo* anti-tumor effect of the B7-H4×4-1BB bispecific antibody, 6-8 week-old female BALB/c-hCD137 mice were used, and each was subcutaneously inoculated with 5×10⁵ CT26-B7-H4 cells on the day of tumor cell inoculation. When the mean tumor volume reached 80 mm³, the mice were randomized into groups of 6. After grouping, drugs diluted with PBS at specific concentrations were administered by intraperitoneal injection (i.p.) twice a week, for a total of 6 doses (BIW×3). PBS was used as a blank control group, and urelumab as a positive control group. Tumor volume and body weight of mice were measured on the day of the first administration and three times a week over the following 45 days. Tumor size calculation formula: tumor volume (mm³) = 0.5 × (long diameter of tumor × short diameter of tumor²). Mice with tumor elimination were inoculated subcutaneously with 5×10⁵ CT26/hB7H4 cells on the other side 45 days after the administration, and a mouse of the same age was used as a new inoculation control group. Tumor volume and body weight of mice were measured three times a week, and the mice were sacrificed on day 66.

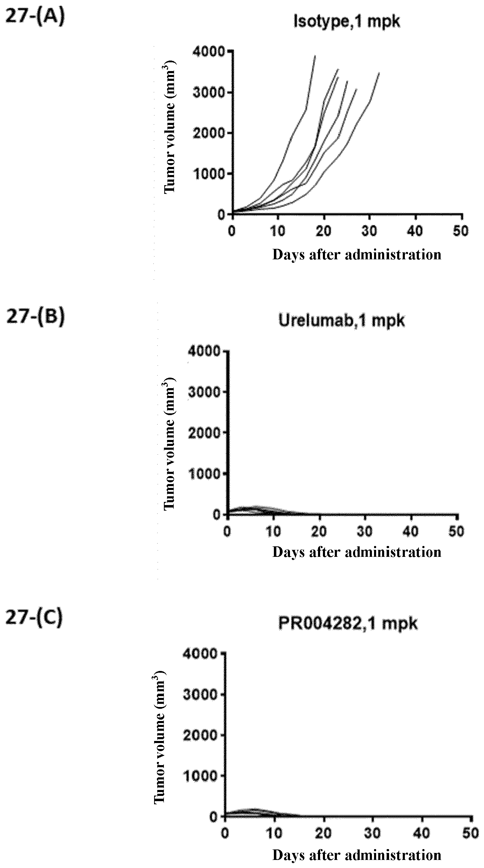

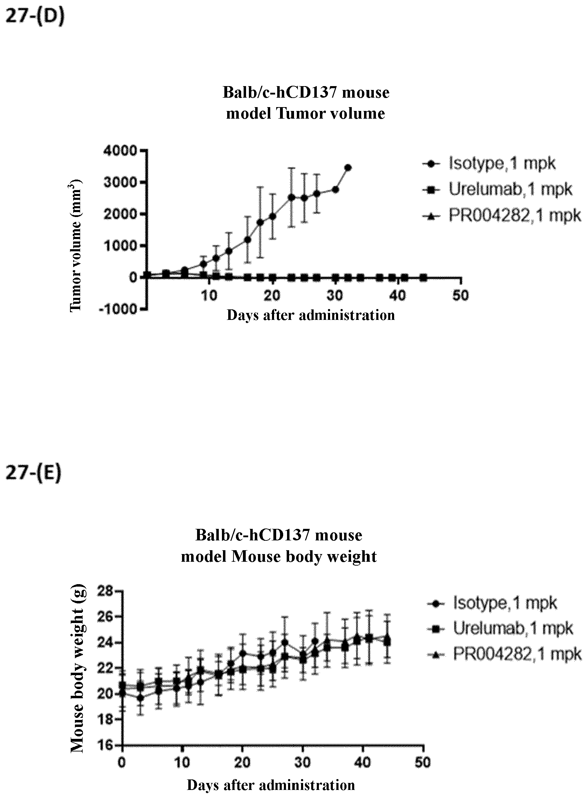

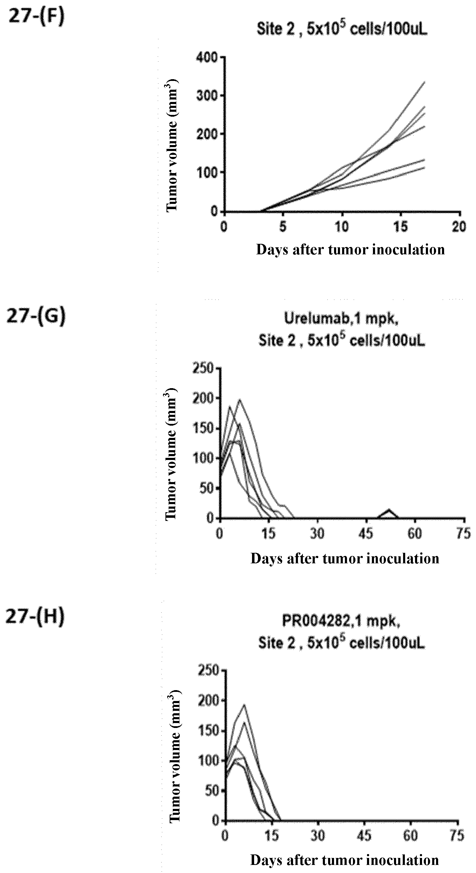

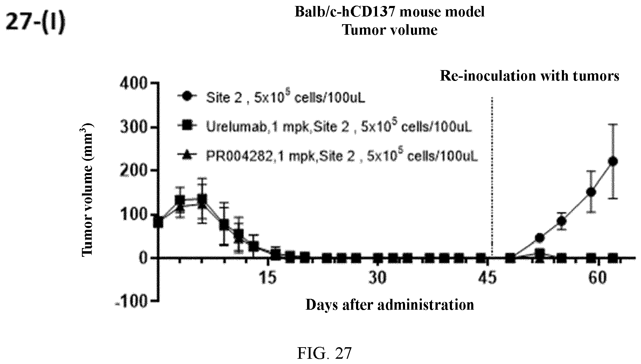

FIG. 27 shows the anti-tumor effect and memory immune effect of the B7-H4×4-1BB bispecific antibody PR004282 in the BALB/c-hCD137/CT26-B7-H4 mouse model. As shown in FIGs. 27-(A)-(D), the B7-H4×4-1BB bispecific antibody PR004282 produced an inhibitory effect against tumor growth in mice at concentration 1 mpk, which is comparable to that of urelumab. As shown in FIG. 27-(E), the change in body weight of mice in each test group is within the normal range. As shown in FIGs. 27-(F)-(I), compared to the mouse of the same age without inoculation and administration, the mice inoculated again with the same tumor after tumor elimination exhibited no tumor growth, indicating that PR004282 can induce the production of memory immune T cells to produce a long-lasting killing effect against tumors.

### Example 25. Specificity Evaluation of Anti-tumor Effect of B7-H4×4-1BB Bispecific Antibody

### 25.1 Specificity evaluation of anti-tumor effect of B7-H4×4-1BB bispecific antibody in JIMT-1 xenograft mouse model

6-8 week-old female NCG mice were used, and each was subcutaneously inoculated with 5×10⁶ JIMT-1 cells on the day of tumor cell inoculation. When the mean tumor volume reached 120 mm³, the mice were randomized into groups of 6. After grouping, the mice were inoculated with 5×10⁶ human PBMCs. The next day, drugs diluted with PBS at specific concentrations were administered by intraperitoneal injection (i.p.). The administration was performed twice a week, for a total of 6 doses (BIW×3). An isotype control IgG was used as a control group. Tumor volume and body weight of mice were measured on the day of the first administration and on days 7, 12, 15, 19, 22, 26 and 29 thereafter. Tumor size calculation formula: tumor volume (mm³) = 0.5 × (long diameter of tumor × short diameter of tumor²).

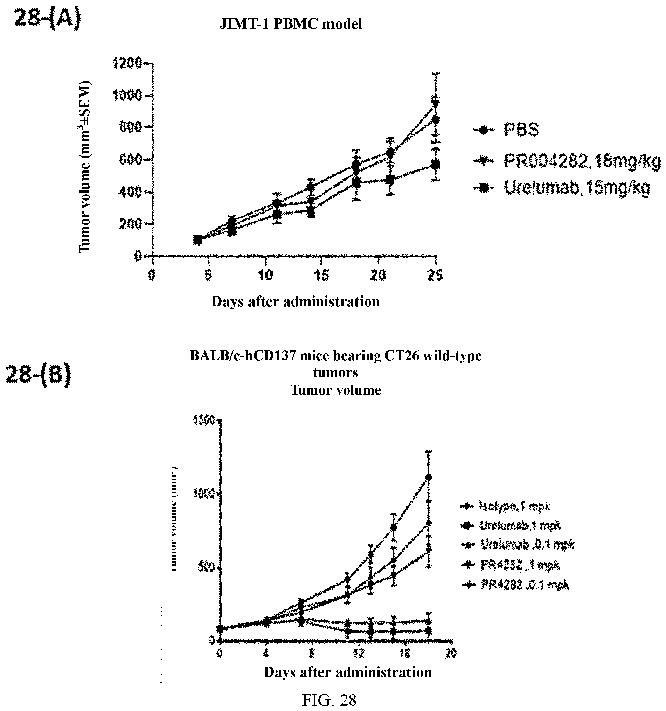

FIG. 28-(A) shows the anti-tumor effect of PR004282 in the JIMT-1 xenograft mouse model. As shown in the drawing, PR004282 produced no inhibitory effect against tumor growth in mice at concentration 18 mpk, while urelumab produced a certain effect at concentration 15 mpk. Since JIMT-1 is a cell that does not express B7H4, it is suggested that PR004282 is unable to kill tumor cells that do not express B7H4.

### 25.2 Specificity evaluation of anti-tumor effect of B7-H4×4-1BB bispecific antibody in BALB/c-hCD137/CT26 mouse model

To evaluate the specificity of the *in vivo* anti-tumor effect of the B7-H4×4-1BB bispecific antibody, 6-8-week-old female BALB/c-hCD137 mice were used, and each was subcutaneously inoculated with 5×10⁵ CT26 wild-type cells on the day of tumor cell inoculation. When the mean tumor volume reached 80 mm³, the mice were randomized into groups of 6. After grouping, drugs diluted with PBS at specific concentrations were administered by intraperitoneal injection (i.p.) twice a week, for a total of 6 doses (BIW×3). PBS was used as a blank control group. Tumor volume and body weight of mice were measured on the day of the first administration and on days 4, 7, 11, 14 and 18 thereafter. Tumor size calculation formula: tumor volume (mm³) = 0.5 × (long diameter of tumor × short diameter of tumor²).

FIG. 28-(B) show that the B7-H4 ×4-1BB bispecific antibody PR004282 did not produce a strong inhibitory effect against tumor growth in mice in the BALB/c-hCD137/CT26 mouse model, while urelumab produced a strong effect. Since CT26 is a cell that does not express B7H4, it is suggested that PR004282 is unable to kill cells that do not express B7H4. In another aspect, according to the results in Example 24, after overexpression of human B7H4 on CT26 cells, the growth of CT26-hB7-H4 tumor cells was inhibited by PR004282. By combining the two experiments, it can be concluded that the killing of tumor cells by PR004282 is dependent on B7H4 expression.

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

## Claims

1. A bispecific antibody comprising a B7-H4-targeting antigen-binding domain and a 4-1BB-targeting antigen-binding domain.

2. The bispecific antibody according to claim 1, wherein the B7-H4-targeting antigen-binding domain comprises HCDR1, HCDR2 and HCDR3, wherein the HCDR1 comprises an amino acid sequence as set forth in SEQ ID NO: 16 or 23, the HCDR2 comprises an amino acid sequence as set forth in SEQ ID NO: 46, 59 or 60, and the HCDR3 comprises an amino acid sequence as set forth in SEQ ID NO: 98 or 84;
preferably, the B7-H4-targeting antigen-binding domain comprises: HCDR1 with a sequence as set forth in SEQ ID NO: 16, HCDR2 with a sequence as set forth in SEQ ID NO: 46 and HCDR3 with a sequence as set forth in SEQ ID NO: 84; HCDR1 with a sequence as set forth in SEQ ID NO: 23, HCDR2 with a sequence as set forth in SEQ ID NO: 59 and HCDR3 with a sequence as set forth in SEQ ID NO: 98; or, HCDR1 with a sequence as set forth in SEQ ID NO: 16, HCDR2 with a sequence as set forth in SEQ ID NO: 60 and HCDR3 with a sequence as set forth in SEQ ID NO: 84; preferably comprises a VH with a sequence as set forth in SEQ ID NO: 142, SEQ ID NO: 159 or SEQ ID NO: 160; and more preferably comprises a heavy chain with a sequence as set forth in SEQ ID NO: 174, SEQ ID NO: 191 or SEQ ID NO: 192;
more preferably, the B7-H4-targeting antigen-binding domain further comprises LCDR1 with an amino acid sequence as set forth in SEQ ID NO: 112 or 113, LCDR2 with an amino acid sequence as set forth in SEQ ID NO: 118 and LCDR3 with an amino acid sequence as set forth in any one of SEQ ID NOs: 131-133;
even more preferably, the B7-H4-targeting antigen-binding domain further comprises: LCDR1 with a sequence as set forth in SEQ ID NO: 112, LCDR2 with a sequence as set forth in SEQ ID NO: 118 and LCDR3 with a sequence as set forth in SEQ ID NO: 131; LCDR1 with a sequence as set forth in SEQ ID NO: 113, LCDR2 with a sequence as set forth in SEQ ID NO: 118 and LCDR3 with a sequence as set forth in SEQ ID NO: 132; or, LCDR1 with a sequence as set forth in SEQ ID NO: 112, LCDR2 with a sequence as set forth in SEQ ID NO: 118 and LCDR3 with a sequence as set forth in SEQ ID NO: 133; preferably further comprises a VL with a sequence as set forth in any one of SEQ ID NOs: 166-169; and more preferably further comprises a light chain with a sequence as set forth in any one of SEQ ID NOs: 198-201.

3. The bispecific antibody according to claim 2, wherein the B7-H4-targeting antigen-binding domain comprises HCDR1 with a sequence as set forth in SEQ ID NO: 16, HCDR2 with a sequence as set forth in SEQ ID NO: 46, HCDR3 with a sequence as set forth in SEQ ID NO: 84, LCDR1 with a sequence as set forth in SEQ ID NO: 112, LCDR2 with a sequence as set forth in SEQ ID NO: 118 and LCDR3 with a sequence as set forth in SEQ ID NO: 131; HCDR1 with a sequence as set forth in SEQ ID NO: 23, HCDR2 with a sequence as set forth in SEQ ID NO: 59, HCDR3 with a sequence as set forth in SEQ ID NO: 98, LCDR1 with a sequence as set forth in SEQ ID NO: 113, LCDR2 with a sequence as set forth in SEQ ID NO: 118 and LCDR3 with a sequence as set forth in SEQ ID NO: 132; or, HCDR1 with a sequence as set forth in SEQ ID NO: 16, HCDR2 with a sequence as set forth in SEQ ID NO: 60, HCDR3 with a sequence as set forth in SEQ ID NO: 84, LCDR1 with a sequence as set forth in SEQ ID NO: 112, LCDR2 with a sequence as set forth in SEQ ID NO: 118 and LCDR3 with a sequence as set forth in SEQ ID NO: 133;
preferably, the B7-H4-targeting antigen-binding domain comprises a VH with a sequence as set forth in SEQ ID NO: 142 and a VL with a sequence as set forth in SEQ ID NO: 166; a VH with a sequence as set forth in SEQ ID NO: 159 and a VL with a sequence as set forth in SEQ ID NO: 167; a VH with a sequence as set forth in SEQ ID NO: 160 and a VL with a sequence as set forth in SEQ ID NO: 168; or, a VH with a sequence as set forth in SEQ ID NO: 159 and a VL with a sequence as set forth in SEQ ID NO: 169;
and more preferably, the B7-H4-targeting antigen-binding domain comprises a heavy chain with a sequence as set forth in SEQ ID NO: 174 and a light chain with a sequence as set forth in SEQ ID NO: 198; a heavy chain with a sequence as set forth in SEQ ID NO: 191 and a light chain with a sequence as set forth in SEQ ID NO: 199; a heavy chain with a sequence as set forth in SEQ ID NO: 192 and a light chain with a sequence as set forth in SEQ ID NO: 200; or, a heavy chain with a sequence as set forth in SEQ ID NO: 191 and a light chain with a sequence as set forth in SEQ ID NO: 201.

4. The bispecific antibody according to claim 2, wherein the B7-H4-targeting antigen-binding domain is in the form of single VH, tandem VH, ScFv, Fab or IgG; the tandem VH preferably is a tandem of two or more, e.g., 2, 3 or 4, VHs; when in the form of the IgG, the B7-H4-targeting antigen-binding domain comprises a constant region preferably derived from a human IgG1 comprising mutations L234A, L235A and P329G or mutations L234A and L235A.

5. The bispecific antibody according to any one of claims 1-4, wherein the 4-1BB-targeting antigen-binding domain comprises HCDR1 with a sequence as set forth in SEQ ID NO: 19 or a variant 1 thereof, or SEQ ID NO: 14, HCDR2 with a sequence as set forth in SEQ ID NO: 49 or a variant 2 thereof, SEQ ID NO: 51 or SEQ ID NO: 43 and HCDR3 with a sequence as set forth in SEQ ID NO: 86 or a variant 3 thereof, SEQ ID NO: 96, SEQ ID NO: 89 or SEQ ID NO: 81; wherein
the variant 1 comprises mutations of one or more of T3I, S6N/R and Y7F; preferably, the sequence of variant 1 is as set forth in any one of SEQ ID NOs: 17-18 and SEQ ID NOs: 20-22 in the sequence listing;
the variant 2 comprises mutations of one or more of S1N/D, G2S/A, S3D/G, GSD/F/S/V and S6T/N/D; preferably, the sequence of variant 2 is as set forth in any one of SEQ ID NOs: 47-48, SEQ ID NO: 50, SEQ ID NOs: 52-58 and SEQ ID NO: 61 in the sequence listing;
the variant 3 comprises mutations of one or more of G2R/D/A/K, S3A/T, S4G/N/A/T/H E5T/V/M/G, T6A, D7G/S, H9Y/S, Y10H, Y11F, N12G/D and V13I/M/T; preferably, the amino acid sequence of variant 3 is as set forth in any one of SEQ ID NO: 85, SEQ ID NOs: 87-88 and SEQ ID NOs: 90-95 in the sequence listing;
preferably, the 4-1BB-targeting antigen-binding domain comprises: HCDR1, HCDR2 and HCDR3 with sequences as set forth in SEQ ID NOs: 17, 47 and 85, respectively;
or, HCDR1, HCDR2 and HCDR3 with sequences as set forth in SEQ ID NOs: 18, 48 and 86, respectively;
or, HCDR1, HCDR2 and HCDR3 with sequences as set forth in SEQ ID NOs: 18, 49 and 87, respectively;
or, HCDR1, HCDR2 and HCDR3 with sequences as set forth in SEQ ID NOs: 19, 50 and 88, respectively;
or, HCDR1, HCDR2 and HCDR3 with sequences as set forth in SEQ ID NOs: 20, 51 and 89, respectively;
or, HCDR1, HCDR2 and HCDR3 with sequences as set forth in SEQ ID NOs: 18, 52 and 90, respectively;
or, HCDR1, HCDR2 and HCDR3 with sequences as set forth in SEQ ID NOs: 18, 49 and 90, respectively;
or, HCDR1, HCDR2 and HCDR3 with sequences as set forth in SEQ ID NOs: 21, 53 and 91, respectively;
or, HCDR1, HCDR2 and HCDR3 with sequences as set forth in SEQ ID NOs: 21, 54 and 92, respectively;
or, HCDR1, HCDR2 and HCDR3 with sequences as set forth in SEQ ID NOs: 19, 55 and 93, respectively;
or, HCDR1, HCDR2 and HCDR3 with sequences as set forth in SEQ ID NOs: 18, 49 and 86, respectively;
or, HCDR1, HCDR2 and HCDR3 with sequences as set forth in SEQ ID NOs: 19, 49 and 94, respectively;
or, HCDR1, HCDR2 and HCDR3 with sequences as set forth in SEQ ID NOs: 22, 56 and 86, respectively;
or, HCDR1, HCDR2 and HCDR3 with sequences as set forth in SEQ ID NOs: 18, 57 and 95, respectively;
or, HCDR1, HCDR2 and HCDR3 with sequences as set forth in SEQ ID NOs: 19, 58 and 96, respectively;
or, HCDR1, HCDR2 and HCDR3 with sequences as set forth in SEQ ID NOs: 18, 61 and 95, respectively;
or, HCDR1, HCDR2 and HCDR3 with sequences as set forth in SEQ ID NOs: 14, 43 and 81, respectively; more preferably, the 4-1BB-targeting antigen-binding domain comprises one or more heavy chain variable regions with a sequence as set forth in SEQ ID NO: 143-157, 139, 284 or 161;
even more preferably, the 4-1BB-targeting antigen-binding domain comprises a heavy chain with a sequence as set forth in SEQ ID NO: 175-189, 193, 285 or 171.

6. The bispecific antibody according to claim 5, wherein the 4-1BB-targeting antigen-binding domain further comprises LCDR1, LCDR2 and LCDR3 with sequences as set forth in SEQ ID NOs: 109, 118, and 128, respectively; preferably comprises a light chain variable region with a sequence as set forth in SEQ ID NO: 163; and more preferably comprises a light chain with a sequence as set forth in SEQ ID NO: 195.

7. The bispecific antibody according to claim 5, wherein the 4-1BB-targeting antigen-binding domain is in the form of single VH or tandem VH, HCAb or ScFv; the tandem VH preferably is a tandem of two or more, e.g., 2, 3 or 4 VHs.

8. The bispecific antibody according to any one of claims 1-7, wherein the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 17, 47 and 85, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 87, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 50 and 88, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 20, 51 and 89, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 52 and 90, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 53 and 91, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 54 and 92, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 55 and 93, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 22, 56 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 17, 47 and 85, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 61 and 95, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 19, 58 and 96, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 49 and 90, respectively.

9. The bispecific antibody according to any one of claims 1-8, wherein the B7-H4-targeting antigen domain is in the form of IgG and the 4-1BB-targeting antigen-binding domain is in the form of single VH;
or, the B7-H4-targeting antigen domain is in the form of IgG and the 4-1BB-targeting antigen-binding domain is in the form of ScFv;
or, the B7-H4-targeting antigen domain is in the form of IgG and the 4-1BB-targeting antigen-binding domain is in the form of a tandem of 2 or 3 VHs;
or, the B7-H4-targeting antigen domain is in the form of Fab and the 4-1BB-targeting antigen-binding domain is in the form of HCAb or HCAb and VH (in the form of HCAb-VH );
or, the B7-H4-targeting antigen domain is in the form of Fab and the 4-1BB-targeting antigen-binding domain is in the form of VH, preferably wherein the form of VH is the form of single VH, or a tandem of 2 or 3 VHs;
preferably, the bispecific antibody is in a form where:
(1) the C-terminus of an IgG is connected with an ScFv, and an VH of the ScFv is connected to the C-terminus; preferably, the bispecific antibody in this form comprises a polypeptide chain 1 shown as a formula of VLB7-H4-CL, and a polypeptide chain 2 shown as a formula of VHB7-H4-CH1-hinge-CH2-CH3-linker-VH4-1BB-linker-VL4-1BB;
(2) the C-terminus of an IgG is connected with a VH or a tandem VH; preferably, the bispecific antibody in this form comprises a polypeptide chain 1 shown as a formula of VLB7-H4-CL, and a polypeptide chain 2 shown as a formula of VHB7-H4-CH1-hinge-CH2-CH3-linker-(VH4-1BB)n;
(3) the N-terminus of an HCAb is connected with an Fab; preferably, the bispecific antibody in this form comprises a polypeptide chain 1 shown as a formula of VHB7-H4-CH1, and a polypeptide chain 2 shown as a formula of VLB7-H4-CL-linker-VH4-1BB-linker-CH2-CH3;
(4) the N-terminus of an HCAb is connected with an Fab, and the C-terminus of the Fab is connected with an VH; preferably, the bispecific antibody in this form comprises a polypeptide chain 1 shown as a formula of VHB7-H4-CH1, and a polypeptide chain 2 shown as a formula of VLB7-H4-CL-linker-VH4-1BB-linker-CH2-CH3-VH4-1BB;
or (5) the N-termini of two CH2s of an Fc are each connected with an Fab, and a VH or a tandem VH; preferably, the bispecific antibody in this form comprises three polypeptide chains:
a polypeptide chain 1 shown as a formula of VLB7-H4-CL, a polypeptide chain 2 shown as a formula of VHB7-H4-CH1-hinge-CH2-CH3, and a polypeptide chain 3 shown as a formula of VH 4-1 BB-linker -CH2-CH3 or (VH4-1BB)n-linker-CH2-CH3.

10. The bispecific antibody according to claim 9, wherein the linker is selected from the group consisting of SEQ ID NOs: 241-261, 282 and 288-289, wherein
the linker in form (1) preferably comprises a sequence as set forth in SEQ ID NO: 245; the linker in form (2) preferably comprises a sequence as set forth in any one of SEQ ID NOs: 243 and 245-247 and SEQ ID NOs: 288-289;
the linker in form (3) preferably comprises a sequence as set forth in SEQ ID NO: 250;
the linker in form (4) preferably comprises a sequence as set forth in SEQ ID NO: 282; and
the linker in form (5) preferably comprises a sequence as set forth in SEQ ID NO: 245.

11. The bispecific antibody according to claim 10, wherein the amino acid sequence of polypeptide chain 1 is as set forth in SEQ ID NO: 198, and the amino acid sequence of polypeptide chain 2 is as set forth in any one of SEQ ID NOs: 202-215;
or, the amino acid sequence of polypeptide chain 1 is as set forth in SEQ ID NO: 201, and the amino acid sequence of polypeptide chain 2 is as set forth in SEQ ID NO: 217, 230, 238, 240, 226, 262 or 239;
or, the amino acid sequence of polypeptide chain 1 is as set forth in SEQ ID NO: 200, and the amino acid sequence of polypeptide chain 2 is as set forth in any one of SEQ ID NOs: 218-225, 235-237, 263-268, 274-281, 286 and 287;
or, the amino acid sequence of polypeptide chain 1 is as set forth in SEQ ID NO: 201, the amino acid sequence of polypeptide chain 2 is as set forth in SEQ ID NO: 227, and the amino acid sequence of polypeptide chain 3 is as set forth in SEQ ID NO: 228, 229, 231 or 232;
or, the amino acid sequence of polypeptide chain 1 is as set forth in SEQ ID NO: 233, and the amino acid sequence of polypeptide chain 2 is as set forth in any one of SEQ ID NOs: 234 and 269-273.

12. An isolated nucleic acid encoding the bispecific antibody according to any one of claims 1-11.

13. An expression vector comprising the isolated nucleic acid according to claim 12.

14. A host cell comprising the expression vector according to claim 13, preferably, wherein the host cell is a prokaryotic cell or a eukaryotic cell.

15. A method for preparing a bispecific antibody comprising culturing the host cell according to claim 14 and obtaining the bispecific antibody from the culture.

16. A pharmaceutical composition comprising the bispecific antibody according to any one of claims 1-11.

17. Use of the bispecific antibody according to any one of claims 1-11 or the pharmaceutical composition according to claim 16 in the manufacture of a medicament for the prevention or treatment of a 4-1BB and/or B7-H4-associated disease, wherein
the disease is preferably cancer, wherein the cancer is preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer; preferably, the cancer is breast cancer, ovarian cancer, endometrial cancer, renal cancer or cholangiocarcinoma; more preferably, the cancer is breast cancer.

18. A chimeric antigen receptor comprising the bispecific antibody according to any one of claims 1-11.

19. An antibody-drug conjugate comprising a cytotoxic agent and the bispecific antibody according to any one of claims 1-11, wherein preferably, the cytotoxic agent is MMAF or MMAE.

20. A kit comprising the bispecific antibody according to any one of claims 1-11, the chimeric antigen receptor according to claim 18, the antibody-drug conjugate according to claim 19 and/or the pharmaceutical composition according to claim 16, wherein
preferably, the kit further comprises (i) a device for administering the antibody or an antigen-binding fragment thereof or the antibody-drug conjugate or the pharmaceutical composition; and/or (ii) instructions for use.

21. A combination of kits comprising a kit A and a kit B, wherein:
the kit A comprises the bispecific antibody according to any one of claims 1-11, the chimeric antigen receptor according to claim 18, the antibody-drug conjugate according to claim 19 and/or the pharmaceutical composition according to claim 16;
the kit B comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

22. A method for diagnosing, treating and/or preventing a 4-1BB and/or B7-H4-mediated disease or disorder comprising administering to a patient in need thereof a therapeutically effective amount of the bispecific antibody according to any one of claims 1-11, the chimeric antigen receptor according to claim 18, the antibody-drug conjugate according to claim 19 or the pharmaceutical composition according to claim 16, or treating a patient in need thereof with the combination of kits according to claim 21.

23. The method according to claim 22, wherein the disease or disorder is a tumor, preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer; preferably, the cancer is breast cancer, ovarian cancer, endometrial cancer, renal cancer or cholangiocarcinoma; more preferably, the cancer is breast cancer.

24. A method for immuno-detection or determination of 4-1BB or B7H4 comprising using the bispecific antibody according to any one of claims 1-11, the chimeric antigen receptor according to claim 18, the antibody-drug conjugate according to claim 19 or the pharmaceutical composition according to claim 16, wherein preferably, the detection is for non-diagnostic and/or therapeutic purposes.

25. A combination therapy comprising administering to a patient in need thereof the bispecific antibody according to any one of claims 1-11, the chimeric antigen receptor according to claim 18, the antibody-drug conjugate according to claim 19 or the pharmaceutical composition according to claim 16, and a second therapeutic agent, wherein the second therapeutic agent preferably comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

## Amended claims

### Amended claims under Art. 19.1 PCT

1. A bispecific antibody comprising a B7-H4-targeting antigen-binding domain and a 4-1BB-targeting antigen-binding domain, wherein the B7-H4-targeting antigen-binding domain comprises HCDR1, HCDR2 and HCDR3, wherein the HCDR1 comprises an amino acid sequence as set forth in SEQ ID NO: 16 or 23, the HCDR2 comprises an amino acid sequence as set forth in SEQ ID NO: 46, 59 or 60, and the HCDR3 comprises an amino acid sequence as set forth in SEQ ID NO: 98 or 84.

2. The bispecific antibody according to claim 1, wherein the B7-H4-targeting antigen-binding domain comprises: HCDR1 with a sequence as set forth in SEQ ID NO: 16, HCDR2 with a sequence as set forth in SEQ ID NO: 60 and HCDR3 with a sequence as set forth in SEQ ID NO: 84; or, HCDR1 with a sequence as set forth in SEQ ID NO: 16, HCDR2 with a sequence as set forth in SEQ ID NO: 46 and HCDR3 with a sequence as set forth in SEQ ID NO: 84; HCDR1 with a sequence as set forth in SEQ ID NO: 23, HCDR2 with a sequence as set forth in SEQ ID NO: 59 and HCDR3 with a sequence as set forth in SEQ ID NO: 98; ; preferably comprises a VH with a sequence as set forth in SEQ ID NO: 142, SEQ ID NO: 159 or SEQ ID NO: 160; and more preferably comprises a heavy chain with a sequence as set forth in SEQ ID NO: 174, SEQ ID NO: 191 or SEQ ID NO: 192;
more preferably, the B7-H4-targeting antigen-binding domain further comprises LCDR1 with an amino acid sequence as set forth in SEQ ID NO: 112 or 113, LCDR2 with an amino acid sequence as set forth in SEQ ID NO: 118 and LCDR3 with an amino acid sequence as set forth in any one of SEQ ID NOs: 131-133;
even more preferably, the B7-H4-targeting antigen-binding domain further comprises: LCDR1 with a sequence as set forth in SEQ ID NO: 112, LCDR2 with a sequence as set forth in SEQ ID NO: 118 and LCDR3 with a sequence as set forth in SEQ ID NO: 133; or, LCDR1 with a sequence as set forth in SEQ ID NO: 112, LCDR2 with a sequence as set forth in SEQ ID NO: 118 and LCDR3 with a sequence as set forth in SEQ ID NO: 131; LCDR1 with a sequence as set forth in SEQ ID NO: 113, LCDR2 with a sequence as set forth in SEQ ID NO: 118 and LCDR3 with a sequence as set forth in SEQ ID NO: 132; preferably further comprises a VL with a sequence as set forth in any one of SEQ ID NOs: 166-169; and more preferably further comprises a light chain with a sequence as set forth in any one of SEQ ID NOs: 198-201.

3. The bispecific antibody according to claim 1 or 2, wherein the B7-H4-targeting antigen-binding domain comprises HCDR1 with a sequence as set forth in SEQ ID NO: 16, HCDR2 with a sequence as set forth in SEQ ID NO: 60, HCDR3 with a sequence as set forth in SEQ ID NO: 84, LCDR1 with a sequence as set forth in SEQ ID NO: 112, LCDR2 with a sequence as set forth in SEQ ID NO: 118 and LCDR3 with a sequence as set forth in SEQ ID NO: 133; or, HCDR1 with a sequence as set forth in SEQ ID NO: 16, HCDR2 with a sequence as set forth in SEQ ID NO: 46, HCDR3 with a sequence as set forth in SEQ ID NO: 84, LCDR1 with a sequence as set forth in SEQ ID NO: 112, LCDR2 with a sequence as set forth in SEQ ID NO: 118 and LCDR3 with a sequence as set forth in SEQ ID NO: 131; HCDR1 with a sequence as set forth in SEQ ID NO: 23, HCDR2 with a sequence as set forth in SEQ ID NO: 59, HCDR3 with a sequence as set forth in SEQ ID NO: 98, LCDR1 with a sequence as set forth in SEQ ID NO: 113, LCDR2 with a sequence as set forth in SEQ ID NO: 118 and LCDR3 with a sequence as set forth in SEQ ID NO: 132; ;
preferably, the B7-H4-targeting antigen-binding domain comprises a VH with a sequence as set forth in SEQ ID NO: 160 and a VL with a sequence as set forth in SEQ ID NO: 168; a VH with a sequence as set forth in SEQ ID NO: 142 and a VL with a sequence as set forth in SEQ ID NO: 166; a VH with a sequence as set forth in SEQ ID NO: 159 and a VL with a sequence as set forth in SEQ ID NO: 167; or, a VH with a sequence as set forth in SEQ ID NO: 159 and a VL with a sequence as set forth in SEQ ID NO: 169;
and more preferably, the B7-H4-targeting antigen-binding domain comprises a heavy chain with a sequence as set forth in SEQ ID NO: 192 and a light chain with a sequence as set forth in SEQ ID NO: 200; a heavy chain with a sequence as set forth in SEQ ID NO: 174 and a light chain with a sequence as set forth in SEQ ID NO: 198; a heavy chain with a sequence as set forth in SEQ ID NO: 191 and a light chain with a sequence as set forth in SEQ ID NO: 199; or, a heavy chain with a sequence as set forth in SEQ ID NO: 191 and a light chain with a sequence as set forth in SEQ ID NO: 201.

4. The bispecific antibody according to claim 1 or 2, wherein the B7-H4-targeting antigen-binding domain is in the form of single VH, tandem VH, ScFv, Fab or IgG; the tandem VH preferably is a tandem of two or more, e.g., 2, 3 or 4, VHs; when in the form of the IgG, the B7-H4-targeting antigen-binding domain comprises a constant region preferably derived from a human IgG1 comprising mutations L234A, L235A and P329G or mutations L234A and L235A.

5. The bispecific antibody according to any one of claims 1-4, wherein the 4-1BB-targeting antigen-binding domain comprises HCDR1 with a sequence as set forth in SEQ ID NO: 19 or a variant 1 thereof, or SEQ ID NO: 14, HCDR2 with a sequence as set forth in SEQ ID NO: 49 or a variant 2 thereof, SEQ ID NO: 51 or SEQ ID NO: 43 and HCDR3 with a sequence as set forth in SEQ ID NO: 86 or a variant 3 thereof, SEQ ID NO: 96, SEQ ID NO: 89 or SEQ ID NO: 81; wherein
the variant 1 comprises mutations of one or more of T3I, S6N/R and Y7F; preferably, the sequence of variant 1 is as set forth in any one of SEQ ID NOs: 17-18 and SEQ ID NOs: 20-22 in the sequence listing;
the variant 2 comprises mutations of one or more of S1N/D, G2S/A, S3D/G, GSD/F/S/V and S6T/N/D; preferably, the sequence of variant 2 is as set forth in any one of SEQ ID NOs: 47-48, SEQ ID NO: 50, SEQ ID NOs: 52-58 and SEQ ID NO: 61 in the sequence listing;
the variant 3 comprises mutations of one or more of G2R/D/A/K, S3A/T, S4G/N/A/T/H E5T/V/M/G, T6A, D7G/S, H9Y/S, Y10H, Y11F, N12G/D and V13I/M/T; preferably, the amino acid sequence of variant 3 is as set forth in any one of SEQ ID NO: 85, SEQ ID NOs: 87-88 and SEQ ID NOs: 90-95 in the sequence listing;
preferably, the 4-1BB-targeting antigen-binding domain comprises: HCDR1, HCDR2 and HCDR3 with sequences as set forth in SEQ ID NOs: 18, 61 and 95, respectively;
or, HCDR1, HCDR2 and HCDR3 with sequences as set forth in SEQ ID NOs: 17, 47 and 85, respectively;
or, HCDR1, HCDR2 and HCDR3 with sequences as set forth in SEQ ID NOs: 18, 48 and 86, respectively;
or, HCDR1, HCDR2 and HCDR3 with sequences as set forth in SEQ ID NOs: 18, 49 and 87, respectively;
or, HCDR1, HCDR2 and HCDR3 with sequences as set forth in SEQ ID NOs: 19, 50 and 88, respectively;
or, HCDR1, HCDR2 and HCDR3 with sequences as set forth in SEQ ID NOs: 20, 51 and 89, respectively;
or, HCDR1, HCDR2 and HCDR3 with sequences as set forth in SEQ ID NOs: 18, 52 and 90, respectively;
or, HCDR1, HCDR2 and HCDR3 with sequences as set forth in SEQ ID NOs: 18, 49 and 90, respectively;
or, HCDR1, HCDR2 and HCDR3 with sequences as set forth in SEQ ID NOs: 21, 53 and 91, respectively;
or, HCDR1, HCDR2 and HCDR3 with sequences as set forth in SEQ ID NOs: 21, 54 and 92, respectively;
or, HCDR1, HCDR2 and HCDR3 with sequences as set forth in SEQ ID NOs: 19, 55 and 93, respectively;
or, HCDR1, HCDR2 and HCDR3 with sequences as set forth in SEQ ID NOs: 18, 49 and 86, respectively;
or, HCDR1, HCDR2 and HCDR3 with sequences as set forth in SEQ ID NOs: 19, 49 and 94, respectively;
or, HCDR1, HCDR2 and HCDR3 with sequences as set forth in SEQ ID NOs: 22, 56 and 86, respectively;
or, HCDR1, HCDR2 and HCDR3 with sequences as set forth in SEQ ID NOs: 18, 57 and 95, respectively;
or, HCDR1, HCDR2 and HCDR3 with sequences as set forth in SEQ ID NOs: 19, 58 and 96, respectively;
or, HCDR1, HCDR2 and HCDR3 with sequences as set forth in SEQ ID NOs: 14, 43 and 81, respectively;
more preferably, the 4-1BB-targeting antigen-binding domain comprises one or more heavy chain variable regions with a sequence as set forth in SEQ ID NO: 143-157, 139, 284 or 161;
even more preferably, the 4-1BB-targeting antigen-binding domain comprises a heavy chain with a sequence as set forth in SEQ ID NO: 175-189, 193, 285 or 171.

6. The bispecific antibody according to claim 5, wherein the 4-1BB-targeting antigen-binding domain further comprises LCDR1, LCDR2 and LCDR3 with sequences as set forth in SEQ ID NOs: 109, 118, and 128, respectively; preferably comprises a light chain variable region with a sequence as set forth in SEQ ID NO: 163; and more preferably comprises a light chain with a sequence as set forth in SEQ ID NO: 195.

7. The bispecific antibody according to claim 5, wherein the 4-1BB-targeting antigen-binding domain is in the form of single VH or tandem VH, HCAb or ScFv; the tandem VH preferably is a tandem of two or more, e.g., 2, 3 or 4 VHs.

8. The bispecific antibody according to any one of claims 1-7, wherein the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 61 and 95, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises LCDR1, LCDR2 and LCDR3 with sequences as set forth in SEQ ID NOs: 109, 118 and 128, respectively, and HCDR1, HCDR2 and HCDR3 with sequences as set forth in SEQ ID NOs: 14, 43 and 81, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 17, 47 and 85, respectively;
the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 87, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 50 and 88, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 20, 51 and 89, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 52 and 90, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 53 and 91, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 21, 54 and 92, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 55 and 93, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 22, 56 and 86, respectively;
or, the B7-H4-targeting antigen-binding domain comprises LCDR1, LCDR2 and LCDR3 with sequences as set forth in SEQ ID NOs: 112, 118 and 131, respectively, and HCDR1, HCDR2 and HCDR3 with sequences as set forth in SEQ ID NOs: 16, 46 and 84, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 17, 47 and 85, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 18, 49 and 86, respectively;
or, the B7-H4-targeting antigen-binding domain comprises LCDR1, LCDR2 and LCDR3 with sequences as set forth in SEQ ID NOs: 112, 118 and 133, respectively, and HCDR1, HCDR2 and HCDR3 with sequences as set forth in SEQ ID NOs: 16, 60 and 84, respectively; and the 4-1BB-targeting antigen-binding domain comprises HCDR1, HCDR2 and HCDR3 with sequences as set forth in SEQ ID NOs: 19, 49 and 94, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 48 and 86, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 19, 58 and 96, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 57 and 95, respectively;
or, the B7-H4-targeting antigen-binding domain comprises LCDR1, LCDR2 and LCDR3 with sequences as set forth in SEQ ID NOs: 113, 118 and 132, respectively, and HCDR1, HCDR2 and HCDR3 with sequences as set forth in SEQ ID NOs: 23, 59 and 98, respectively; and the 4-1BB-targeting antigen-binding domain comprises HCDR1, HCDR2 and HCDR3 with sequences as set forth in SEQ ID NOs: 18, 49 and 90, respectively.

9. The bispecific antibody according to any one of claims 1-8, wherein the B7-H4-targeting antigen domain is in the form of IgG and the 4-1BB-targeting antigen-binding domain is in the form of single VH;
or, the B7-H4-targeting antigen domain is in the form of IgG and the 4-1BB-targeting antigen-binding domain is in the form of ScFv;
or, the B7-H4-targeting antigen domain is in the form of IgG and the 4-1BB-targeting antigen-binding domain is in the form of a tandem of 2 or 3 VHs;
or, the B7-H4-targeting antigen domain is in the form of Fab and the 4-1BB-targeting antigen-binding domain is in the form of HCAb or HCAb and VH (in the form of HCAb-VH);
or, the B7-H4-targeting antigen domain is in the form of Fab and the 4-1BB-targeting antigen-binding domain is in the form of VH, preferably wherein the form of VH is the form of single VH, or a tandem of 2 or 3 VHs;
preferably, the bispecific antibody is in a form where:
(1) the C-terminus of an IgG is connected with an ScFv, and an VH of the ScFv is connected to the C-terminus; preferably, the bispecific antibody in this form comprises a polypeptide chain 1 shown as a formula of VLB7-H4-CL, and a polypeptide chain 2 shown as a formula of VHB7-H4-CH1-hinge-CH2-CH3-linker-VH4-1BB-linker-VL4-1BB;
(2) the C-terminus of an IgG is connected with a VH or a tandem VH; preferably, the bispecific antibody in this form comprises a polypeptide chain 1 shown as a formula of VLB7-H4-CL, and a polypeptide chain 2 shown as a formula of VHB7-H4-CH1-hinge-CH2-CH3-linker-(VH4-1BB)n;
(3) the N-terminus of an HCAb is connected with an Fab; preferably, the bispecific antibody in this form comprises a polypeptide chain 1 shown as a formula of VHB7-H4-CH1, and a polypeptide chain 2 shown as a formula of VLB7-H4-CL-linker-VH4-1BB-linker-CH2-CH3;
(4) the N-terminus of an HCAb is connected with an Fab, and the C-terminus of the Fab is connected with an VH; preferably, the bispecific antibody in this form comprises a polypeptide chain 1 shown as a formula of VHB7-H4-CH1, and a polypeptide chain 2 shown as a formula of VLB7-H4-CL-linker-VH4-1BB-linker-CH2-CH3-VH4-1BB;
or (5) the N-termini of two CH2s of an Fc are each connected with an Fab, and a VH or a tandem VH; preferably, the bispecific antibody in this form comprises three polypeptide chains:
a polypeptide chain 1 shown as a formula of VLB7-H4-CL, a polypeptide chain 2 shown as a formula of VHB7-H4-CH1-hinge-CH2-CH3, and a polypeptide chain 3 shown as a formula of VH4-1BB-linker-CH2-CH3 or (VH4-1BB)n-linker-CH2-CH3.

10. The bispecific antibody according to claim 9, wherein the linker is selected from the group consisting of SEQ ID NOs: 241-261, 282 and 288-289, wherein
the linker in form (1) preferably comprises a sequence as set forth in SEQ ID NO: 245;
the linker in form (2) preferably comprises a sequence as set forth in any one of SEQ ID NOs: 243 and 245-247 and SEQ ID NOs: 288-289;
the linker in form (3) preferably comprises a sequence as set forth in SEQ ID NO: 250;
the linker in form (4) preferably comprises a sequence as set forth in SEQ ID NO: 282; and
the linker in form (5) preferably comprises a sequence as set forth in SEQ ID NO: 245.

11. The bispecific antibody according to claim 10, wherein the amino acid sequence of polypeptide chain 1 is as set forth in SEQ ID NO: 198, and the amino acid sequence of polypeptide chain 2 is as set forth in any one of SEQ ID NOs: 202-215;
or, the amino acid sequence of polypeptide chain 1 is as set forth in SEQ ID NO: 201, and the amino acid sequence of polypeptide chain 2 is as set forth in SEQ ID NO: 217, 230, 238, 240, 226, 262 or 239;
or, the amino acid sequence of polypeptide chain 1 is as set forth in SEQ ID NO: 200, and the amino acid sequence of polypeptide chain 2 is as set forth in any one of SEQ ID NOs: 218-225, 235-237, 263-268, 274-281, 286 and 287;
or, the amino acid sequence of polypeptide chain 1 is as set forth in SEQ ID NO: 201, the amino acid sequence of polypeptide chain 2 is as set forth in SEQ ID NO: 227, and the amino acid sequence of polypeptide chain 3 is as set forth in SEQ ID NO: 228, 229, 231 or 232;
or, the amino acid sequence of polypeptide chain 1 is as set forth in SEQ ID NO: 233, and the amino acid sequence of polypeptide chain 2 is as set forth in any one of SEQ ID NOs: 234 and 269-273.

12. An isolated nucleic acid encoding the bispecific antibody according to any one of claims 1-11.

13. An expression vector comprising the isolated nucleic acid according to claim 12.

14. A host cell comprising the expression vector according to claim 13, preferably, wherein the host cell is a prokaryotic cell or a eukaryotic cell.

15. A method for preparing a bispecific antibody comprising culturing the host cell according to claim 14 and obtaining the bispecific antibody from the culture.

16. A pharmaceutical composition comprising the bispecific antibody according to any one of claims 1-11.

17. Use of the bispecific antibody according to any one of claims 1-11 or the pharmaceutical composition according to claim 16 in the manufacture of a medicament for the prevention or treatment of a 4-1BB and/or B7-H4-associated disease, wherein
the disease is preferably cancer, wherein the cancer is preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer; preferably, the cancer is breast cancer, ovarian cancer, endometrial cancer, renal cancer or cholangiocarcinoma; more preferably, the cancer is breast cancer.

18. A chimeric antigen receptor comprising the bispecific antibody according to any one of claims 1-11.

19. An antibody-drug conjugate comprising a cytotoxic agent and the bispecific antibody according to any one of claims 1-11, wherein preferably, the cytotoxic agent is MMAF or MMAE.

20. A kit comprising the bispecific antibody according to any one of claims 1-11, the chimeric antigen receptor according to claim 18, the antibody-drug conjugate according to claim 19 and/or the pharmaceutical composition according to claim 16, wherein
preferably, the kit further comprises (i) a device for administering the antibody or an antigen-binding fragment thereof or the antibody-drug conjugate or the pharmaceutical composition; and/or (ii) instructions for use.

21. A combination of kits comprising a kit A and a kit B, wherein:
the kit A comprises the bispecific antibody according to any one of claims 1-11, the chimeric antigen receptor according to claim 18, the antibody-drug conjugate according to claim 19 and/or the pharmaceutical composition according to claim 16;
the kit B comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

22. A method for diagnosing, treating and/or preventing a 4-1BB and/or B7-H4-mediated disease or disorder comprising administering to a patient in need thereof a therapeutically effective amount of the bispecific antibody according to any one of claims 1-11, the chimeric antigen receptor according to claim 18, the antibody-drug conjugate according to claim 19 or the pharmaceutical composition according to claim 16, or treating a patient in need thereof with the combination of kits according to claim 21.

23. The method according to claim 22, wherein the disease or disorder is a tumor, preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma or colorectal cancer; preferably, the cancer is breast cancer, ovarian cancer, endometrial cancer, renal cancer or cholangiocarcinoma; more preferably, the cancer is breast cancer.

24. A method for immuno-detection or determination of 4-1BB or B7H4 comprising using the bispecific antibody according to any one of claims 1-11, the chimeric antigen receptor according to claim 18, the antibody-drug conjugate according to claim 19 or the pharmaceutical composition according to claim 16, wherein preferably, the detection is for non-diagnostic and/or therapeutic purposes.

25. A combination therapy comprising administering to a patient in need thereof the bispecific antibody according to any one of claims 1-11, the chimeric antigen receptor according to claim 18, the antibody-drug conjugate according to claim 19 or the pharmaceutical composition according to claim 16, and a second therapeutic agent, wherein the second therapeutic agent preferably comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.
